# EUROPEAN PATENT SPECIFICATION

(11) **EP 1 848 278 B1**
(45) Date of publication and mention of the grant of the patent: **07.09.2016**
(21) Application number: 06733931.7
(22) Date of filing: 27.01.2006
(51) Int. Cl.: A01N 65/00, A61K 47/10, A61K 47/26, A61K 47/38, A61K 47/40, A61K 47/44, A61K 31/05

(54) **ORAL FORMULATIONS FOR DELIVERY OF CATECHOLIC BUTANES INCLUDING NDGA COMPOUNDS**
FORMULIERUNGEN ZUR ORALEN VERABREICHUNG VON CATECHOLISCHEN BUTANEN MIT NDGA-VERBINDUNGEN
FORMULATIONS ORALES POUR L'ADMINISTRATION DE BUTANES CATECHOLIQUES COMPRENANT DES COMPOSES NDGA

(30) Priority: 27.01.2005 US 647495 P; 27.01.2005 US 647648 P
(43) Date of publication of application: 31.10.2007
(73) Proprietor: Erimos Pharmaceuticals LLC, Houston, TX 77036 (US)
(72) Inventor: LOPEZ, Rocio Alejandra, Durham, North Carolina 27703 (US); BLOMBERG, Jessica Andrea LoDuca, Raleigh, North Carolina 27615 (US); RHODES, Melissa Claire, Raleigh, North Carolina 27617 (US); HELLER, Jonathan Daniel, San Francisco, CA 94114 (US); GOODMAN, Amanda Beth, Wake Forest, North Carolina 27587 (US)
(74) Representative: Grünecker Patent- und Rechtsanwälte PartG mbB
(86) International application number: PCT/US2006/002806
(87) International publication number: WO 2006/081363

(56) References cited:
- WO-A1-98/15184
- WO-A1-99/17761
- WO-A1-2004/054560
- WO-A2-2004/112695
- US-A- 4 880 637
- US-A- 5 541 232
- US-A- 6 159 500
- US-A1- 2003 171 416

## Description

This application relates to a composition for oral administration to an animal comprising tetra-O-methyl NDGA. The application also relates to a composition for use in the treatment of diseases, for example, cancer or other proliferative or inflammatory diseases, metabolic diseases or neurodegenerative diseases.

Catecholic butanes such as nordihydroguaiaretic acid ("NDGA") have been tested for therapeutic applications in experimental animals by intratumor injection or topical application of such compounds. For example, Jordan et al. in US 5,008,294 described the effect of NDGA on human mammary carcinoma, MX-1, which was implanted subcutaneously into mice on day 0 (Example 2). Mice containing tumors were injected with various doses of NDGA on day 1, in a single intratumor injection. In another experiment, Jordan et al. injected a human breast adenocarcinoma, MX-1, intradermally into mice and treated the animals by topical application of NDGA after day 23 (Example 7).

Huang et al. described the testing of certain derivatives of NDGA (i.e., "NDGA derivatives") in US 6,214,874. In one experiment, mice were injected with an immortal mouse cell line, C3 cells, and treated by intratumor injection with M₄N alone or in combination with G₄N, both being derivatives of NDGA.

WO9917761 (A1) discloses formulations comprising nordihydroguaiaretic acid (NDGA) and an amphiphilic vehicle for lowering serum triglyceride, free fatty acid or glycerol level in animals.

WO9815184 (A1) discloses a nontoxic, therapeutic agent having pharmacological activity comprising a concentrated extract made by extracting Larrea tridentata plant material and adding a reducing agent such as ascorbic acid to reduce the toxic NDGA quinone, which naturally occurs in the plant material, to NDGA itself.

It would be desirable if an oral formulation of these anti-cancer compounds were discovered which would allow for easier administration, especially self-administration without the need for hospitalization. The present invention provides these desirable benefits.

It is, therefore, one of the objects of the present invention to provide one or more novel oral formulations of catecholic butanes and/or NDGA compounds, such as NDGA derivatives, for treatment of diseases by oral administration of such formulations to subjects in need of such treatment.

It is another one of the objects to provide formulations as above that are safe and have few adverse side effects when administered to animals, including humans.

It is another one of the objects to provide formulations as one or more of the foregoing that have a commercially reasonable period of stability.

It is another one of the objects to provide for formulations as one or more of the foregoing that have a commercially reasonable half-life in circulation upon administration to animals.

In accordance to the foregoing one or more objects of the invention, there is provided embodiments of the present invention as exemplified below:
The present invention is directed to a composition for oral administration to an animal comprising an active pharmaceutical ingredient and a pharmaceutically acceptable carrier, wherein the active pharmaceutical ingredient comprises tetra-O-methyl NDGA, and the carrier comprises polyethylene glycol, a cyclodextrin and optionally at least one of a solubilizing agent and an excipient selected from the group consisting of: (a) a water-soluble organic solvent other than DMSO; provided that when the water-soluble organic solvent is propylene glycol, the propylene glycol is in the absence of white petrolatum, in the absence of xanthan gum and in the absence of at least one of glycerine or glycine, when the water-soluble organic solvent is polyethylene glycol, the polyethylene glycol is present in the absence of ascorbic acid or butylated hydroxytoluene, and when the polyethylene glycol is polyethylene glycol 400, the polyethylene glycol 400 is present in the absence of polyethylene glycol 8000; (b) an ionic, non-ionic or amphipathic surfactant, provided that when the surfactant is a non-ionic surfactant, the non-ionic surfactant is present in the absence of xanthan gum; (c) a modified cellulose; (d) a water-insoluble lipid, provided that when the water-insoluble lipid is castor oil, the castor oil is present in the absence of beeswax or carnuba wax; and a combination of any of the carriers (a) - (d).

In another embodiment, the present invention is directed to a pharmaceutical composition comprising the composition of the invention.

In another embodiment, the present invention is directed to the composition of the invention for use in the treatment of a proliferative disease, hypertension, obesity, diabetes, a central nervous system disease, a neurodegenerative disease, pain, Alzheimer's disease, amyotrophic lateral sclerosis, dementia, Parkinson's disease, stroke, an inflammatory disease, premalignant neoplasia, dysplasia or an infection.

In another embodiment, the present invention is directed to a kit comprising the composition of the invention and instructions for use thereof.

Preferred embodiments are set forth in the subclaims.

The foregoing summary, as well as the following detailed description of the invention, will be better understood when read in conjunction with the appended drawings. For the purpose of illustrating the invention, there are shown in the drawings embodiments which are presently preferred.

In the drawings:

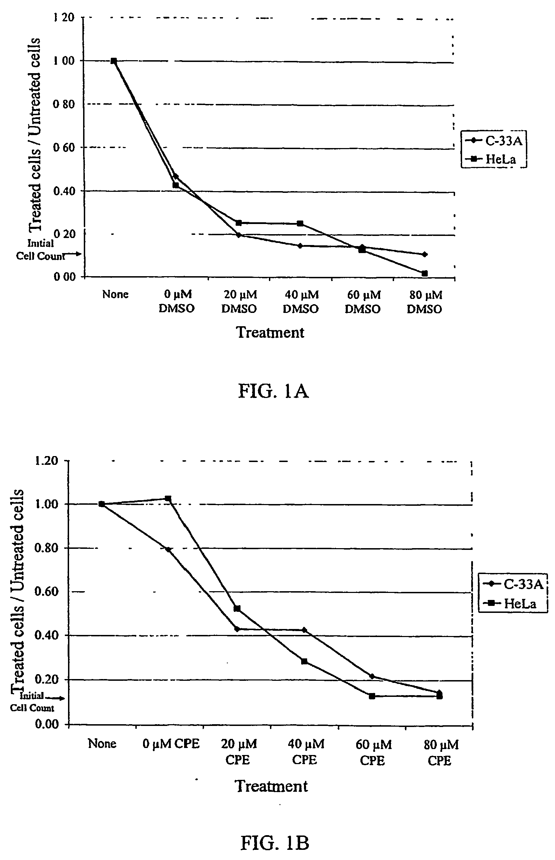

Fig. 1 comprises Figs. 1A and 1B and depicts the results of cell proliferation assays conducted for the C-33A cell line and the HeLa cell line after M₄N treatment. Fig. 1A is a graphical representation of the ratio of number of cells present after M₄N treatment over the number of cells present in the absence of M₄N treatment, where M₄N was provided in amounts varying from 0 µM to 80 µM in a DMSO formulation. Fig. 1B is a graphical representation of the ratio of number of cells present after M₄N treatment over the number of cells present in the absence of M₄N treatment, where M₄N was provided in amounts varying from 0 µM to 80 µM in a HP-β-CD/PEG formulation ("CPE" formulation).

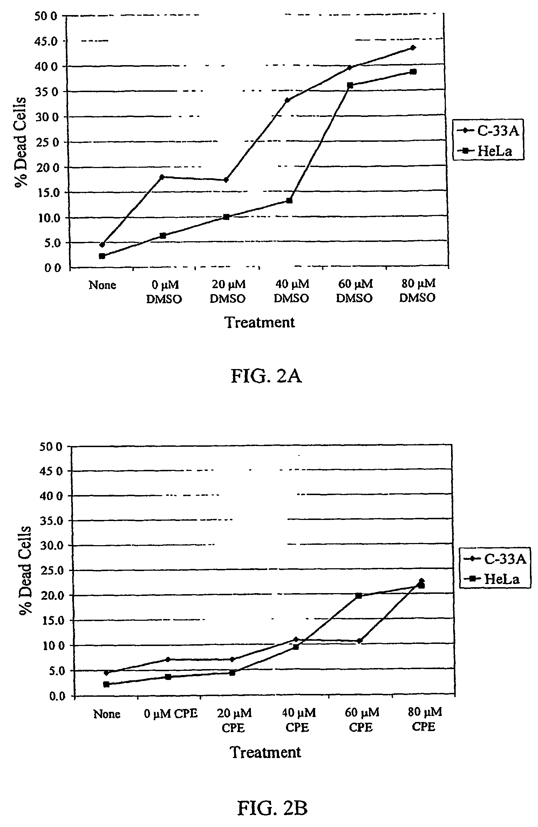

Fig. 2 comprises Figs. 2A and 2B and is a graphical representation of cell death measurements based on percentage of dead cells for C-33A cells and HeLa cells in the absence or presence of varying concentrations of M₄N in a DMSO (Fig. 2A) formulation or in a HP-β-CD/PEG formulation (Fig. 2B). The M₄N concentrations varied from 0 µM to 80 µM.

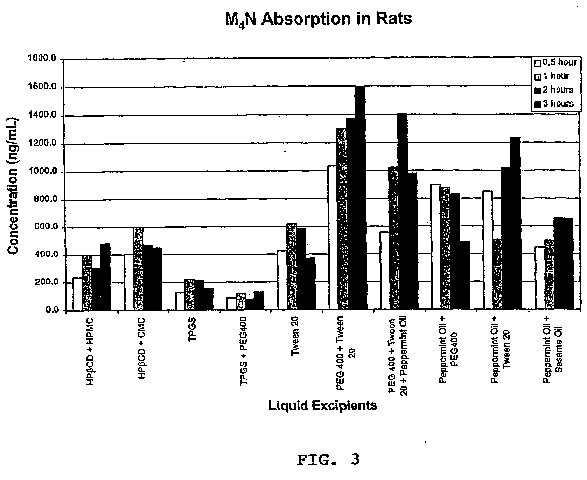

Fig. 3 is a is a histogram showing the absorption of M₄N in Sprague Dawley rats at time points 0.5 hr, 1 hr, 2 hr and 3 hr after oral administration of a single 500 mg/kg dose of M₄N in different liquid solubilizing agents and/or excipients ("carrier"). M₄N was present at a concentration of about 60 mg/mL in all the carriers. The carriers include: (a) HP-β-CD (500 mg/mL) + HPMC (5 mg/mL); (b) HP-β-CD (500 mg/mL) + CMC (5 mg/mL); (c) TPGS (200 mg/mL); (d) TPGS (100 mg/mL) + PEG 400 (50% v/v); (e) Tween® 20; (f) PEG 400 (50% v/v) + Tween® 20 (50% v/v); (g) PEG 400 (33% v/v) + Tween® 20 (33% v/v) + Peppermint oil (33%); (h) Peppermint oil (50%) + PEG 400 (50% v/v); (h) Peppermint oil (50%) + Tween® 20 (50% v/v); (i) Peppermint oil (50%) + Sesame Oil (50%).;

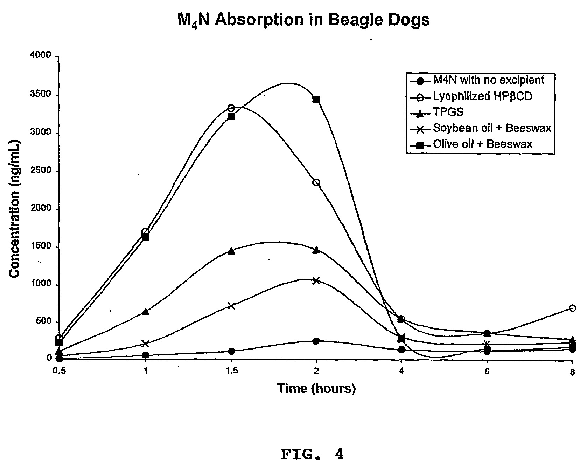

Fig. 4 is a graphical representation of the absorption of M₄N by beagle dogs at time points 0.5 hr, 1 hr, 1.5 hr, 2 hr, 4 hr, 6 hr and 8 hr after oral administration of a single 100 mg/kg dose of M₄N in a powder form or in different solid carriers, as follows: (a) M₄N powder; (b) Lyophilized HP-β-CD (81%) + M₄N (185 mg/g) ; (c) TPGS (20%) + M₄N (133 mg/g); (d) soybean oil (95%) + beeswax (5%) + M₄N (200 mg/g); and (d) olive oil (95%) + beeswax (5%) + M₄N (200 mg/g).;

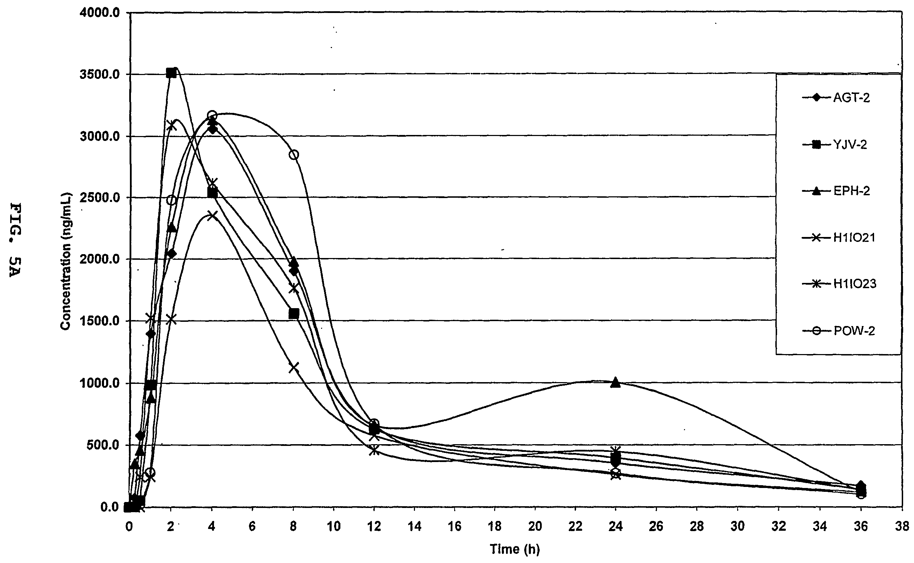

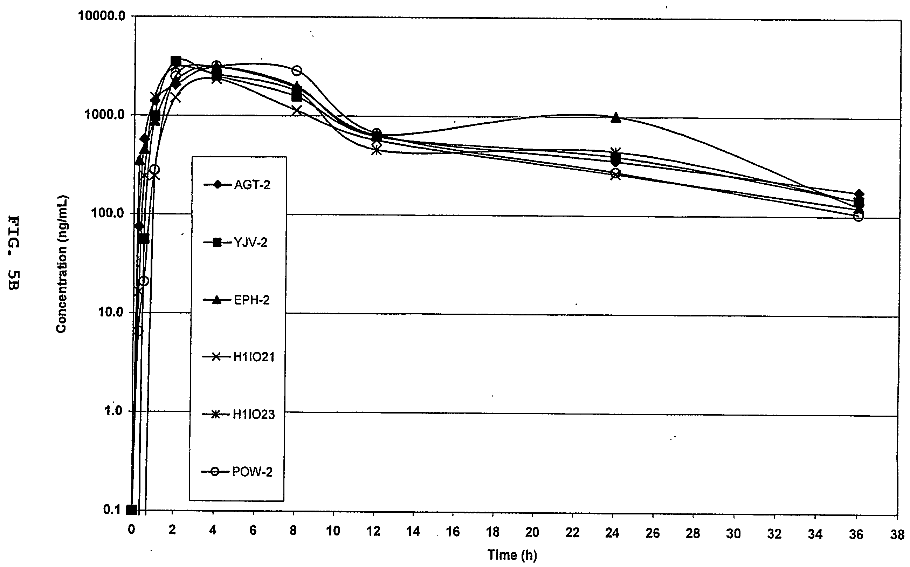

Fig. 5 comprises Figs. 5A and 5B and is a graphical representation of absorption of non-micronized M₄N in glycerol monooleate by beagle dogs at time points 0.5 hr, 1 hr, 1.5 hr, 2 hr, 4 hr, 8 hr, 12 hr, 24 hr and 36 hr after oral administration of a single 100 mg/kg dose. Fig. 5A is on a non-logarithmic scale. Fig. 5B is on a logarithmic scale.

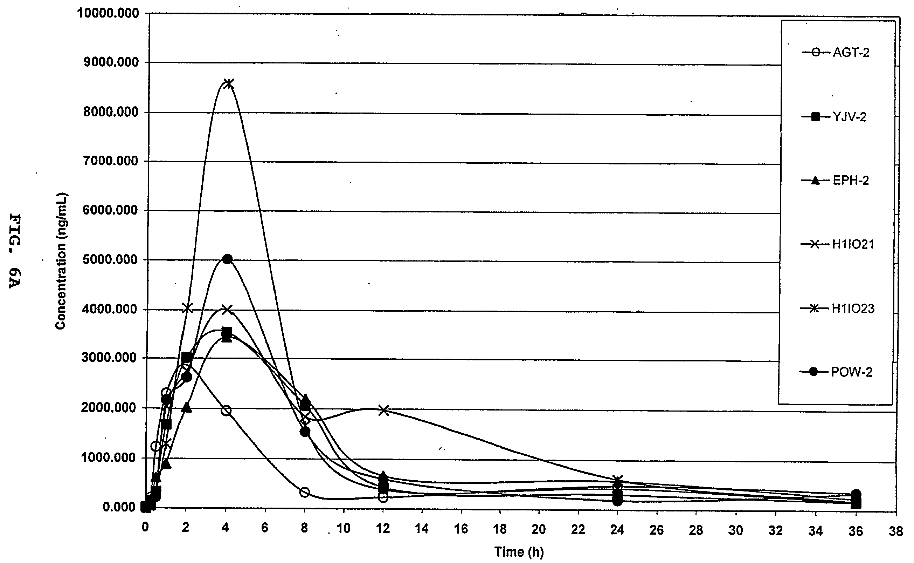

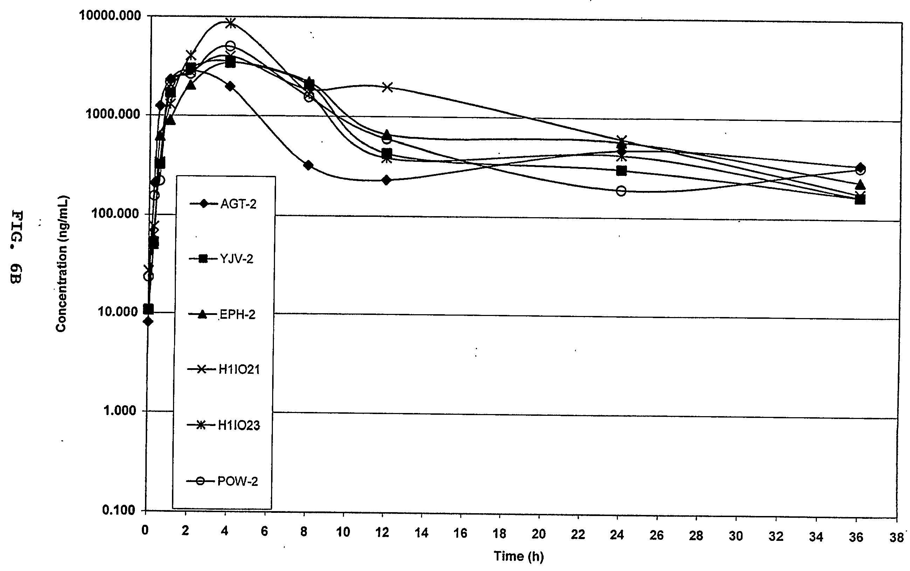

Fig. 6 comprises Figs. 6A and 6B and is a graphical representation of absorption of micronized M₄N in glycerol monooleate by beagle dogs at time points 0.5 hr, 1 hr, 1.5 hr, 2 hr, 4 hr, 8 hr, 12 hr, 24 hr and 36 hr after oral administration of a single 100 mg/kg dose. Fig. 6A is on a non-logarithmic scale. Fig. 6B is on a logarithmic scale.

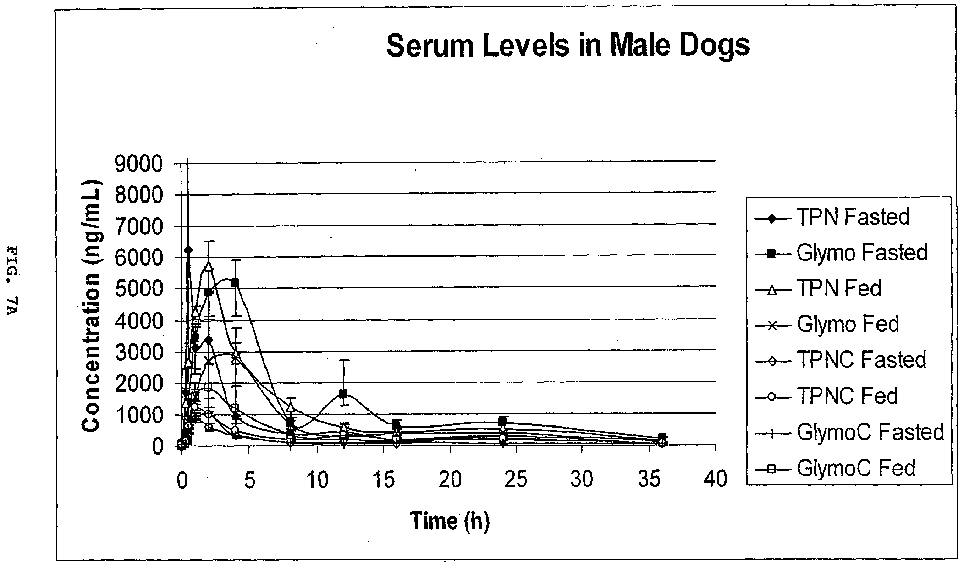

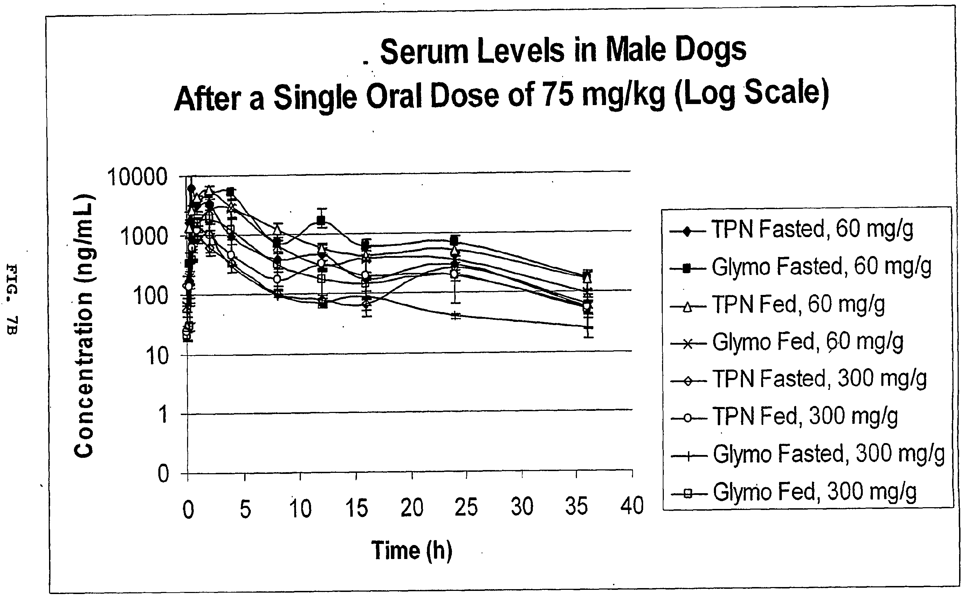

Fig. 7 comprises Figs. 7A and 7B and is a graphical representation of serum levels of M₄N in various carriers at various concentrations by male beagle dogs at time points 0.5 hr, 1 hr, 1.5 hr, 2 hr, 4 hr, 8 hr, 12 hr, 16 hr, 24 hr and 36 hr after oral administration of a single 75 mg/kg oral dose. The abbreviations of the carrier used, concentration of M₄N administered, and an indication of whether the administration was to dogs that fasted or were fed are best shown in Fig. 7B. Fig. 7A presents the data on a non-logarithmic scale. Fig. 7B presents the data on a logarithmic scale.

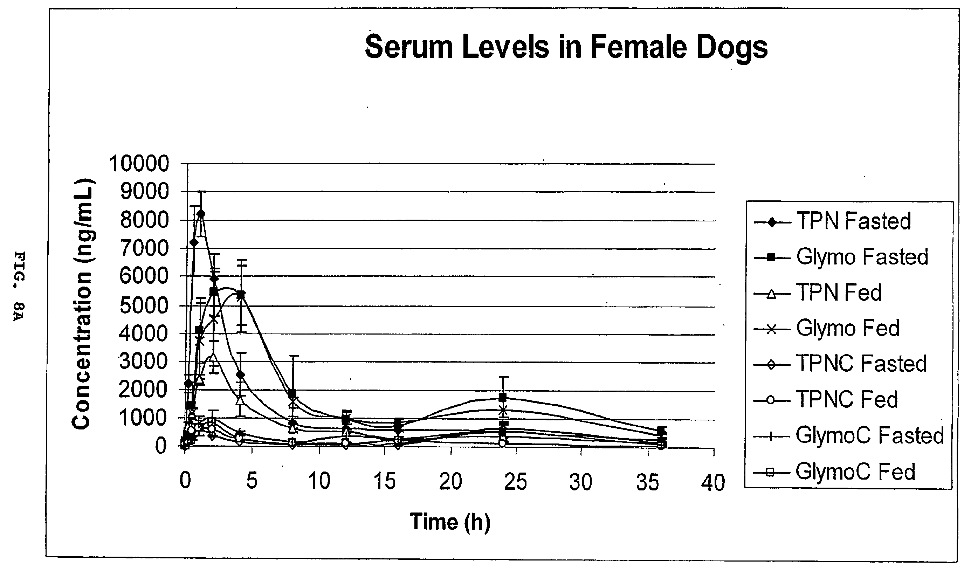

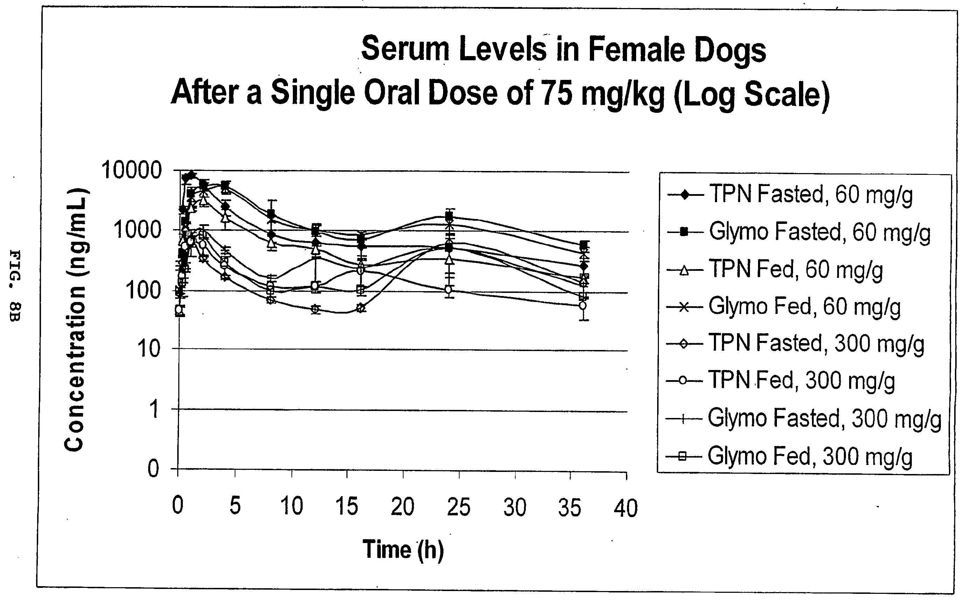

Fig. 8 comprises Figs. 8A and 8B and is a graphical representation of serum levels of M₄N in various carriers at various concentrations by female beagle dogs at time points 0.5 hr, 1 hr, 1.5 hr, 2 hr, 4 hr, 8 hr, 12 hr, 16 hr, 24 hr and 36 hr after oral administration of a single 75 mg/kg oral dose. The abbreviations of the carrier used, concentration of M₄N administered, and an indication of whether the administration was to dogs that fasted or were fed are best shown in Fig. 8B. Fig. SA presents the data on a non-logarithmic scale. Fig. 8B presents the data on a logarithmic scale.

The present invention provides for a novel composition, kit and composition for use in the treatment of diseases, including proliferative diseases such as cancer and psoriasis, hypertension, obesity, diabetes, type I or type II, central nervous system diseases or neurodegenerative disorders including, pain, Alzheimer's disease, stroke, and inflammatory disease, premalignant neoplasia or dysplasia, infection including viral infections such as HIV, HTLV, HPV, HSV, HBV, EBV, Varicella-zoster, adenovirus, parvovirus, JC virus or others. The diseases include proliferative diseases
such as cancer and psoriasis, hypertension, obesity, type I or type II diabetes, central nervous system diseases or neurodegenerative diseases including pain, Alzheimer's disease, amyotrophic lateral sclerosis, Parkinson's disease, dementia, stroke, and inflammatory disease, premalignant neoplasia or dysplasia, infection including viral infections such as human immunodeficiency viruses ("HIV"), human T-cell lymphotropic virus ("HTLV), human papilloma virus ("HPV"), herpes simplex viruses ("HSV"), hepatitis B virus ("HBV"), Epstein-Barr virus ("EBV"), Varicella-zoster, adenovirus, parvovirus, Jakob Creutzfeldt virus ("JC virus") or others.

The present invention provides for a novel composition containing tetra-O-methyl NDGA that is dissolved in certain pharmaceutically acceptable solubilizing agents, which together with other diluents, excipients and the like (collectively, "carrier") constitute formulations appropriate for administration to subjects, such as humans, for treatment of diseases. Such formulations are suitable for oral administration. A suitable pharmaceutically acceptable carrier includes at least one selected from:
(a) a water-soluble organic solvent other than DMSO, such as polyethylene glycol ("PEG"), for example, PEG 300, PEG 400 or PEG 400 monolaurate, propylene glycol ("PG"), polyvinyl pyrrolidone ("PVP"), ethanol, benzyl alcohol or dimethylacetamide;
(b) an ionic, non-ionic or amphipathic surfactant, such as polyoxyethylene sorbitan monolaurate (also known as polysorbate), which is a non-ionic surfactant, for example, polysorbate 20 and polysorbate 80, commercially available as Tween® 20 or Tween® 80, d-alpha-tocopheryl polyethylene glycol 1000 succinate ("TPGS"), glycerol monooleate (also known as glyceryl monooleate), an esterified fatty acid or a reaction product between ethylene oxide and castor oil in a molar ratio of 35:1, commercially available as Cremophor® EL; (c) a modified cellulose, such as ethyl cellulose ("EC"), hydroxylpropyl methylcellulose ("HPMC"), methyl cellulose ("MC") or carboxy methylcellulose ("CMC"); and (d) a water-insoluble lipid, such as a wax, oil or a fat emulsion, for example Intralipid®. The cyclodextrin can be an unmodified cyclodextrin or a modified cyclodextrin such as
   hydroxypropyl-β-cyclodextrin ("HP-β-CD") or sulfobutyl ether-β-cyclodextrin ("SBE-β-CD").

In one embodiment of the invention, the compounds herein are dissolved or dissolved and diluted in different carriers to form an oral liquid composition for administration into animals. For example, in one aspect of the embodiment, the present active pharmaceutical ingredient ("API") compound is solubilized and/or diluted in a combination formulation
containing a PEG compound and HP-β-CD.

In yet another embodiment, the compounds herein are dissolved in ionic, non-ionic or amphipathic surfactants such as Tween® 20, Tween® 80, TPGS or an esterified fatty acid. For example, the present compounds can be dissolved in TPGS alone, or Tween® 20 alone, or in combinations such as TPGS and PEG 400, or Tween® 20 and PEG 400.

In a further embodiment, the present compounds are dissolved in a water-insoluble lipid such as a wax, fat emulsion, for example Intralipid®, or oil. For example, the present compounds can be dissolved in peppermint oil alone, or in combinations of peppermint oil with Tween® 20 and PEG 400, or peppermint oil with PEG 400, or peppermint oil with Tween® 20, or peppermint oil with sesame oil.

Of course, ethyl cellulose may be substituted or added in place of the HPMC or CMC in the foregoing examples; PEG 300 or PEG 400 monolaurate can be substituted or added in place of PEG 400 in the foregoing examples; Tween® 80 may be substituted or added in place of Tween® 20 in the foregoing examples; and other oils such as corn oil, olive oil, soybean oil, mineral oil or glycerol, may be substituted or added in place of the peppermint oil or sesame oil in the foregoing examples.

Further, heating may be applied, for example, heating to a temperature of about 30°C to about 90°C, in the course of formulating any of these compositions to achieve dissolution of the compounds herein or to obtain an evenly distributed suspension of the present compounds.

In one embodiment, the compounds herein are first dissolved in a liquid carrier as in the foregoing examples, and

In a further embodiment, the present compounds are dissolved or suspended in a TPGS solution, with heating as appropriate to obtain an evenly distributed solution or suspension. Upon cooling, the composition becomes creamy and is suitable for oral administration.

In yet another embodiment, the present compounds are dissolved in oil and beeswax is added to produce a waxy solid composition.

In as yet a further embodiment of the invention, the compounds herein are solubilized in modified celluloses such as EC. The modified celluloses such as ethyl cellulose can be diluted in ethanol ("EtOH") prior to use.

The present invention also provides water-insoluble lipids as solubilizers for the present compounds. The water-insoluble lipids include, for example, oils as well as mixed fat emulsion compositions such as Intralipid® (Pharmacia & Upjohn, now Pfizer), used as per the manufacturer's recommendation. For example, adult dosage is recommended to be not exceeding 2 g of fat/kg body weight/day (20 mL, 10 mL and 6.7 mL/kg of Intralipid® 10%, 20% and 30%, respectively). Intralipid® 10% is believed to contain in 1,000 mL: purified soybean oil 100g, purified egg phospholipids 12 g, glycerol anhydrous 22 g, water for injection q.s. ad 1,000 mL. pH is adjusted with sodium hydroxide to pH approximately 8. Intralipid® 20% contains in 1,000 mL: purified soybean oil 200 g, purified egg phospholipids 12 g, glycerol anhydrous 22 g, water for injection q.s. ad 1,000 mL. pH is adjusted with sodium hydroxide to pH approximately 8. Intralipid® 30% contains in 1,000 mL: purified soybean oil 300 g, purified egg phospholipids 12 g, glycerol anhydrous 16.7 g, water for injection q.s. ad 1,000 mL. pH is adjusted with sodium hydroxide to pH approximately 7.5. These Intralipid® products are stored at controlled room temperature below 25°C and should not be frozen.

In general, in preparing the oral formulations, the compounds herein are first solubilized before other excipients are added so as to produce compositions of higher stability. Unstable formulations are not desirable. Unstable liquid formulations frequently form crystalline precipitates or biphasic solutions. Unstable solid formulations frequently appear grainy and clumpy and sometimes contain runny liquids. An optimal solid formulation appears smooth, homogenous, and has a small melting temperature range. In general, the proportions of excipients in the formulation may influence stability. For example, too little stiffening agent such as beeswax may leave the formulation too runny.

Hence, in general, for the liquid formulations of the present invention, the excipients used should be good solvents of the API M₄N. In other words, the exipients should be able to dissolve the API without heating. The excipients should also be compatible with each other independent of the API such that they can form a stable solution, suspension or emulsion. Also, in general, for the solid formulations of the present invention, the excipients used should also be good solvents of the API to avoid clumps and non-uniform formulations. To avoid solid formulations that are too runny or heterogeneous in texture, which are undesirable, the excipients should be compatible with each other such that they form a smooth homogeneous solid, even in the absence of the API.

The terms used herein have their ordinary dictionary meaning and as used by persons skilled in the art unless otherwise provided. In particular, the present invention can be better understood in light of the following definitions, which are used with other terms as defined elsewhere herein:

The term "about" as used herein in reference to a concentration or dose means the specified number up to ± 10% to 20%.

The term "active pharmaceutical ingredient," "API" or reference to the "compounds" herein means tetra-O-methyl NDGA.

The "buffer" suitable for use herein includes any buffer conventional in the art, such as, for example, Tris, phosphate, imidazole, and bicarbonate.

A "carrier" as used herein refers to a non-toxic solid, semisolid or liquid filler, diluent, vehicle, excipient, solubilizing agent, encapsulating material or formulation auxiliary of any conventional type, and encompasses all of the components of the composition other than the active pharmaceutical ingredient. The carrier may contain additional agents such as wetting or emulsifying agents, or pH buffering agents. Other materials such as anti-oxidants, humectants, viscosity stabilizers, and similar agents may be added as necessary.

A "cyclodextrin" as used herein means an unmodified cyclodextrin or a modified cyclodextrin, and includes α-cyclodextrin, β-cyclodextrin, γ-cyclodextrin and any modified cyclodextrins containing modifications thereto, such as HP-β-CD or SBE-β-CD. Cyclodextrin typically has 6 (α-cyclodextrin), 7 (β-cyclodextrin), and 8 (γ-cyclodextrin) sugars, up to three substitutions per sugar, and 0 to 24 primary substitutions are therefore possible (primary substitutions are defined as substitutions connected directly to the cyclodextrin ring). The modified or unmodified cyclodextrins used in the present invention may have any appropriate number and location of primary substitutions or other modifications.

The term "disease" as used herein includes all diseases, conditions, infections, syndromes or disorders for which the application of the present composition produces a therapeutic effect. Such "disease" includes, for example, cancer, psoriasis and other proliferative diseases, inflammatory disorders including rheumatoid arthritis, osteoarthritis, ulcerative colitis, Crohn's disease, atherosclerosis, chronic obstructive pulmonary disease ("COPD"), hypertension, obesity, diabetes, pain, stroke and/or other neuronal disorders or neurodegenerative diseases or conditions, including Alzheimer's disease, Parkinson's disease, multiple sclerosis, amyotrophic lateral sclerosis ("ALS") and premalignant conditions such as intraepithelial neoplasia or dysplasia, and infectious diseases.

"M₄N" or "tetra-O-methyl NDGA" as used herein is an NDGA derivative of Formula II in which R₁₄, R₁₅, R₁₆ and R₁₇ each independently represents -OCH₃, and R₁₈ and R₁₉ are each -CH₃.

A "modified cellulose" as used herein means a cellulose that contains one or more modifications to the cellulose molecule and includes, for example EC, HPMC, CMC and MC.

"NDGA" as used herein means nordihydroguaiaretic acid and has the following formula:

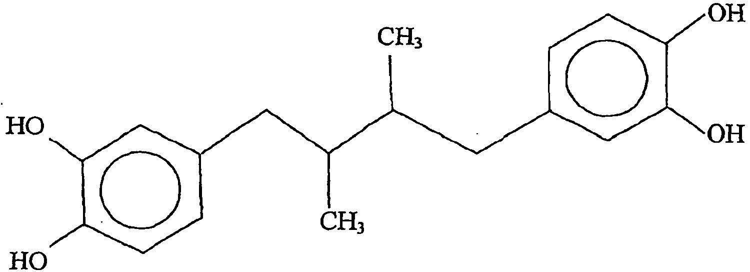

"NDGA derivative" as used herein means a derivative of NDGA having a Formula II

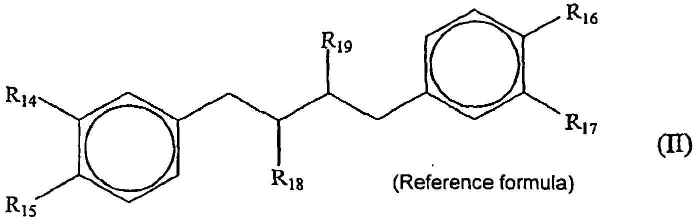

As used herein, "percent," "percentage" or the symbol "%" means the percent of the component indicated in the composition based on the amount of the carrier present in the composition, on a weight/weight (w/w), weight/volume (w/v) or volume/volume (v/v), as indicated with respect to any particular component, all based on the amount of the carrier present in the composition. Thus, different types of carriers may be present in an amount of up to 100% as indicated, which does not preclude the presence of the API, the amount of which may be indicated as a % or as a certain number of mg present in the composition or a certain number of mg/g present, or a certain number of mg/mL present, where the % or or mg/g or mg/mL is based on the amount of the total carrier present in the composition. Certain types of carriers may be present in combination to make up 100% of the carrier.

A "pharmaceutically acceptable carrier" as used herein is non-toxic to recipients at the dosages and concentrations employed, and is compatible with other ingredients of the formulation. For example, the carrier for a formulation containing tetra-O-methyl NDGA preferably does not include oxidizing agents and other compounds that are known to be deleterious to such. A pharmaceutically acceptable carrier comprises a solubilizing agent. Suitable pharmaceutically acceptable carriers include water, dextrose, glycerol, saline, ethanol, buffer, Cremaphor® EL, phosphate buffered saline, PEG 300, PEG 400, modified cyclodextrin, and combinations thereof, all as set forth above.

The term "pharmaceutically acceptable excipient," includes vehicles, adjuvants, or diluents or other auxiliary substances, such as those conventional in the art, which are readily available to the public, and which are non-toxic to recipients at the dosages and concentrations employed, and is compatible with other ingredients of the formulation.. For example, pharmaceutically acceptable auxiliary substances include pH adjusting and buffering agents, tonicity adjusting agents, stabilizers, and wetting agents.

The term "solubilizing agent" as used herein means a composition in tetra-O-methyl NDGA dissolves. A
solubilizing agent may also be a carrier or a pharmaceutically acceptable carrier.

The terms "subj ect," "host," and "patient," are used interchangeably herein to refer to an animal being treated with the present compositions, including simians, humans, felines, canines, equines, bovines, porcines, ovines, caprines, mammalian farm animals, mammalian sport animals, and mammalian pets.

A "substantially purified" compound in reference to the catecholic butanes or NDGA derivatives as used herein is one that is substantially free of materials that are not the catecholic butane, NDGA compounds or NDGA derivatives (collectively, "non-NDGA materials"). By substantially free is meant at least about 50% free of non-NDGA materials, preferably at least about 70%, more preferably at least about 80%, even more preferably at least about 90%, and still more preferably at least about 95% free of non-NDGA materials.

As used herein, the terms "treatment," "treating," and the like, refer to obtaining a desired pharmacologic and/or physiologic effect. The effect may be prophylactic in terms of completely or partially preventing a condition or disease or symptom thereof and/or may be therapeutic in terms of a partial or complete cure for a condition or disease and/or adverse effect attributable to the condition or disease. "Treatment," of a subject covers, for example, any treatment of a disease in a mammal, particularly in a human, and includes: (a) preventing the disease from occurring in a subject who may be predisposed to the disease but has not yet been diagnosed as having it; (b) inhibiting the disease from developing or progressing, such as, arresting its development; and (c) relieving, alleviating or ameliorating the disease, such as, for example, causing regression or remission of the disease.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

It must be noted that as used herein, the singular forms "a", "an", and "the" include plural referents unless the context clearly dictates otherwise. Thus, for example, reference to "a compound" includes a plurality of such compounds.

Citations to references mentioned in the text of this application are identified more fully in the bibliography preceding the claims.

Tetra-O-methyl NDGA,
also known as meso-1,4-bis(3,4-dimethoxyphenyl)-2,3-dimethylbutane, or M₄N, is made as follows: a solution is made containing NDGA and potassium hydroxide in methanol in a reaction flask. Dimethyl sulfate is then added to the reaction flask and the reaction is allowed to proceed. The reaction is finally quenched with water, causing the product to precipitate. The precipitate is isolated by filtration and dried in a vacuum oven. The compound is then dissolved in a solution of methylene chloride and toluene and subsequently purified through an alumina column. The solvents are removed by rotary evaporation and the solid is resuspended in isopropanol and isolated by filtration. The filter cake is dried in a vacuum oven. The resulting tetra-O-methyl NDGA (M₄N) is crystallized by refluxing the filter cake in isopropanol and re-isolating the crystals by filtration.

### Preparation of the Therapeutic Compositions

The present invention provides compositions, including pharmaceutical compositions, comprising tetra-O-methyl NDGA as active pharmaceutical ingredients ("API"), and pharmaceutically acceptable carriers or excipients. Typically, the compositions of the instant invention will contain from less than about 0.1 % up to about 99 % of the API; optionally, the present invention will contain about 2% to about 90% of the API.

The present invention additionally provides compositions in which M₄N, is present in concentrations of 1 mg/mL to 200 mg/mL, or 10 mg/mL to 175 mg/mL, or 20 mg/mL to 150 mg/mL, or 30 mg/mL to 125 mglmL, or 40 mg/mL to 100 mg/mL, or 50 mg/mL to 75 mg/mL. In one embodiment, M4N is present in the compositions herein at a concentration of 1 mg/mL, 2 mg/mL, 2.5 mg/mL, 5 mg/mL, 10 mg/mL, 15 mg/mL, 20 mg/mL, 25 mg/mL, 30 mg/mL, 35 mg/mL, 40 mg/mL, 45 mg/mL, 50 mgtmL, 55 mg/mL 60 mg/mL 75 mg/mL, 80 mg/mL, 90 mg/mL, 100 mg/mL, 120 mg/mL, 125 mg/mL, 150 mg/mL, 175 mg/mL or 200 mg/mL.

The present invention additionally provides compositions in which M₄N, is present in concentrations in the range of about 1 mg/g to 250 mg/g, or optionally 20 mg/g to 200 mg/g, or 40 mg/g to 180 mg/g, or 60 mg/g to 160 mg/g, or 80 mg/g to 130 mg/g, or 50 mg/g to 100 mg/g. In one embodiment, the present compounds are present in the compositions herein at a concentration of 133 mg/g, 185 mg/g, 200 mg/g, and 250 mg/g. Exemplary amounts of the API in the composition include, 20 mg/g, 50 mg/g, 75 mg/g 100 mg/g, 120 mg/g, 130 mg/g, 140 mg/g 150 mg/g, 175 mg/g or 200 mg/g.

Expressed alternatively, other embodiments of the composition of the present invention may contain less than 0.1 mg to 200 mg or more of the API, such as 10 mg, 20 mg, 25 mg 30 mg 40 mg 50 mg, 60 mg, 75 mg 100 mg or 200 mg of the API.

In one embodiment, the invention provides a composition as above that is a liquid composition that is suitable for oral administration. In another embodiment, the invention provides a composition as above that is a solid composition that is also suitable for oral administration.

Among preferred water-soluble organic solvents are ethanol, benzyl alcohol, dimethylacetamide, PVP, PG and PEG compounds such as: PEG 300, PEG 400, or PEG 400 monolaurate. The PEG compound in the present compositions is provided in an amount of 5% to 100%, or 5% to 60%, or 10% to 90%, or 20% to 80%, or 30% to 70%, or 40% to 60%, all concentrations being a percentage of volume/volume (v/v). PG may be present at a concentration of 2.5% to 100% (v/v).

The concentration of the PEG compounds in the present compositions can vary depending on what other solubilizers or diluents or excipients are also present. For example, the PEG 300, PEG 400 or PEG 400 monolaurate of the present invention can be at a concentration of 5%, 10%, 12.5%, 15%, 20%, 25%, 30%, 35%, 40%, 45%, 50%, 55%, 60%, 65%, 70%, 75%, 80%, 85%, 90%, or 95%, all such concentrations being given as a percentage of volume/volume (v/v).

The present invention also provides compositions of tetra-O-methyl NDGA
in a cyclodextrin, which includes modified cyclodextrins. The cyclodextrins herein may be α-cyclodextrin, β-cyclodextrin, γ-cyclodextrin, and the modified cyclodextrins may include HP-β-CD and SBE-β-CD, for example. In one embodiment, the present composition contains a modified cyclodextrin in a concentration of 5% to 80%, or 10% to 70%, or 20% to 60%, or 30% to 50%, all such concentrations being given as a percentage of weight/volume (w/v).

In yet another embodiment, the modified cyclodextrins, such as HP-β-CD, is present in the compositions at a concentration of 12.5%, 15%, 20%, 5%, 30%, 35%, 40%, 45%, 50%, 55%, 60%, 65%, 70% or 75%, all such concentrations being given as a percentage of weight/volume (w/v).

Another pharmaceutically acceptable carrier or excipient that may be used alone or with others in the composition of the present invention is an ionic, non-ionic or amphipathic surfactant, such as Cremophor® EL, polysorbates, which are non-ionic surfactants, for example, polysorbate 20 and polysorbate 80, commercially available as Tween® 20 or Tween® 80, TSGS, which is an amphipathic surfactant, among many others. Further examples of suitable surfactants include, glycerol monooleate and an esterified fatty acid, such as those typically made by transesterification of vegetable oils, available in several varieties and grades as Labrafil®, Labrasol®, and Gelucire®, from Gattefosse Corp., Paramus, NJ, U.S.A. The surfactant can be present in any desired effective amount, such as at a concentration of about 1% (v/v) to about 100% (v/v), preferably about 9% (v/v) to about 80% (v/v), and more preferably, about 10% (v/v) to about 50% (v/v). As specific examples, preferred concentrations of a non-ionic surfactant are Tween® 20 at a concentration of about 9% (v/v) to about 100% (v/v) and Tween® 80 of about 33% (v/v) to about 100% (v/v). All percentages of the surfactant are volume percentages (v/v).

Another pharmaceutically acceptable carrier or excipient that may be used alone or with others in the composition of the present invention is a modified cellulose, such as EC, HPMC, MC and CMC. The modified cellulose can be present in any desired effective amount, such as a concentration of about 0.1 % to about 25%, or about 0.5% to about 7.5%, or about 1.0% to about 5%. As specific examples, EC may be present at a concentration of about 5% to about 20%; HPMC may be present at a concentration of about 0.5% to about 1%; MC may be present at a concentration of about 1% to about 3%; and CMC may be present at a concentration of about 1% to about 4%. The percentages of modified cellulose are in weight per volume (w/v).

In another embodiment, the present invention provides a composition as above that contains a PEG compound, such as PEG 400, for example, and a surfactant, such as Tween® 20, for example, as the solubilizing agent and/or excipient.

Another pharmaceutically acceptable carrier or excipient that may be used alone or with others in the composition of the present invention is a water-insoluble lipid, such as an oil, fat emulsion or wax. The water-insoluble lipid carriers can be present in any desired effective amount, such as a concentration of about 10% to about 100%, or about 15% to about 85%, or about 25% to about 75%.

Examples of oils include corn oil, olive oil, peppermint oil, soy bean oil, sesame seed oil, mineral oil and glycerol. In one embodiment, the oil is present at a concentration of about 10% (v/v) to about 100% (v/v). Mixed fat emulsion compositions are available, such as Intralipid® emulsion, as described above. In various embodiments, mixed fat emulsions may be present at a concentration of about 10% (w/v) to about 30% (w/v); and preferably about 20% (w/v).

Examples of suitable waxes are beeswax and carnuba wax. In one embodiment, the wax is present at a concentration of about 5% (w/w) to about 50% (w/w).

The water-insoluble carriers may be used in combination with any or more of the water-soluble carriers, such as PEG compounds, and the surfactants, such as Tween® 20 or Tween® 80. As a further example, the present composition contains a combination of solubilizing agent and/or excipient such as, for example, oil and surfactant, like peppermint oil and Tween® 20 or oil and a PEG compound, such as peppermint oil and PEG 400.

In a further embodiment, the composition of the invention contains oil, a surfactant and a PEG compound, such as, for example, peppermint oil, Tween® 20, and a PEG 400 compound.

As a further example, the present composition includes a combination of oils or oil and wax, such as, for example, peppermint oil and sesame oil, soybean oil and beeswax or olive oil and beeswax. The beeswax may be present at a concentration in the range of between about 5% to 50% (w/w).

Alternatively, in other embodiments, Tween® 80 may be substituted for Tween® 20, and PEG 300 or PEG 400 monolaurate may be substituted for PEG 400, and any of the oils can be substituted for the peppermint oil, soybean oil, and olive oil.

Still other excipients include glycerol monooleate and various types of esterified fatty acids, such as Labrafil®, Labrasol®, and Gelucire® products. These typically are classified as surfactants or emulsifiers, but the Labrasol®, and Gelucire® products are also used as bioavailability enhancing agents.

Labrafil®, and particularly Labrafil® M 1944 CS, can be used as an excipient with an API, such as M₄N, with the API dissolved in a concentration of about 5 mg/mL to about 500 mg/mL. The final product may be a solution, suspension or solid.

Labrasol® can be used as an excipient with an API, such as M₄N, with the API dissolved in a concentration of about 1 mg/mL to about 500 mg/mL. The final product may be a solution, suspension or solid.

Gelucire®, and particularly Gelucire® 44/14, can be used as an excipient with an API, such as M₄N, with the API dissolved in a concentration of about 30 mg/mL to about 500 mg/mL. The final product is a solid.

Glycerol monooleate can be used as an excipient with an API, such as M₄N, with the API dissolved in a concentration of about 0.1 mg/mL to about 500 mg/mL. The final product can be a solution, suspension or a solid.

Combinations of the various carrier components may be used with the API, as noted above. One example of such an embodiment is a composition of 10 mg/ml M₄N in 25% (w/v) PEG 300, 30% (w/v) HP-β-CD, balance of the carrier being "water suitable for injection" into animals ("WFI," which designates a recognized grade of water in the pharmaceutical industry). In this preferred embodiment, the HP-β-CD has 6 to 8 degrees of substitution, but other substitutions in other embodiments are well within the scope of this invention, as noted above.

Yet other excipients and additives, such as bioavailability enhancing agents, may be used in combination with any of the carriers, in the compositions of the present invention. Suitable bioavailability enhancing agents include, for example, eugenol, cinnamaldehyde, lecithin, naringenin, naringin and piperin (also known as piperine), in addition to those mentioned above.

It is to be understood that as long as the catecholic butanes herein are dissolved and remain in solution, suspension or maintained within a semi-solid or solid form of the composition, one or more of the solubilizers or diluents or excipients herein may be added to the composition to optimize delivery of such to a subject in need of such treatment.

Other pharmaceutically acceptable carriers or excipients suitable for use herein are described in a variety of publications. Examples of useful carriers or excipients are described in, for example, Gennaro, A.R. (2003); Ansel, H.C. et al. (2004); Rowe, R.C. et al. (2003); and Garg, S. et al. (2001).

The compositions in liquid form may include a buffer, which is selected according to the desired use of tetra-O-methyl NDGA and may
also include other substances appropriate for the intended use. Those skilled in the art can readily select an appropriate buffer, a wide variety of which are known in the art, suitable for an intended use.

### Therapeutic Methods

The compositions containing tetra-O-methyl NDGA
find use as therapeutic agents or for treatment in subjects in need of such treatment in any number of diseases in which tetra-O-methyl NDGA
can be used.

The present invention provides for a composition for use in the treatment of disease including, for example, proliferative diseases such as benign and malignant cancer, psoriasis and premalignant conditions and neoplasia, such as intraepithelial neoplasia, or dysplasia. The present invention also provides for treatment of diabetes, including type I and type II diabetes, obesity and complications resulting from such, including cardiovascular diseases, stroke and hypertension. The present invention further provides for treatment of inflammatory diseases including rheumatoid arthritis, osteoarthritis, multiple sclerosis, ulcerative colitis, Crohn's disease, chronic obstructive pulmonary disease (COPD) and other immune system associated diseases. Additionally, the present invention provides for treatment of neurological diseases, including central nervous system diseases and neurodegenerative diseases such as Alzheimer's disease, dementia, amyotrophic lateral sclerosis (ALS) and Parkinson's disease. In a further embodiment, the present invention provides for treatment of infections, such as viral infections including viruses that require Sp1 binding for transcription or replication. Examples of such viruses that require Spl binding include: HIV, HTLV, HPV, HSV, HBV; EBV, Varicella-zoster virus, adenovirus, parvovirus and JC virus.

A variety of animal hosts are treatable according to the subject methods, including human and non-human animals. Generally such hosts are "mammals" or "mammalian," where these terms are used broadly to describe organisms which are within the class mammalia, including the orders carnivore (*e.g.,* dogs and cats), rodentia (*e.g.,* guinea pigs, and rats), and other mammals, including cattle, goats, horses, sheep, rabbits, pigs, and primates (*e*.*g*., humans, chimpanzees, and monkeys). In many embodiments, the hosts will be humans. Animal models are of interest for experimental investigations, such as providing a model for treatment of human disease. Further, the present invention is applicable to veterinary care as well.

### Formulations, dosages, and routes of administration

In one embodiment of the invention, an effective amount of the present composition is administered to the host, where an "effective amount" means a dosage sufficient to produce a desired result. In some embodiments, for example, the desired result is at least an inhibition of progression of the neoplasia or dysplasia.

The present invention additionally provides compositions in which

M4N is administered to animals at an oral dose of less than 1 mg/kg to 600 mg/kg weight of the animals, such as humans, for example. Optionally, the animals may be treated with 1 mg/kg, or 50 mg/kg, or 100 mg/kg, or 150 mg/kg, or 200 mg/kg, or 250 mg/kg, or 300 mg/kg, or 350 mg/kg, or 400 mg/kg, or 450 mg/kg, or 500 mg/kg, or 550 mg/kg. Such a dose may be administered once or repeatedly over a period of days or weeks or months. Alternatively, such a dose may also be spread out over a period of time, depending on the health of the subject, the subject's sensitivity, the extent of the disease to be treated, the subject's age and the like.

In one embodiment, the therapeutic compositions herein are made by first dissolving the active pharmaceutical ingredient in a solubilizing agent, with stirring and heating as necessary. Other excipients are added to the solubilized mixture to create the desired proportions in terms of texture and stability requirements. In another embodiment, the active pharmaceutical ingredient may not actually dissolve in the solubilizing agent but may simply be evenly distributed in a suspension. In a further embodiment, the solubilized composition may be lyophilized and used in a powder form. The final oral compositions may be in the form of a liquid solution or suspension or may be a solid powder, tablet, or capsule.

As indicated above, the appropriate dose to be administered depends on the subject to be treated, such as the general health of the subject, the age of the subject, the state of the disease or condition, the weight of the subject, the extent of the disease such as the size of the tumor, for example. Generally, about 0.1 mg to about 500 mg or less may be administered to a child and about 0.1 mg to about 5 grams or less may be administered to an adult. The active agent can be administered in a single or, more typically, multiple doses. Preferred dosages for a given agent are readily determinable by those of skill in the art by a variety of means. Other effective dosages can be readily determined by one of ordinary skill in the art through routine trials establishing dose response curves. The amount of agent will, of course, vary depending upon the particular agent used.

The frequency of administration of the active agent, as with the doses, will be determined by the care giver based on age, weight, disease status, health status and patient responsiveness. Thus, the agents may be administered one or more times daily, weekly, monthly or as appropriate as conventionally determined. The agents may be administered intermittently, such as for a period of days, weeks or months, then not again until some time has passed, such as 3 or 6 months, and then administered again for a period of days, weeks, or months.

In pharmaceutical dosage forms, the active agents may be administered alone or in appropriate association, as well as in combination, with other pharmaceutically active agents or therapeutics including other small molecules, antibodies or protein therapeutics.

In addition, if desired, the carrier or excipient may contain minor amounts of auxiliary substances such as pH adjusting and buffering agents, tonicity adjusting agents, stabilizers, wetting agents or emulsifying agents. Actual methods of preparing such dosage forms are known, or will be apparent, to those skilled in the art. *See, e.g.,* Remington's Pharmaceutical Sciences, 20th ed., Mack Publishing Co. Rawlins EA, (1997). The composition or formulation to be administered will, in any event, contain a quantity of the API adequate to achieve the desired state in the subject being treated.

Kits with multiple or unit doses of the active agent are included in the present invention. In such kits, in addition to the containers holding the multiple or unit doses of the compositions containing tetra-O-methyl NDGA
will be an informational package insert with instructions describing the use and attendant benefits of the drugs in treating pathological condition of interest. Optionally, an applicator for administration of the present composition is included in each kit.

### Example 1. Formulations Containing M₄N in HP-β-CD and/or PEG 300.

In this example, M₄N was prepared as described in PCT/US2004/016117 and was solubilized in a solubilizing agent. The resulting solution was optionally mixed with an excipient and/or a diluent. The solubilizing agent and the excipient may be used interchangeably or in combination with each other. One solubilizing agent or excipient used was endotoxin-controlled hydroxypropyl-β-cyclodextrin ("HP-β-CD") obtained from Research Diagnostics, Inc. (Cat. No. RDI-82004HPB, lot no. H3N188P) (Flanders, NJ, U.S.A.). Another solubilizing agent or excipient used was PEG 300, obtained from Spectrum Chemicals, Inc. (Cat. No. P0108, lot no. TB1228) (Gardena, CA, U.S.A.).

In one embodiment of the invention, HP-β-CD and PEG 300 were present in a single formulation. To make this formulation, M₄N was first dissolved in PEG 300 to form a M₄N in PEG 300 solution ("M₄N/PEG 300"). The M₄N/PEG 300 solution was then added to a pre-made solution of HP-β-CD to form a M₄N solution in a PEG 300 and HP-β-CD (hereafter, a "CPE" formulation).

When preparing the HP-β-CD solution, a volume expansion must be accounted for. For example, for a 40% (w/v) HP-β-CD solution, a volume expansion of 0.7 mL/g (i.e., 0.7 mL of water displaced per gram of HP-β-CD added) must be accounted for.

A 100 mL solution of 40% HP-β-CD for use as a solubilizing agent and/or excipient was made as follows: 65 mL of WFI were placed in a glass beaker containing a stir bar. The beaker was placed on a magnetic plate, and the stir bar was set to stir at medium speed. About forty (40) grams of HP-β-CD were added slowly to the stirring WFI, using a spatula to direct the HP-β-CD to the center of the beaker so as to prevent HP-β-CD crystals from sticking to the beaker wall. The HP-β-CD solution was stirred for about 24 hr or until the HP-β-CD was dissolved completely upon visual inspection. The resulting solution measured about 93 mL. About 7 mL of WFI were added to this resulting solution to obtain 100 mL, producing a final solution of about 40% HP-β-CD. The final solution was stirred for about 1 hr, stored at room temperature, and was protected from light This method of making the modified cyclodextrin solution may be scaled up or down to obtain the desired volume or concentration. Other concentrations or other modified cyclodextrin solutions may be similarly made, for example, by substituting HP-β-CD with other modified cyclodextrins, adjusted for the appropriate concentrations in the process described above.

A 10 mL solution of M₄N at a concentration of about 10 mg/mL in 40% HP-β-CD was made as follows. About 10 mL of the 40% HP-β-CD solution were added to a glass beaker containing a stir bar. The beaker was placed on a magnetic plate and the stir bar was set to stir at medium speed. About 10 mg of M₄N were slowly added to the 40% HP-β-CD in the center of the beaker with the aid of a spatula. The M₄N/40% HP-β-CD mixture was stirred for 2 hr or until all M₄N was uniformly suspended without any clumps being present. The M₄N/40% HP-β-CD mixture was optionally heated at 80°C for about 30 min. (or longer if a larger volume of solution was desired, for example, 1 hr at 80° C for 100 mL of the M₄N/HP-β-CD mixture), or longer as needed to ensure complete dissolution of M₄N. The M₄N/HP-β-CD mixture or solution was observed for presence of any undissolved M₄N by holding the beaker against a white background followed by a dark background, looking for presence of particulates. The final M₄N/40% HP-β-CD solution was stored at room temperature and was kept protected from light. This process may be scaled up or down to obtain the requisite volume or concentration of M₄N. Formulations containing M₄N in other cyclodextrin solutions may be similarly made, such as, for example, by substituting HP-β-CD in the process described above with other cyclodextrins. Results shown in Table 1 demonstrate that M₄N remained in solution after cooling at concentrations of 1 mg/mL and 10 mg/mL in the 40% HP-β-CD formulation for greater than 7 days.

A 100 mL solution of M₄N at a concentration of about 25 mg/mL in PEG 300 was made as follows. About 100 mL of PEG 300 were added to a glass beaker containing a stir bar. The beaker was placed on a magnetic plate and the stir bar was set to stir at medium speed. About 2.5 g of M₄N were slowly added to the PEG 300 in the center of the beaker with the aid of a spatula to prevent M₄N from sticking to the beaker wall. The M₄N/PEG 300 mixture was stirred for 24 hr or until all M₄N had dissolved or was uniformly suspended without any clumps being present. The M₄N/PEG 300 mixture was optionally heated at about 60° C for about 30 min. (or longer if a larger volume of solution was desired, for example, 1 hr at 60° C for 500 mL of the M₄N/PEG 300 mixture), or longer as needed to ensure complete dissolution of M₄N. The M₄N/PEG 300 mixture or solution was observed for presence of any undissolved M₄N by holding the beaker against a white background followed by a dark background, looking for presence of particulates. After all M₄N was observed to be dissolved, the resulting M₄N/PEG 300 solution was used immediately or before the expiration of 48 hr, otherwise crystals or other precipitates might form. If crystals formed, the M₄N /PEG 300 solution could be heated again at 60° C for about 1 hr, with stirring, on a hot magnetic plate until all M₄N was dissolved back in solution. The final M₄N/PEG 300 solution was stored at room temperature and was kept protected from light. This process may be scaled up or down to obtain the requisite volume or concentration of M₄N. Formulations containing M₄N in other PEGs may be similarly made, such as, for example, by substituting PEG 300 in the process described above with PEG 400 or PEG 400 monolaurate.

A 100 mL stock solution of a formulation containing 50% PEG 300 (v/v), 20% HP-β-CD (w/v) and 12.5 mg M₄N was made by adding 50 mL of the 40% pre-made HP-β-CD solution (made as described above) to a glass beaker containing a stir bar on a magnetic plate, with the stir bar stirring at medium speed, and slowly adding 50 mL of the pre-made M₄N/PEG 300 solution (made as described above), for example, at a rate of about 10 mL per min. The M₄N/PEG 300 was added by use of a pipette to the center of the beaker to avoid its sticking to the walls of the beaker and to ensure complete dissolution. The addition of M₄N/PEG 300 to the HP-β-CD solution initially appeared as a white solution, but eventually became clear upon continuous mixing. This recipe may be scaled up or down as appropriate to produce the desired volume or concentration of M₄N/PEG 300 and HP-β-CD. The stock solution was filter-sterilized using a 0.22 µm PVDF membrane, such as a pre-sterilized vacuum driven disposal bottle-top filter membrane obtained from Millipore (Cat. No. SCGV T05 RE) (Billerica, MA, U.S.A.). The filtration process was driven by vacuum force and the filtrate is collected in pre-sterilized 250 mL glass bottles. The bottles were then sealed tightly, stored at room temperature and protected from light. A stock solution of M₄N/PEG 400 or M₄N/PEG 400 monolaurate in HP-β-CD can be similarly made by substituting PEG 300 with PEG 400 or PEG 400 monolaurate in the processes described above.

The M₄N/PEG 300/HP-β-CD, M₄N/PEG 400/HP-β-CD or M₄N/PEG 400 monolaurate/HP-β-CD stock solution made in the foregoing manner can be diluted prior to use *in vitro* or for administration into animals. If dilution is necessary, the stock solution is preferably diluted in WFI, instead of saline, for example, so as to keep the osmolarity down. To make a 100 mL of a 1:1 dilution of the stock solution in WFI, about 50 mL of the stock solution was added to a glass vial. About 50 mL of WFI was added to the 50 mL of the stock solution in the vial to form a diluted solution. The glass vial was closed and the diluted solution was mixed well by shaking and inverting the vial a few times. The diluted solution was filter-sterilized using a 0.22 µm PVDF membrane, such as a pre-sterilized vacuum driven disposal bottle-top filter membrane obtained from Millipore (Cat. No. SCGV T05 RE) (Billerica, MA, U.S.A.). The filtration process was driven by vacuum force and the filtrate was collected in pre-sterilized 250 mL glass bottles. The bottles were then sealed tightly, stored at room temperature and protected from light. This process may be scaled up or down to obtain the requisite volume or dilutions, such as 1:2 or 1:4 dilutions, for example.

A formulation suitable for use as placebo control containing 50% PEG 300 and 20% HP-β-CD can be made as follows. To make a 100 mL solution of the placebo or control formulation, about 50 mL of 40% HP-β-CD are added to a glass beaker containing a stir bar on a magnetic plate. The magnetic plate is set to stir the HP-β-CD solution at medium speed. About 50 mL of PEG 300 are slowly added to the 50 mL of HP-β-CD in the glass beaker by pipette to the center of the beaker to prevent the PEG 300 from sticking to the wall of the beaker. The mixture is stirred for about 1 hr or until mixing is complete. This placebo formulation is filter-sterilized using a 0.22 µm PVDF membrane filter driven by vacuum force. The filtrate is collected in pre-sterilized 250 mL glass bottles. The glass bottles are sealed tightly, stored at room temperature, and kept protected from light. This recipe may be scaled up or down as required to produce the desired concentration and volume amounts. Further, PEG 300 may be substituted with PEG 400 or PEG 400 monolaurate, as desired.

Results of the solubility of M₄N in formulations containing HP-β-CD and/or PEG 300, PEG 400, and in formulations containing HP-β-CD and propylene glycol ("PG"), hydroxypropyl methylcellulose (HPMC), carboxyl methylcellulose (CMC), polyvinyl pyrrolidone (PVP), or Tween® 80 made in accordance to the foregoing or similar processes as well as characteristics of the resulting formulations are shown in Tables 1 - 5, where N represents "No" and Y represents "Yes."

**Table 1. Modified Cyclodextrins as Solubilizing Agents and/or Excipients**

| Excipients | Excipient Concentration (in w/v unless otherwise specified) | Drug Concentration (in mg/mL unless otherwise specified) | Dissolution After Rotation | Dissolution After Heating at X°C | Dissolution After Cool Down | Stability at Room Temperature |
|---|---|---|---|---|---|---|
| α-CD | 15% | 1 | N | N at 90° | | |
| | | 10 | N | N at 90° | | |
| | | 50 | N | N at 90° | | |
| | | 100 | N | N at 90° | | |
| | | | | | | |
| β-CD | 1.50% | 1 | N | N at 90° | | |
| | | 10 | N | N at 90° | | |
| | | | | | | |
| γ-CD | 5% | 1 | N | N at 90° | | |
| | | | | | | |
| HP-β-CD | 50% | 1 | N | Y at 90° | Y | > 7 days |
| | | 10 | N | Y at 90° | Y | > 7 days |
| | | 20 | N | N at 90° | | |
| | | 50 | N | N at 90° | | |
| | | 100 | N | N at 90° | | |
| | 40% | 1 | N | Y at 80° | Y | > 7 days |
| | | 10 | N | Y at 80° | Y | > 7 days |
| | | 12 | N | N at 80° | | |
| | | 14 | N | N at 80° | | |
| | | 16 | N | N at 80° | | |
| | | 20 | N | N at 80° | | |
| | | 50 | N | N at 80° | | |
| | | 100 | N | N at 80° | | |
| | 30% | 1 | N | Y at 90° | Y | > 7 days |
| | | 10 | N | Y at 90° | Y | < 3 days |
| | | 20 | N | N at 90° | | |
| | | 50 | N | N at 90° | | |
| | | 100 | N | N at 90° | | |
| | 20% | 1 | N | Y at 90° | Y | > 7 days |
| | | 10 | N | N at 90° | | |
| | 81.5% (w/w), Lyophilized | 185 mg/g | powder | | | |
| | | | | | | |
| HP-β-CD | 50% in saline | 10 | N | Y at 90° | Y | >7 days |
| | 20% in saline | 1 | N | | | |
| | | 10 | N | | | |
| | | 50 | N | | | |
| | | | | | | |
| HP-β-CD and Propylene Glycol (PG) | 40% HP-β-CD, 2.5% PG (v/v) | 1 | N | N at 80° | | |
| | | 10 | N | N at 80° | | |
| | | | | | | |
| HP-β-CD and CMC | 50% HP-β-CD, 0.5% CMC | 1 | Suspension | | | > 7days |
| | | 20 | Suspension | | | > 7days |
| | | 60 | Suspension | | | > 7days |
| | | | | | | |
| HP-β-CD and PVP | 50% HP-β-CD, 1.25% PVP (w/v) | 1 | N | Y at 90° | Y | < 3 days |
| | | 10 | N | N | | |
| | | 50 | N | N | | |
| | 40% HP-β-CD, 1% PVP (w/v) | 1 | N | N | | |
| | | 10 | N | N | | |
| | | | | | | |
| HP-β-CD and PEG 300 | 27% HP-β-CD, 33% PEG 300 (v/v) | 13.3 | N | Y at 60° | Y | > 7 days |
| | 23% HP-β-CD, 43 PEG 300 (v/v) | 12.9 | N | Y at 60° | Y | > 7 days |
| | 20% HP-β-CD, 50% PEG 300 (v/v) | 12.5 | N | Y at 60° | Y | > 7 days |
| | 15% HP-β-CD, 50% PEG 300 (v/v) | 12.5 | N | Y at 60° | Y | < 7 days |
| | 12.5% HP-β-CD, 50% PEG 300 (v/v) | 12.5 | N | Y at 60° | Y | < 1 day |
| | 13% HP-β-CD, 67% PEG 300 (v/v) | 16.7 | N | Y at 60° | N | |
| | 10% HP-β-CD, 77% PEG 300 (v/v) | 19.3 | N | Y at 60° | N | |
| | 10% HP-β-CD, 25% PEG 300 (v/v) | 6.25 | N | Y at 60° | Y | > 7 days |
| | 6.7% HP-β-CD, 16.7% PEG 300 (v/v) | 4.17 | N | Y at 60° | Y | > 7 days |
| | 5% HP-β-CD, 12.5% PEG 300 (v/v) | 3.13 | N | Y at 60° | Y | < 7 days |
| | | | | | | |
| HP-β-CD and PEG 400 | 32% HP-β-CD, 20% PEG 400 (v/v) | 10 | N | Y at 60° | Y | > 7 days |
| | 30% HP-β-CD, 25% PEG 400 (v/v) | 12.5 | N | Y at 60° | Y | > 7 days |
| | 27% HP-β-CD, 33% PEG 400 (v/v) | 13.3 | N | Y at 60° | Y | > 7 days |
| | 23% HP-β-CD, 43% PEG 400 (v/v) | 12.9 | N | Y at 60° C | Y | > 7 days |
| | 20% HP-β-CD, 50% PEG 400 (v/v) | 12.5 | N | Y at 60° | Y | < 7 days |
| | 15% HP-β-CD, 50% PEG 400 (v/v) | 12.5 | N | Y at 60° | Y | < 3 days |
| | 12.5% HP-β-CD, 50% PEG 400 (v/v) | 12.5 | N | Y at 60° | Y | < 1 day |
| | 40% HP-β-CD, 5% PEG 400 (v/v) | 10 | N | Y at 60° | N | |
| | | | | | | |
| HP-β-CD and Tween® 80 | 27% HP-β-CD, 33% Tween® 80 (v/v) | 13.3 | N | Y at 60° | N | |

All formulations withstand 4°C for 24 hr and 5 min. centrifugation at 5000 rpm without forming visible precipitates. The formulation containing 50% PEG 300, 20% HP-β-CD, 12.5 mg/mL M₄N stock solution withstands 4°C for at least 4 months. Dilutions of the same stock made in 1:1 or 1:2 dilutions also withstand 4°C for at least 4 months.

**Table 2. Formulations of M₄N in PEG 300 and HP-β-CD**

| Undiluted Stock Solutions | | | For each 10 mg of M₄N | | For each 50 mg of M₄N | | For each 100 mg of M₄N | |
|---|---|---|---|---|---|---|---|---|
| PEG 300 (v/v) | HP-β-CD (w/v) | M₄N (mg/mL) | PEG 300 in mL (mg) | HP-β-CD (mg) | PEG 300 in mL (mg) | HP-β-CD (mg) | PEG 300 in mL (mg) | HP-β-CD (mg) |
| 50% | 15% | 12.5 | 0.4 (450) | 120 | 2.0 (2250) | 600 | 4.0 (4500) | 1200 |
| 50% | 20% | 12.5 | 0.4 (450) | 160 | 2.0 (2250) | 800 | 4.0 (4500) | 1600 |
| 43% | 23% | 12.9 | 0.33 (375) | 178 | 1.67 (1875) | 890 | 3.33 (3746) | 1780 |
| 33% | 27% | 13.3 | 0.25 (281) | 200 | 1.25 (1406) | 1000 | 2.5 (2813) | 2000 |

**Table 3. Formulations of M₄N in PEG 400 and HP-β-CD**

| Undiluted Stock Solutions | | | For each 10 mg of M₄N | | For each 50 mg of M₄N | | For each 100 mg of M₄N | |
|---|---|---|---|---|---|---|---|---|
| PEG 400 (v/v) | HOP-β-CD (w/v) | M₄N (mg/mL) | PEG 400 in mL (mg) | HP-β-CD (mg) | PEG 400 in mL (mg) | HP-β-CD (mg) | PEG 400 in mL (mg) | HP-β-CD (mg) |
| 50% | 20% | 12.5 | 0.4 (450) | 160 | 2.0 (2250) | 800 | 4.0 (4500) | 1600 |
| 43% | 23% | 12.9 | 0.33 mL (375) | 178 | 1.67 (1875) | 890 | 3.33 (3746) | 1780 |
| 33% | 27% | 13.3 | 0.25 (281) | 200 | 1.25 (1406) | 1000 | 2.5 (2813) | 2000 |
| 25% | 30% | 12.5 | 0.20 (225) | 240 | 1.0 (1125) | 1200 | 2.0 (2250) | 2400 |
| 20% | 32% | 10.0 | 0.20 (225) | 320 | 1.0 (1125) | 1600 | 2.0 (2250) | 3200 |

**Table 4. Stability of M₄N formulations in PEG 300**

| Formulation | PEG 300 (v/v) | HP-β-CD (w/v) | M₄N (mg/mL) | Stability at Room Temperature |
|---|---|---|---|---|
| A | 50% | 15% | 12.5 | > 3 days |
| B | 50% | 20% | 12.5 | > 5 months |
| C | 43% | 23% | 12.9 | > 5 months |
| D | 33% | 27% | 13.3 | > 5 months |

**Table 5. Stability of M₄N formulations in PEG 400**

| Formulation | PEG 400 (v/v) | CD (w/v) | M₄N (mg/mL) | Stability at Room Temperature |
|---|---|---|---|---|
| E | 50% | 20% | 12.5 | > 6 days |
| F | 43% | 23% | 12.9 | > 5 months |
| G | 33% | 27% | 13.3 | > 5 months |
| H | 25% | 30% | 12.5 | > 5 months |
| I | 20% | 32% | 10.0 | > 5 months |

Similarly, M₄N may be solubilized in other solubilizing agents, such as water-soluble organic solvents including ethanol, PVP (polyvinyl pyrrolidone), propylene glycol or glycerol.

Example 2. Effect of M₄N in DMSO or Combination PEG 300/ HP-β-CD Formulation on Proliferation and Death of Tumor Cells in Culture

M₄N in 10% (w/v) HP-β-CD and 25% (v/v) PEG 300 (hereafter, the "CPE formulation"), M₄N in 30% (w/v) HP-β-CD and 25% (v/v) PEG 300 (hereafter, the "CPE 25/30 formulation") and M₄N in 27% (w/v) HP-β-CD and 33% (v/v) PEG 300 (hereafter, the "CPE 33/27 formulation") was tested for their effects on cell death and proliferation on two different tumor cell lines: HeLa, an HPV-18 positive human cervical cancer cell line, and C-33A, an HPV-negative human cervical cancer cell line. M₄N in DMSO was also tested in parallel. Both tumor cell lines were treated with increasing amounts of M₄N: 0 µM, 20 µM, 40 µM, 60 µM and 80 µM, for 72 hr with the DMSO or the CPE formulation. Each formulation was added to total 1% of the growth media (Minimal Essential Medium with L-glutamine supplemented with 10% fetal bovine serum, 1mM sodium pyruvate, 1x non-essential amino acid solution, and 1,000 IU/mL penicillin/ 1,000 µg/mL streptomycin solution). Control cells were grown under the same conditions and were left untreated. After 72 hr of treatment or no treatment, the total number of cells and the number of live cells in each sample were counted, using the trypan blue exclusion method. The cell proliferation rate and the percentage of dead cells in each sample were analyzed. Results of this experiment are shown in Fig. 1, Fig. 2, and Tables 6 through 12.

Fig. 1 is a graphical representation of the ratio of the number of treated cells/number of untreated cells plotted against increasing concentrations of M₄N in either the DMSO formulation or the CPE formulation for treatment of the C-33A cells and HeLa cells. FIG. 2 is a graphical representation of the percentage of dead cells plotted against increasing concentrations of M₄N and in either DMSO formulation or the CPE formulation for treatment of the two cancer cell lines, C-33A and HeLa cells in culture.

Results show that DMSO alone, in the absence of M₄N, has a significant anti-proliferative effect and some toxic effect, as measured by % of dead cells, on both of the tumor cell lines tested as compared to the untreated controls. In contrast, the CPE formulation alone has an anti-proliferative effect and very little toxic effect on the two tumor cell lines when compared to the untreated controls.

Cell proliferation rate was reduced in both cell lines after treatment with M₄N in either the DMSO formulation or the CPE formulation, when compared to the non-M₄N-treated controls (i.e., 0 µg/mL or 0 of M₄N) in the same formulation. In fact, the anti-proliferative effect appeared to be M₄N dose-dependent in the CPE formulation. In the CPE formulation, for example, about 20 µM or 7.2 µg/mL of M₄N was found to be sufficient to cause about a 50% inhibition in cell proliferation for both tumor cell types. Further increases in the concentration of M₄N in the CPE formulation resulted in further increases in anti-proliferative effect for both cell types.

In general, higher doses M₄N in either the DMSO formulation or the CPE formulation induced higher percentages of cell death for both the C-33A cells and the HeLa cells. However, M₄N in the DMSO formulation was more toxic to cells than the corresponding concentrations of M₄N in the CPE formulation. While the highest concentration of M₄N tested (80 µM or 28.7 µg/mL) in DMSO formulation promoted cell death in about 40% of the cell population, the same concentration of M₄N in the CPE formulation promoted cell death in only about 20% of the cell population in this experiment.

These results were found to be reproducible in both cell lines. Data from this study indicate that M₄N in the CPE formulation has the ability to arrest cell proliferation, similar to M₄N in the DMSO formulation, while inducing less cellular toxicity than the DMSO formulation.

Data were collected from continuing time points to test the effectiveness of the CPE formulation over time. The data showed that after a twelve month period of being stored at 2-8°C the CPE formulation is just as effective as when it is new. The viability of cells remained similar over the twelve months the CPE formulation was stored. Cell death and proliferation rates remained within the same range.

Data were collected to compare the original CPE formulation with the new CPE 25/30 formulation. Studies were conducted at 0 and 3 months to test the effectiveness of the formulation over time as well as to test how well the new formulation works in relation to the old. The data showed that the CPE 25/30 formulation is as effective in inhibiting the growth of tumor cells as is the original CPE formulation. Cell viability was similar between HeLa cells treated with various concentrations of drug using either the CPE formulation or the CPE 25/30 formulation. Cell death and proliferation remained within the same range even over time.

Information was collected comparing the original CPE formulation with the CPE 33/27 formulation at time zero on HeLa cells. The data showed that CPE 33/27 had the same affects on HeLa cells in cell viability, percent of dead cells and proliferation rate.

**Table 6. Effect of M₄N in DMSO or the CPE Formulation on C-33A Cells**

| Treatment | % Viability | % Dead Cells | Proliferation Rate |
|---|---|---|---|
| None | 95.4 | 4.6 | 1.00 |
| 0 µM DMSO | 82.0 | 18.0 | 0.47 |
| 20 µM DMSO | 82.6 | 17.4 | 0.20 |
| 40 µM DMSO | 67.0 | 33.0 | 0.15 |
| 60 µM DMSO | 60.4 | 39.6 | 0.14 |
| 80 µM DMSO | 56.7 | 43.3 | 0.11 |
| 0 µM CPE | 92.8 | 7.2 | 0.79 |
| 20 µM CPE | 93.0 | 7.0 | 0.43 |
| 40 µM CPE | 89.1 | 10.9 | 0.43 |
| 60 µM CPE | 89.4 | 10.6 | 0.22 |
| 80 µM CPE | 77.5 | 22.5 | 0.15 |

**Table 7. Effect of M₄N in DMSO or the CPE Formulation on HeLa Cells at time 0**

| Treatment | % Viability | % Dead Cells | Proliferation Rate |
|---|---|---|---|
| None | 97.7 | 2.3 | 1.00 |
| 0 µM DMSO | 93.7 | 6.3 | 0.43 |
| 20 µM DMSO | 90.1 | 9.9 | 0.25 |
| 40 µM DMSO | 86.8 | 13.2 | 0.25 |
| 60 µM DMSO | 63.9 | 36.1 | 0.13 |
| 80 µM DMSO | 61.4 | 38.6 | 0.02 |
| 0 µM CPE | 96.3 | 3.7 | 1.03 |
| 20 µM CPE | 95.6 | 4.4 | 0.52 |
| 40 µM CPE | 90.6 | 9.4 | 0.28 |
| 60 µM CPE | 80.4 | 19.6 | 0.13 |
| 80 µM CPE | 78.5 | 21.5 | 0.13 |

**Table 8. Effect of M₄N in DMSO or the CPE formulation on HeLa Cells at 9 months**

| Treatment | % Viability | % Dead Cells | Proliferation Rate |
|---|---|---|---|
| None | 95.7 | 4.2 | 1.00 |
| 0µM DMSO | 94.9 | 5.1 | 0.76 |
| 20µM DMSO | 89.3 | 10.7 | 0.19 |
| 40µM DMSO | 91.2 | 8.8 | 0.14 |
| 60µM DMSO | 67.5 | 32.5 | 0.03 |
| 80µM DMSO | 50.0 | 50.0 | 0.02 |
| 0µM CPE | 95.6 | 4.4 | 0.72 |
| 20µM CPE | 68.7 | 31.3 | 0.24 |
| 40µM CPE | 72.8 | 27.2 | 0.10 |
| 60µM CPE | 86.4 | 13.6 | 0.09 |
| 80µM CPE | 88.1 | 11.9 | 0.08 |

**Table 9. Effect of M₄N in DMSO or the CPE formulation on HeLa Cells at 12 months**

| Treatment | % Viability | % Dead Cells | Proliferation Rate |
|---|---|---|---|
| None | 92.1 | 7.9 | 1.00 |
| 0µM DMSO | 90.5 | 9.5 | 0.77 |
| 20µM DMSO | 87.4 | 12.6 | 0.23 |
| 40µM DMSO | 86.4 | 13.7 | 0.04 |
| 60µM DMSO | 64.6 | 35.4 | 0.04 |
| 80µM DMSO | 76.5 | 23.6 | 0.15 |
| OµM CPE | 95.6 | 4.4 | 1.50 |
| 20µM CPE | 96.8 | 3.3 | 0.74 |
| 40µM CPE | 95.0 | 5.0 | 0.21 |
| 60µM CPE | 75.0 | 25.0 | 0.05 |
| 80µM CPE | 52.8 | 47.2 | 0.03 |

**Table 10. Comparison of the CPE formulation and the CPE 25/30 Formulation in HeLa Cells**

| Treatment | % Viability | % Dead Cells | Proliferation Rate |
|---|---|---|---|
| None | 93.5 | 6.6 | 1.00 |
| 0µM CPE | 92.3 | 7.8 | 0.92 |
| 20µM CPE | 93.5 | 6.6 | 0.33 |
| 40µM CPE | 76.7 | 23.4 | 0.08 |
| 60µM CPE | 44.5 | 55.6 | 0.03 |
| 80µM CPE | 43.4 | 56.7 | 0.04 |
| 0µM CPE 25/30 | 90.7 | 9.3 | 0.81 |
| 20µM CPE 25/30 | 90.7 | 9.4 | 0.34 |
| 40µM CPE 25/30 | 89.1 | 11.0 | 0.31 |
| 60µM CPE 25/30 | 77.3 | 22.8 | 0.12 |
| 80µM CPE 25/30 | 54.8 | 45.2 | 0.07 |

**Table 11. Comparison of the CPE formulation and the CPE 25/30 Formulation in HeLa Cells at 3 months**

| Treatment | % Viability | % Dead Cells | Proliferation Rate |
|---|---|---|---|
| None | 95.8 | 4.2 | 1.00 |
| 0µM CPE | 95.0 | 5.0 | 0.91 |
| 20µM CPE | 91.0 | 9.0 | 0.39 |
| 40µM CPE | 83.5 | 16.5 | 0.09 |
| 60µM CPE | 56.9 | 43.1 | 0.03 |
| 80µM CPE | 71.0 | 29.0 | 0.03 |
| 0µM CPE 25/30 | 93.4 | 6.6 | 0.87 |
| 20µM CPE 25/30 | 88.1 | 11.9 | 0.39 |
| 40µM CPE 25/30 | 86.6 | 13.4 | 032 |
| 60µM CPE 25/30 | 76.9 | 23.1 | 0.11 |
| 80µM CPE 25/30 | 64.4 | 35.6 | 0.04 |

**Table 12. Comparison of CPE formulation and the CPE 33/27 formulation in HeLa cells at time zero**

| Treatment | % Viability | % Dead Cells | Proliferation Rate |
|---|---|---|---|
| None | 95.0 | 5.0 | 1.00 |
| 0µM CPE | 96.4 | 3.6 | 1.11 |
| 20µM CPE | 88.1 | 11.9 | 0.35 |
| 40µM CPE | 74.2 | 25.9 | 0.14 |
| 60µM CPE | 73.9 | 26.2 | 0.10 |
| 80µM CPE | 78.4 | 21.6 | 0.11 |
| 0µM CPE 33/27 | 95.4 | 4.7 | 0.92 |
| 20µM CPE 33/27 | 89.2 | 10.9 | 0.43 |
| 40µM CPE 33/27 | 86.4 | 13.6 | 0.19 |
| 60µM CPE 33/27 | 70.7 | 29.3 | 0.10 |
| 80µM CPE 33/27 | 75.0 | 25.0 | 0.09 |

### Example 3. Multiple lots of M₄N can be used and create the same results

Various lots of M₄N were tested to show the effectiveness of the drug of different lots. HeLa cells were treated with increasing amounts of M₄N: 0 µM, 20 µM, 40 µM, 60 µM and 80 µM, for 72 hr with the CPE formulation. Each formulation was added to total 1% of the growth media (Minimal Essential Medium with L-glutamine supplemented with 10% fetal bovine serum, 1mM sodium pyruvate, 1x non-essential amino acid solution, and 1,000 IU/mL penicillin/ 1,000 µg/mL streptomycin solution). Control cells were grown under the same conditions and were left untreated. After 72 hr of treatment or no treatment, the total number of cells and the number of live cells in each sample were counted, using the trypan blue exclusion method. The cell proliferation rate and the percentage of dead cells in each sample were analyzed. Results of this experiment are shown in Tables 13 and 14. These result show that regardless of the lot of M₄N used, the effectiveness of the drug remains the same.

**Table 13. Treatment of HeLa cells with various lots of M₄N (lot EM1001)**

| Treatment | % Viability | % Dead Cells | Proliferation Rate |
|---|---|---|---|
| None | 96.2 | 3.7 | 1.00 |
| 0µM CPE | 96.2 | 3.8 | 0.92 |
| 20µM CPE | 94.9 | 5.1 | 0.25 |
| 40µM CPE | 73.7 | 26.3 | 0.10 |
| 60µM CPE | 44.4 | 55.6 | 0.01 |
| 80µM CPE | 59.8 | 40.2 | 0.01 |

**Table 14. Treatment of HeLa cells with various lots of M₄N (lot EM1002)**

| Treatment | % Viability | % Dead Cells | Proliferation Rate |
|---|---|---|---|
| None | 95.9 | 4.1 | 1.00 |
| 0µM CPE | 96.1 | 3.8 | 0.65 |
| 20µM CPE | 88.0 | 12.0 | 0.32 |
| 40µM CPE | 74.2 | 25.8 | 0.11 |
| 60µM CPE | 41.9 | 58.1 | 0.05 |
| 80µM CPE | 40.3 | 59.7 | 0.03 |

### Example 4. Solubility of M₄N in Modified Celluloses (Reference Example)

A 10 mL solution of 50% HP-β-CD (w/v) and 0.5% hydroxypropyl methylcellulose ("HPMC") (w/v) for use as a solubilizing agent and/or excipient was made as follows: 5.9 mL of WFI were placed in a glass beaker containing a stir bar. The beaker was placed on a magnetic plate, and the stir bar was set to stir at medium speed. Five grams of HP-β-CD were added slowly to the stirring WFI, using a spatula to direct the HP-β-CD to the center of the beaker. The HP-β-CD solution was stirred for about 24 hr or until the HP-β-CD was dissolved completely upon visual inspection. The resulting solution measured about 9.4 mL. About 0.6 mL of WFI was added to this resulting solution to reach 10 mL, to produce a solution of 50% HP-β-CD (w/v). Fifty milligrams of HPMC were added to the 50% HP-β-CD solution and stirred for about 1 hr or until the HPMC was dissolved upon visual inspection. The final solution was stirred for about 1 hr and was then stored at room temperature, protected from light. This method of making modified cyclodextrins with modified celluloses may be scaled up or down to obtain the desired volume or concentration of HP-β-CD/HPMC solution. Other modified cyclodextrin/modified cellulose solutions may be similarly made, for example, by substituting HP-β-CD with other modified cyclodextrins, or HPMC with other modified celluloses, in the process described above.

A 10 mL solution of M₄N at a concentration of about 10 mg/mL in 50% HP-β-CD/0.5% HPMC was made as follows. About 10 mL of the 50% HP-β-CD/0.5% HPMC solution were added to a glass beaker containing a stir bar. The beaker was placed on a magnetic plate and the stir bar was set to stir at medium speed. About 100 mg of M₄N were slowly added to the 50% HP-β-CD/0.5% HPMC in the center of the beaker with the aid of a spatula. The M₄N/50% HP-β-CD/0.5% HPMC mixture was stirred for 24 hr or until all M₄N was uniformly suspended without any clumps being present. The M₄N/50% HP-β-CD/0.5% HPMC mixture was heated at about 90°C for about 30 min. (or longer if a larger volume of solution was desired, for example, 1 hr at 90°C for 500 mL of the M₄N/50% HP-β-CD/0.5% HPMC mixture), or longer as needed to ensure complete dissolution of M₄N. The M₄N/50% HP-β-CD/0.5% HPMC mixture was observed for presence of any undissolved M₄N by holding the beaker against a white background followed by a dark background, looking for presence of particulates. The final M₄N/50% HP-β-CD/0.5% HPMC solution was stored at room temperature and was kept protected from light. M₄N was dissolved in this 50% HP-β-CD/0.5% HPMC formulation at the 1 mg/mL and 10 mg/mL concentrations when heated at 90°C and remained in solution after cool down, with stability at room temperature for greater than 7 days. M₄N did not dissolve in this same formulation at the 50 mg/mL concentration even at 90°C.

The foregoing process may be scaled up or down to obtain the requisite volume or concentration of M₄N. Formulations containing M₄N in other cyclodextrin/cellulose solutions may be similarly made, such as, for example, by substituting HP-β-CD in the process described above with other cyclodextrins, or by substituting HPMC with other modified celluloses.

A 10 mL solution of 5% ethylcellulose ("EC") in ethanol (w/v) for use as a solubilizing agent and/or excipient was made as follows: 10 mL of 100% ethyl alcohol ("EtOH") were placed in a glass beaker containing a stir bar, covered by a round Teflon® cover. The beaker was placed on a magnetic plate, and the stir bar was set to stir at medium speed. Five hundred (500) milligrams of EC was added slowly to the stirring ethanol, using a spatula to direct the EC to the center of the beaker so as to prevent EC powder from sticking to the beaker wall. The EC solution was stirred for about 2 hr or until the EC was dissolved completely upon visual inspection. The final solution was stored at room temperature, and was protected from light.

This method of making modified cellulose solutions may be scaled up or down to obtain the desired volume or concentration. Other modified cellulose solutions may be similarly made, for example, by substituting EC with other modified celluloses, in the process described above.

A 10 mL solution of M₄N at a concentration of about 20 mg/mL in 5% EC (w/v) (the "EC formulation") was made as follows. About 10 mL of 5% EC formulation, made as described above, were added to a glass beaker containing a stir bar. The beaker was placed on a magnetic plate and the stir bar was set to stir at medium speed, and covered with a round Teflon® cover. About 200 mg of M₄N were slowly added to the 5% EC formulation in the center of the beaker with the aid of a spatula to prevent M₄N from sticking to the beaker wall. The M₄N/EC mixture was stirred for 2 hr or until all M₄N had dissolved or was uniformly suspended without any clumps being present. The M₄N/EC mixture was heated at about 60° C for about 30 min. (or longer if a larger volume of solution was desired, for example, 1 hr at 60° C for 500 mL of the M₄N/EC mixture), or longer as needed to ensure complete dissolution of M₄N. The M₄N/EC mixture or solution was observed for presence of any undissolved M₄N by holding the beaker against a white background followed by a dark background, looking for presence of particulates. The final M₄N/EC solution was stored at room temperature and was kept protected from light.

The foregoing process may be scaled up or down to obtain the requisite volume or concentration of M₄N and the heating temperature may be increased or decreased to achieve dissolution of M₄N. Formulations containing M₄N in other modified celluloses may be similarly made, such as, for example, HPMC, MC, and CMC. Results of the solubility of M₄N in the modified celluloses are set forth in Table 15.

Results show that M₄N was not soluble in 2.3% (w/v) HPMC at any of the concentrations tested or upon heating to 90°C. M₄N formed a suspension inl% (w/v) HPMC at the 10 mg/mL concentration. This suspension was stable at room temperature for less than 2 days.

In the presence of HP-β-CD (50% w/v) and HPMC (0.5% w/v), M₄N was solubilized at 90°C and remained in solution upon cooling at the 1 mg/mL and 10 mg/mL concentrations. These solutions were stable at room temperature for more than 7 days. M₄N did not dissolve in the same HP-β-CD (50% w/v) and HPMC (0.5% w/v) composition at the 50 mg/mL concentration even upon heating to 90°C.

In another experiment, M₄N formed suspensions in the absence of heat in the HP-β-CD (50% w/v) and HPMC (0.5% w/v) composition at all concentrations tested, i.e., 1 mg/mL, 10 mg/mL, 20 mg/mL and 50 mg/mL. These suspensions were stable at room temperature for more than 7 days.

Results in Table 15 also show that M₄N was soluble at 1 mg/mL in the EC formulation without application of heat, the solution being stable at room temperature for greater than 3 days. M₄N was soluble at the 10 mg/mL concentration at 40°C and remained in solution after cooling, this solution being stable at room temperature for greater than 3 days. M₄N was soluble in the EC formulation at the 20 mg/mL concentration at 60°C and remained in solution after cooling, this solution being stable at room temperature for greater than 3 days. M₄N at the 30 mg/mL concentration was soluble in the EC formulation at 60°C, but did not remain in solution upon cooling. Higher concentrations of M₄N, such as at the 50 mg/mL or 100 mg/mL levels, were soluble in the EC formulation at 90°C, but did not remain in solution upon cooling.

M₄N also formed suspensions in 1% low viscosity CMC at the 10 mg/mL and 20 mg/mL levels. These suspensions were stable at room temperature for less than 2 days.

**Table 15. Solubility of M₄N in Formulations containing modified celluloses**

| Excipients | Excipient Concentration (in w/v unless otherwise stated) | Drug Concentra -tion (in mg/mL unless otherwise state) | Dissolution After Rotation | Dissolution After Heating | Dissolution After Cool-Down | Stability Time at Room Temperature |
|---|---|---|---|---|---|---|
| | | | | | | |
| HPMC | 2.3% | 1 | N | N at 90°C | | |
| | | 10 | N | N at 90°C | | |
| | | 50 | N | N at 90°C | | |
| | | 100 | N | N at 90°C | | |
| | 1% | 10 | suspension | | | < 2 days |
| HP-β-CD and HPMC | 50% HP-β-CD, 0.5% HPMC | 1 | N | Y at 90°C | Y | > 7 days |
| | | 10 | N | Y at 90°C | Y | > 7 days |
| | | 50 | N | N at 90°C | | |
| | 50% HP-β-CD, 0.5% HPMC | 1 | Suspension | | | > 7 days |
| | | 10 | Suspension | | | > 7 days |
| | | 20 | Suspension | | | > 7 days |
| | | 50 | Suspension | | | > 7 days |
| | 84% HP-β-CD, 1% HPMC, Lyophilized | 150 mg/g | powder | | | |
| | | | | | | |
| EC | 5% in EtOH | 1 | Y | | | > 3 days |
| | | 10 | N | Y at 40°C | Y | > 3 days |
| | | 20 | N | Y at 60°C | Y | > 3 days |
| | | 30 | N | Y at 60°C | N | |
| | | 50 | N | Y at 90°C | N | |
| | | 100 | N | Y at 90°C | N | |
| | | | | | | |
| MC | 2% | 1 | N | N at 90°C | | |
| (Low viscosity) | | 10 | N | N at 90°C | | |
| | | | | | | |
| CMC | 1% | 1 | N | N at 90°C | | |
| (High Viscosity) | | 10 | N | N at 90°C | | |
| | | | | | | |
| CMC | 4% | 1 | N | N at 90°C | | |
| (Low Viscosity) | | 10 | N | N at 90°C | | |
| | 1% | 10 | suspension | | | < 2 days |
| | | 20 | suspension | | | < 2 days |

### Example 5. Solubility of M₄N in Water-Insoluble Lipids and in Water-Soluble Organic Solvents (Reference Example)

A 10 mL solution of M₄N at a concentration of about 50 mg/mL in sesame oil was made as follows. About 10 mL of sesame oil were added to a glass beaker containing a stir bar. The beaker was placed on a magnetic plate and the stir bar was set to stir at medium speed. About 500 mg of M₄N were slowly added to the sesame oil in the center of the beaker with the aid of a spatula to prevent M₄N from sticking to the beaker wall. The M₄N/sesame oil mixture was stirred for about 2 hr or until all M₄N had dissolved or was uniformly suspended without any clump being present. The M₄N/sesame oil mixture was heated at about 60°C for about 30 min. (or longer if a larger volume of solution was desired, for example, 1 hr at 60°C for 500 mL of the M₄N/sesame oil mixture), or longer as need to ensure complete dissolution of M₄N. The M₄N/sesame oil mixture or solution was observed for presence of any undissolved M₄N by holding the beaker against a white background followed by a dark background, looking for presence of particulates. If crystals formed, the M₄N/sesame oil solution could be heated again at 60°C for about 1 hr, with stirring, on a hot magnetic plate until all M₄N was dissolved back in solution. The final M₄N/sesame oil solution was stored at room temperature and was kept protected from light.

The foregoing process may be scaled up or down to obtain the requisite volume or concentration of M₄N and the heating temperature may be increased or decreased to achieve dissolution of M₄N. Formulations containing M₄N in other water-insoluble lipids may be similarly made, such as, for example, by substituting sesame oil in the process described above with corn oil, olive oil, soybean oil, peppermint oil, or other solubilizing agents, and combinations thereof. Results are shown in Table 16.

A 10 g mixture of M₄N at a concentration of about 200 mg/g in 95% olive oil and 5% beeswax was made as follows. About 7.6 mL of olive oil were added to a glass beaker containing a stir bar. The beaker was placed on a magnetic plate and the stir bar was set to stir at medium speed. About 2 g of M₄N were slowly added to the olive oil in the center of the beaker with the aid of a spatula to prevent M₄N from sticking to the beaker wall. The M₄N/olive oil mixture was stirred for about 2 hr or until all M₄N had dissolved or was uniformly suspended without any clumps being present. The M₄N/olive oil mixture was optionally heated at about 60° C for about 30 min. (or longer if a larger volume of solution was desired, for example, 1 hr at 60° C for 500 mL of the M₄N/olive oil mixture), or longer as need to ensure complete dissolution of M₄N. The M₄N/olive oil mixture was observed for presence of any undissolved M₄N by holding the beaker against a white background followed by a dark background, looking for presence of particulates. If crystals formed, the M₄N /olive oil solution could be heated again at 60° C for about 1 hr, with stirring, on a hot magnetic plate until all M₄N was dissolved back in solution. About 400 mg of white beeswax were added to a glass beaker containing a stir bar. The beaker was also placed on a magnetic plate and the stir bar was set to stir at medium speed. The beeswax was heated at about 50°C for about 30 min. (or longer if a larger quantity was desired), or until all the beeswax was melted. The M₄N/olive oil solution was then added to about 400 mg of melted beeswax and stirred and heated at about 50°C for about 30 min or until all the M₄N/olive oil/beeswax mixture had dissolved or was uniformly mixed. The stir bar was removed from the beaker and the M₄N/olive oil/beeswax mixture was allowed to cool down. The final M₄N/olive oil/beeswax mixture was stored at room temperature and was kept protected from light.

This process may be scaled up or down to obtain the requisite volume or concentration of M₄N. Formulations containing M₄N in other water-insoluble lipids may be similarly made, such as, for example, by substituting olive oil in the process described above with corn oil, sesame oil, soybean oil, peppermint oil, lecithin, or other solubilizing agents, and combinations thereof, and substituting beeswax with paraffin, PEG 3350, or other stiffening agents, and combinations thereof. Also, if desired, in combination with any of the carriers, a bioavailability enhancing agent may be included, such as eugenol, cinnamaldehyde, lecithin, naringenin, naringin and piperin (also known as piperine), for example. Results are shown in Table 16.

A 10 mL solution of M₄N at a concentration of about 60 mg/mL in 85% sesame oil and 15% Tween® 20 was made as follows. About 8.5 mL of sesame oil were added to a glass beaker containing a stir bar. The beaker was placed on a magnetic plate and the stir bar was set to stir at medium speed. About 1.5 mL of Tween® 20 was slowly added to the center of the beaker. About 600 mg of M₄N were slowly added to the sesame oil/Tween® 20 mixture in the center of the beaker with the aid of a spatula to prevent M₄N from sticking to the beaker wall. The M₄N/sesame oil/Tween® 20 mixture was stirred for about 2 hr or until all M₄N had dissolved or was uniformly suspended without any clumps being present. The M₄N/sesame oil/Tween® 20 mixture was heated at about 60°C for about 30 min. (or longer if a larger volume of solution was desired, for example, 1 hr at 60°C for 500 mL of the M₄N/sesame oil/Tween® 20 mixture), or longer as need to ensure complete dissolution of M₄N. The M₄N/sesame oil/Tween® 20 mixture was observed for presence of any undissolved M₄N by holding the beaker against a white background followed by a dark background, looking for presence of particulates. The final M₄N/sesame oil/Tween® 20 solution was stored at room temperature and was kept protected from light. If crystals were to form during storage, the M₄N /sesame oil/Tween® 20 solution could be heated again at 60°C, with stirring, on a hot magnetic plate until all M₄N dissolved back in solution.

This process may be scaled up or down to obtain the requisite volume or concentration of M₄N and the heating temperature may be increased or decreased to achieve dissolution of M₄N. Formulations containing M₄N in other water-insoluble lipids combined with non-ionic surfactants, ionic surfactants or water-soluble organic solvents may be similarly made, such as, for example, by substituting sesame oil or Tween® 20 in the process described above with corn oil, olive oil, soybean oil, peppermint oil, Tween® 80, TPGS, lecithin, PEG 300, PEG 400, PEG 400 monolaureate, glycerol, PVP, PG, or other solubilizing agents, and combinations thereof. Results of the solubility of M₄N in water-insoluble lipids are set forth in Table 16.

Formulations containing M₄N in other water-insoluble lipids combined with non-ionic, ionic or amphipathic surfactants or water-soluble organic solvents may be similarly made, such as, for example, by substituting sesame oil in the process described above with corn oil, olive oil, soybean oil, peppermint oil, or mineral oil and substituting Tween® 20 with Tween® 80, TPGS, lecithin, PEG 300, PEG 400, PEG 400 monolaurate, glycerol, PVP, PG, or other solubilizing agents, and combinations thereof. Results of the solubility of M₄N in water-insoluble lipids and the stability of such compositions are set forth in Table 16. Stability in reference to the clear liquid solutions herein refers to the time it takes to form crystallized precipitation in solution. A stable solution is one that is clear and free of particulates for an extended period of time.

Table 16 shows, for example, that M₄N was soluble in corn oil when heated at 60°C at concentrations up to 100 mg/mL and, except at the 100 mg/mL level, M₄N at lower concentrations remained in solution after cooling, the solutions being stable for greater than 3 days at the 1 and 10 mg/mL concentrations, for less than 3 days at the 20, 40 and 50 mg/mL levels and for less than 1 day at the 60 mg/mL level. Further, M₄N was soluble in olive oil at 60°C at the 30 mg/mL level but did not remain in solution after cooling.

In sesame oil, M₄N was soluble at room temperature at the 10 mg/mL level. At 60°C, M₄N was soluble up to the 50 mg/mL concentration, and remained in solution after cooling. The 10 mg/mL and 20 mg/mL solutions were stable at room temperature for more than 3 days, the 30 mg/mL solution was stable at room temperature for less than 3 days, and the 50 mg/mL solution was stable at room temperature for less than 1 day.

In peppermint oil, M₄N was soluble between 1 mg/mL and 125 mg/mL. At concentrations of up to 20 mg/mL, M₄N was soluble without heating. These compositions were stable at room temperature for more than 3 days. M₄N was soluble at higher concentrations, up to the 125 mg/mL level, upon heating at 40°C and remained in solution after cooling. Of these higher concentrations of M₄N in peppermint oil, the 40 mg/mL composition was stable at room temperature for more than 3 days.

M₄N also solubilized in soybean oil at concentrations of up to 50 mg/mL upon heating at 60°C. Of these, only the 10 mg/mL concentration remained in solution upon cooling, and this solution remained stable for more than 7 days.

M₄N was also soluble in mineral oil when heated at 60°C at concentrations of up to 200 mg/mL. The 10 mg/mL and 50 mg/mL compositions remained in solution upon cooling. Of these, the 10 mg/mL solution was stable for more than 7 days at room temperature.

In a combination of 50% peppermint oil and 50% PEG 300, M₄N was soluble up to 125 mg/mL when heated at 35°C. Between the 40 mg/mL and 60 mg/mL levels, M₄N remained in solution after cooling and was stable at room temperature for more than 7 days. In a combination of 60% peppermint oil and 40% PEG 300, M₄N was soluble at 60 mg/mL upon heating at 40°C and remained in solution upon cooling. This composition was stable at room temperature for more than 3 days.

When peppermint oil was combined with PEG 400 at 50% each, M₄N was solubilized up to 125 mg/mL when heated at 40°C. At 40 mg/mL and 60 mg/mL M₄N concentrations, the compound remained in solution upon cooling and the compositions were stable at room temperature for more than 7 days. In a combination of 60% peppermint oil and 40% PEG 400, M₄N was solubilized at 60 mg/mL upon heating at 40°C.

In a combination of 50% peppermint oil and 50% Tween® 20, M₄N was soluble up to 125 mg/mL tested upon heating at 40°C. Of these, M₄N remained in solution after cooling at the 40 mg/mL and 60 mg/mL concentrations.

In another combination, such as peppermint oil and sesame oil, each 50%, M₄N was soluble up to the 60 mg/mL concentration tested. M₄N was soluble at 20 mg/mL at room temperature, the composition being stable at room temperature for more than 3 days. The 40 mg/mL and 60 mg/mL solutions of M₄N remained in solution upon cooling, and were stable for more than 3 days at room temperature.

In yet another combination, containing 33% peppermint oil, 33% Tween® 20 and 33% PEG 400, M₄N was soluble at 60 mg/mL when heated at 40°C, and remained in solution upon cooling. These compositions were stable for more than 3 days.

Table 16 also shows, for example, that M₄N can be solubilized in soybean oil and made into a waxy solid when combined with beeswax. In addition, M₄N can also be solubilized in olive oil and beeswax can be added to turn the composition into a waxy solid.

**Table 16. Solubility of M₄N in water-insoluble Lipids**

| Excipients | Excipient Concentration (in v/v unless otherwise stated) | Drug Concentration (in mg/mL unless otherwise stated) | Dissolution After Rotation | Dissolution After Heating | Dissolution After Cool-Down | Stability Time at Room Temperature |
|---|---|---|---|---|---|---|
| | | | | | | |
| Corn Oil | 100% | 1 | N | Y at 60°C | Y | > 3 days |
| | | 10 | N | Y at 60°C | Y | > 3 days |
| | | 20 | N | Y at 60°C | Y | <3 days |
| | | 40 | N | Y at 60°C | Y | <3 days |
| | | 50 | N | Y at 60°C | Y | < 3 days |
| | | 60 | N | Y at 60°C | Y | < 1 day |
| | | 100 | N | Y at 60°C | N | |
| | | | | | | |
| Olive Oil | 100% | 30 | N | Y at 60°C | N | |
| | | | | | | |
| Sesame Oil | 100% | 10 | Y | | | > 3 days |
| | | 20 | N | Y at 60°C | Y | > 3 days |
| | | 30 | N | Y at 60°C | Y | < 3 days |
| | | 50 | N | Y at 60°C | Y | < 1 day |
| | | | | | | |
| Peppermint Oil | 100% | 1 | Y | | | > 3 days |
| | | 10 | Y | | | > 3 days |
| | | 20 | Y | | | > 3 days |
| | | 40 | N | Y at 40°C | Y | > 3 days |
| | | 60 | N | Y at 40°C | Y | < 3 days |
| | | 100 | N | Y at 40°C | Y | < 1 day |
| | | 125 | N | Y at 404°C | Y | < 1 day |
| | | | | | | |
| Soybean Oil | 100% | 10 | N | Y at 60°C | Y | > 7 days |
| | | 30 | N | Y at 60°C | N | |
| | | 50 | N | Y at 60°C | N | |
| | | | | | | |
| Mineral Oil | 100% | 10 | N | Y at 60°C | Y | > 7 days |
| | | 50 | N | Y at 60°C | Y | < 1 day |
| | | 100 | N | Y at 60°C | N | |
| | | 200 | N | Y at 60°C | N | |
| | | | | | | |
| Olive Oil, Soybean Oil | 80% Olive Oil, 20% Soybean Oil | 60 | N | Y at 70°C | N | |
| | | | | | | |
| Sesame Oil, Tween® 20 and Glycerol | 75% Sesame Oil, 9% Tween® 20, 16% Glycerol | 24.3 | N | Y at 60°C | Y | < 7 days |
| | 10% Oil emulsion in 90% saline | 2.4 | Y | | | <1 day |
| Sesame Oil and Tween® 20 | 89% Sesame Oil,11% Tween® 20 | 29 | N | Y at 60°C | Y | > 7 days |
| | 10% Oil emulsion in 90% saline | 2.9 | Y | | | <3 days |
| | 85% Sesame Oil,15% Tween® 20 | 40 | N | Y at 45°C | N | |
| | 85% Sesame Oil,15% Tween® 20 | 60 | N | Y at 55°C | N | |
| | | | | | | |
| Peppermint Oil, PEG 300 | 50% Peppermint Oil, 50% PEG 300 | 40 | N | Y at 35°C | Y | > 7 days |
| | | 60 | N | Y at 35°C | Y | > 7 days |
| | | 125 | N | Y at 35°C | N | |
| | 60% Peppermint Oil, 40% PEG 300 | 60 | N | Y at 40°C | Y | > 3 days |
| Peppermint Oil, PEG 400 | 50% Peppermint Oil, 50% PEG 400 | 40 | N | Y at 40°C | Y | > 7 days |
| | | 60 | N | Y at 40°C | Y | > 7 days |
| | | 100 | N | Y at 40C | N | |
| | | 125 | N | Y at 40°C | N | |
| | 60% Peppermint Oil, 40% PEG 400 | 60 | N | Y at 40°C | N | |
| | | | | | | |
| Peppermint Oil and Tween® 20 | 50% Peppermint Oil, 50% Tween® 20 | 40 | N | Y at 40°C | Y | > 3 days |
| | | 60 | N | Y at 40°C | Y | > 3 days |
| | | 125 | N | Y at 40°C | N | |
| | | | | | | |
| Peppermint Oil, PEG 400, Glycerol | 40% Peppermint Oil, 40% PEG 400, 20% Glycerol | 52 | N | Y at 40°C | N | |
| | 45% Peppermint Oil, 45% PEG 400, 10% Glycerol | 59 | N | Y at 40°C | N | |
| | | | | | | |
| Peppermint Oil and Sesame Oil | 50% Peppermint Oil, 50% Sesame Oil | 20 | Y | | | > 3 days |
| | | 40 | N. | Y at 40°C | Y | > 3 days |
| | | 60 | N | Y at 40°C | Y | > 3 days |
| | | | | | | |
| Peppermint Oil, Tween® 20, PEG 400 | 33% Peppermint Oil, 33% Tween® 20, 33% PEG 400 | 60 | N | Y at 40°C | Y | > 3 days |
| Soybean Oil, Beeswax | 50% Soybean Oil, 50% Beeswax (w/w) | 100 mg/g | N | Y at 60°C | waxy solid | |
| | 75% Soybean Oil, 25% Beeswax (w/w) | 200 mg/g | N | Y at 60°C | waxy solid | |
| | 90% Soybean Oil, 10% Beeswax (w/w) | 200 mg/g | N | Y at 60°C | waxy solid | |
| | 95% Soybean Oil, 5% Beeswax (w/w) | 200 mg/g | N | Y at 60°C | waxy solid | |
| | | | | | | |
| Olive Oil, Beeswax | 90% Olive Oil, 10% Beeswax (w/w) | 200 mg/g | N | Y at 60°C | waxy solid | |
| | 95% Olive Oil, 5% Beeswax (w/w) | 200 mg/g | N | Y at 60°C | waxy solid | |
| Cinnamaldehyde, Olive Oil, Beeswax | 0.4% (v/v) Cinna-maldehyde, 5% (w/v) Beeswax, 94.5% (v/v) Olive Oil | 60mg/mL | N | Y at 50°C | Creamy solid | |
| Eugenol, Beeswax, Olive Oil | 0.5% (v/v) Eugenol | 100mg/m L | N | Y at 50°C | Creamy solid | |
| Camillia Oil | 100% | 50mg/mL | N | Y at 60°C | N | >7 days |
| | | 100mg/m L | N | Y at 60°C | Creamy solid | |

Table 17 shows the results obtained for the solubility of M₄N in water-soluble organic solvents EtOH, PG, PEG 300, PEG 400, PEG 400 monolaureate, glycerol, PVP, and certain combinations thereof.

**Table 17. Solubility of M₄N in Water-Soluble Organic Solvents**

| Excipients | Excipient Concentration (v/v unless noted otherwise)) | Drug Concentration (in mg/mL) | Dissolution After Rotation | Dissolution After Heating | Dissolution After CoolDown | Stability Time at Room Temperature |
|---|---|---|---|---|---|---|
| | | | | | | |
| Ethanol | 100% | 1 | Y | | | > 3 days |
| | | 10 | N | N at 37°C | | |
| | | | | | | |
| PVP | 15% (w/v) | 1 | N | N at 90°C | | |
| | | 10 | N | N at 90°C | | |
| | | | | | | |
| Propylene Glycol | 100% (w/v) | 1 | N | Y at 55°C | Y | < 1 day |
| | | 10 | N | Y at 55°C | Y | < 1 day |
| | | 20 | N | Y at 55°C | Y | < 1 day |
| | | | | | | |
| PEG 400 | 100% | 25 | N | Y at 50°C | Y | < 7 days |
| | | 30 | N | Y at 50°C | Y | < 3 days |
| | | 40 | N | Y at 50°C | Y | < 1 day |
| | | 50 | N | Y at 60°C | Y | < 1 day |
| | | 100 | N | Y at 60°C | N | |
| | 5% | 10 | N | N at 50°C | | |
| | | | | | | |
| PEG 400 monolaurate | 100% | 20 | N | Y at 50°C | Y | > 3 days |
| | | 50 | N | Y at 50°C | Y | < 1 day |
| | | | | | | |
| PEG 300 | 100% | 25 | N | Y at 50°C | Y | < 7 days |
| | | 30 | N | Y at 50°C | Y | < 3 days |
| | | 40 | N | Y at 50°C | Y | < 1 day |
| | | 50 | N | Y at 60°C | Y | < 1 day |
| | | 100 | N | Y at 60°C | N | |
| | 33% | 10 | N | N at 50°C | | |
| | | | | | | |
| Glycerol | 100% | 1 | N | Y at 70°C | N | |
| | | 10 | N | Y at 70°C | N | |
| | | 20 | N | Y at 70°C | N | |
| | | | | | | |
| Propylene Glycol and Ethanol | 40%Propylene Glycol (w/v), 10% Ethanol (v/v) | 10 | N | N | | |

### Example 6. Solubility of M₄N in Non-Ionic Surfactants (Reference Example)

A 10 mL solution of 20% TPGS (w/v) for use as a solubilizing agent and/or excipient was made as follows: 8 mL of WFI were placed in a glass beaker containing a stir bar. The beaker was placed on a hot magnetic plate, and the stir bar was set to stir at medium speed. The WFI was heated at about 95°C for 5 minutes. Two grams of TPGS was added to another glass beaker, and heated at about 40°C for 15 minutes, or until all the TPGS had melted. The melted TPGS was slowly added to the near boiling WFI, using a spatula to direct the TPGS to the center of the beaker so as to prevent any-TPGS from sticking to the beaker wall. The 20% TPGS solution was stirred for about 24 hr or until the TPGS was dissolved completely upon visual inspection. The final solution was then stored at room temperature, and was protected from light.

This method of making TPGS may be scaled up or down to obtain the desired volume or concentration of TPGS solution. Other TPGS solutions may be similarly made in combination with other non-ionic surfactants, ionic surfactants, amphipathic surfactants, water-soluble organic solvents, or other solubilizing agents in the process described above. Results are shown in Table 18.

A 10 mL solution of M₄N at a concentration of about 60 mg/mL in Tween® 20 was made as follows. About 10 mL of Tween® 20 were added to a glass beaker containing a stir bar. The beaker was placed on a magnetic plate and the stir bar was set to stir at medium speed. About 600 mg of M₄N were slowly added to the Tween® 20 in the center of the beaker with the aid of a spatula to prevent M₄N from sticking to the beaker wall. The M₄N/Tween® 20 mixture was stirred for 2 hr or until all M₄N had dissolved or was uniformly suspended without any clumps being present. The M₄N/Tween® 20 mixture was heated at about 60°C for about 30 min. (or longer if a larger volume of solution was desired, for example, 1 hr at 60°C for 500 mL of the M₄N/Tween® 20 mixture), or longer as need to ensure complete dissolution of M₄N. The M₄N/Tween® 20 mixture or solution was observed for presence of any undissolved M₄N by holding the beaker against a white background followed by a dark background, looking for presence of particulates. The final M₄N/Tween® 20 solution was stored at room temperature and was kept protected from light. If crystals were to form during storage, the M₄N/Tween® 20 solution could be heated again at 60° C for about 1 hr, with stirring, on a hot magnetic plate or until all M₄N was dissolved back in solution.

This process may be scaled up or down to obtain the requisite volume or concentration of M₄N and the heating temperature may be increased or decreased to achieve dissolution of M₄N. Formulations containing M₄N in other non-ionic surfactant, ionic surfactants or amphiphilic molecules may be similarly made, such as, for example, by substituting Tween® 20 in the process described above with Tween® 80, other solubilizing agents, and combinations thereof. Results of the solubility of M₄N in Tween® 20, Tween® 80, and a combination of Tween® 20 and PEG 400 are shown in Table 18.

Table 18 shows that M₄N was soluble in Tween® 20 or Tween® 80 at a concentration of 1 mg/mL at room temperature. The M₄N in Tween® 20 solution ("M₄N/Tween® 20") was stable at room temperature for more than 7 days, while the M₄N in Tween® 80 solution ("M₄N/Tween® 80") was stable for more than 3 days of observation. Higher concentrations of M₄N were soluble in Tween® 20 or Tween® 80 at 50°C. Further, M₄N remained in solution after cooling at concentrations of up to 60 mg/mL in Tween® 20 or up to 50 mg/mL in Tween® 80, while becoming insoluble upon cooling at the 80 mg/mL and 100 mg/mL levels in Tween® 20. The 10 mg/mL and 20 mg/mL M₄N/Tween® 20 solutions were observed to be stable at room temperature for greater than 7 days. The 40 mg/mL and 80 mg/mL M₄N/Tween® 20 solutions were observed to be stable at room temperature for less than 3 days. For the M₄N/Tween® 80 solutions, the 10 mg/mL solution was observed to be stable at room temperature for more than 3 days while the 50 mg/mL solution was stable at room temperature for less than 1 day.

Results also show that M₄N was soluble in a combination of 50% Tween® 20 and 50% PEG 400, up to a concentration of 60 mg/mL of M₄N tested when heated at 65°C. M₄N remained in solution in these formulations upon cooling, the solutions being stable at room temperature for more than 3 days.

Combinations of PEG 400 and Tween® 20 were tested at various heating temperatures, at the amount of time heat was added, the method and time of cooling and the concentration of drug added.

A 10mL solution was made starting with 10mL of glycerol monooleate ("Glymo") added to a glass beaker. The beaker was placed on a magnetic plate and the stir bar was set to stir at medium speed. It was heated to 60°C for 30 minutes to allow all of the drug to dissolve. The mixture was observed for the presence of any undissolved M₄N by holding the beaker against a white background followed by a dark background, looking for presence of particulates. The Glymo mixture was cooled to room temperature with heating and the mixture became a suspension. It was stored at room temperature, protected from light. The suspension remained stable for two weeks and then it began to separate. It could be recombined with shaking.

**Table 18. Solubility of M₄N in Non-Ionic Surfactants**

| Excipients | Excipient Concentration | Drug Concentration (in mg/ml unless otherwise stated) | Dissolution After Rotation | Dissolution After Heating | Dissolution After Cool Down | Stability at Room Temperature |
|---|---|---|---|---|---|---|
| | | | | | | |
| TPGS | 20% (w/v) | 1 | N | Y at 30°C | Creamy | |
| | | 10 | N | Y at 60°C | Creamy | |
| | | 125 | N | Y at 60°C | Creamy | |
| | | 200 | N | Y at 60°C | Creamy | |
| | 20% (w/v) | 60 | Suspension | | | > 3 days |
| | | 100 | Suspension | | | > 3 days |
| | | | | | | |
| Tween® 20 | 100% (v/v) | 1 | Y | | | > 7 days |
| | | 10 | N | Y at 50°C | Y | > 7 days |
| | | 20 | N | Y at 50°C | Y | > 7 days |
| | | 40 | N | Y at 50°C | Y | < 3 days |
| | | 60 | N | Y at 50°C | Y | < 3 days |
| | | 80 | N | Y at 50°C | N | |
| | | 100 | N | Y at 50°C | N | |
| Tween® 80 | 100% (v/v) | 1 | Y | | | > 3 days |
| | | 10 | N | Y at 50°C | Y | > 3 days |
| | | 50 | N | Y at 50°C | Y | <1 day |
| | | | | | | |
| Tween® 20, PEG 400 | 50% Tween® 20 (v/v), 50% PEG 400 (v/v) | 30 | N | Y at 65°C | Y | > 3 days |
| | | 40 | N | Y at 65°C | Y | > 3 days |
| | | 50 | N | Y at 65°C | Y | > 3 days |
| | | 60 | N | Y at 65°C | Y | > 3 days |
| | | | | | | |
| Tween® 20, PEG 400, Beeswax | 47.5% Tween® 20 (v/v), 47.5% PEG 400 (v/v), 5% Beeswax (w/v) | 200 mg/g | N | Y at 65°C | Waxy solid | > 3 days |
| | | | | | | |
| TPGS. PEG 400 | 10% TPGS (w/v), 50% PEG 400 (v/v) | 50 | suspension | | | > 7 days |
| PEG 400, Tween® 20, Cinnamadehyde | 0.4% (v/v) Cinnamadehyde 49.8%(v/v) PEG 400, 49.8% (v/v) Tween® 20 | 60mg/mL | N | Y at 60°C | Y | >4 days |
| Tween® 20, PEG 400, Eugenol | 0.4% (v/v) Eugenol, 49.8% (v/v) Tween® 20, 49.8% (v/v) PEG 400 | 60mg/mL | N | Y at 50°C | Y | >2 days |
| Tween® 20, PEG 400, Naringin | 0.4% (vlv) Naringin, 49.8% (v/v) PEG 400, 49.8% (v/v) Tween® 20 | 60mg/mL | N | Y at 50°C | Suspension | |
| | | 120mg/mL | N | Y at 50°C | Suspension | |
| | | 200mg/mL | N | Y at 50°C | Suspension | |
| | | 300mg/mL | N | Y at 50°C | Suspension | |
| PEG 400, Tween® 20, Lecithin | 25%(w/v) Lecithin, 37.5% (v/v) PEG 400, 37.5% (v/v) Tween® 20 | 60mg/mL | N | Y at 50°C | Y | > 14 days |
| PEG 400, Tween® 20, Glycerol Monostearate | 3% (w/v)Glycerol Monostearate, 48.5% (v/v) PEG 400, 48.5% (v/v) Tween® 20 | 60mg/mL | N | Y at 80°C | Y | >24 hours |
| PEG 400, Tween® 20, Naringenin | 0.3% (w/v) Naringenin, 49.8%(v/v) PEG 400 49.8% (v/v) Tween® 20 | 60mg/mL | N | Y at 50°C | Creamy Solid | |
| PEG 400, Tween® 20, Peppermint Oil, Cinnamaldeh yde | 0.4% (v/v) Cinnamaldehyd e, 4.6% (v/v) Peppermint Oil, 47.5% (v/v) PEG 400, 47.5% (v/v) Tween® 20 | 60mg/mL | N | Y at 60°C | Y | >2 days |
| PEG 400, Tween® 20, Peppermint Oil | 5% (v/v) Peppermint Oil, 47.5% (v/v) Tween® 20, 47.5% (v/v) PEG 400 | 60mg/mL | N | Y at 80°C | Y | >2 days |
| PEG 400, Tween® 20, Piperin | 0.3% (v/v) Piperin, 49.8% (v/v) PEG 400, 49.8% (v/v) Tween® 20 | 60mg/mL | N | Y at 55°C | Suspension | >9 days |
| | | | | | | |
| Tween® 20, PEG 400, Beeswax | 47.5% Tween® 20 (v/v), 47.5% PEG 400 (v/v), 5% Beeswax (w/v) | 200 mg/g | N | Y at 65°C | Waxy solid | 3 days |
| Tween® 20, PEG 400, PEG 3350, Beeswax | 47.5% (v/v) Tween® 20, 47.5% (v/v) PEG 400, 2.5% (w/v) Beeswax 2.5% (w/v) PEG 3350 | 60mg/mL | N | Y at 65°C | Y | >4 days |
| Tween® 20, PEG 400, PEG 3350, Beeswax, Naringenin | 50.3% (v/v) Tween® 20, 44.6% (v/v) PEG 400, 2.4% (w/v) Beeswax, 2.4% (w/v) PEG 3350, 0.3% (w/v) Naringenin | 60mg/mL | N | Y at 65°C | Y | >4 days |
| Tween® 20, PEG 400, PEG 3350, Beeswax, Lecithin | 50% (v/v) Tween® 20, 40% (v/v) PEG 400, 5% (w/v) Lechitin, 2.5% (w/v) PEG 3350,2.5% (w/v) Beeswax | 60mg/mL | N | Y at 60°C | Creamy Solid | |
| | | | | | | |
| TPGS, PEG 400 | 10% TPGS (w/v), 50% PEG 400 (v/v) | 50 | suspension | | | > 7 days |
| Glycerol Monoleate | 100% (w/v) | 0.10 | N | Y at 60°C | Suspension | Remains in suspension for up to 10 days. Can be mixed back together |
| | | 0.25 | N | Y at 60°C | Suspension | Remains in suspension for up to 10 days. Can be mixed back together |
| | | 1.00 | N | Y at 60°C | Suspension | Remains in suspension for up to 10 days. Can be mixed back together |
| | | 5.0 | N | Y at 60°C | Suspension | Remains in suspension for up to 10 days. Can be |
| | | | | | | mixed back together |
| | | 10.0 | N | Y at 60°C | Suspension | Remains in suspension for up to 10 days. Can be mixed back together |
| | | 12.0 | N | Y at 60°C | Suspension | Remains in suspension for up to 10 days. Can be mixed back together |
| | | 15.0 | N | Y at 60°C | Suspension | Remains in suspension for up to 10 days. Can be mixed back together |
| | | 17.5 | N | Y at 60°C | Suspension | Remains in suspension for up to 10 days. Can be mixed back together |
| | | 20.0 | N | Y at 60°C | Suspension | Remains in suspension for up to 10 days. Can be mixed back together |
| | | 25.0 | N | Y at 60°C | Suspension | Remains in suspension for up to 10 days. Can be mixed back together |
| | | 30.0 | N | Y at 60°C | Suspension | Remains in suspension for up to 10 days. Can be mixed back together |
| | | 40.0 | N | Y at 60°C | Suspension | Remains in suspension for up to 10 days. Can be mixed back together |
| | | 50.0 | N | Y at 60°C | Suspension | Remains in suspension for up to 10 days. Can be mixed back together |
| | | 60.0 | N | Y at 60°C | Suspension | Remains in suspension for up to 10 days. Can be mixed back together |
| | | 65.0 | N | Y at 60°C | Suspension | Remains in suspension for up to 10 days. Can be mixed back together |
| | | 70.0 | N | Y at 60°C | Suspension | Remains in suspension for up to 10 days. Can be mixed back together |
| | | 80.0 | N | Y at 60°C | Suspension | Remains in suspension for up to 10 days. Can be mixed back together |
| | | 100.0 | N | Y at 60°C | Suspension | Remains in suspension for up to 10 days. Can be mixed back together |
| | | 200.0 | N | Y at 60°C | Suspension | Remains in suspension for up to 10 days. Can be mixed back together |
| | | 300.0 | N | Y at 60°C | Suspension | Remains in suspension for up to 10 days. Can be mixed back together |
| PEG 400, Tween® 20, Glycerol Monooleate | 25% (v/v) PEG 400, 25% (v/v) Tween® 20, 50% (v/v) Glycerol Monooleate | 60mg/mL | N | Y at 50°C | N-separated into 2 separate layers | |
| | 30% (v/v) PEG 400, 30% (v/v) Tween® 20, 40% (v/v) Glycerol Monooleate | 60mg/mL | N | Y at 50°C | N-separated into 2 separate layers | |
| Glycerol Monostearate | 15% (w/v) solution in water | 60mg/mL | N | N | | |
| Gelucire® 44/14 | 100% | 60mg/mL | N | Y at 60°C | Creamy solid | |
| | | 200mg/mL | N | Y et 60°C | Creamy solid | |
| | | 300mg/mL | N | Y at 60°C | Creamy solid | |
| Labrasol® | 100% | 50mg/mL | N | Y at 60°C | Y | > 4 days |
| | | 60mg/mL | N | Y at 60°C | Y | > 4 days |
| | | 70mg/mL | N | Y at 60°C | Y | > 4 days |
| | | 80mg/mL | N | Y at 60°C | Y | > 4 days |
| | | 90mg/mL | N | Y at 60°C | suspension | |
| | | 100mg/mL | N | Y at 60°C | suspension | |
| | | 200mg/mL | N | Y at 60°C | suspension | |
| Labrafil® | 100% | 60mg/mL | N | Y at 60°C | Y | > 4 days |
| | | 200mg/mL | N | Y at 60°C | Creamy solid | |

### Example 7. (Reference Example)

The melting point of the 47.5% (v/v) Tween® 20, 47.5% (v/v)PEG 400,2.5% (w/v) PEG 3350,2.5% (w/v) Beeswax of Example 6 (see Table 18) was determined for several different concentrations of M₄N, as follows:

| Concentration of M₄N | Melting Temperature |
|---|---|
| 0% | 47.5°C |
| 5% | 47.5°C |
| 10% | 48°C |
| 20% | 55°C |
| 30% | 55°C |
| 40% | 62°C |
| 50% | 62°C |

### Example 8. Lyophilized Formulations Containing M₄N in HP-β-CD (Reference Example)

A 120 mg lyophilized powder of M₄N at a concentration of about 185 mg/g (w/w) in HP-P-CD was made as follows. Equal molar amounts of HP-β-CD and M₄N were used to increase the complexation rate between HP-β-CD and M₄N. About 98 mg of HP-β-CD and about 22.2 mg M₄N were mixed together in a 1.5 mL-sized polypropylene tube. About 0.2 mL WFI was added to the mixture in the polypropylene tube containing the HP-β-CD/M₄N powder mixture and vortexed for 1 minute to produce a HP-β-CD/M₄N suspension in water. The HP-β-CD/M₄N suspension was frozen at -20°C for 24 hours. The HP-β-CD/M₄N suspension was then centrifuged at 1,400 rpm under vacuum at 60°C for about 2 hours to remove all the water from the suspension. The dry powder of HP-β-CD/M₄N complex weighed about 120 mg. This HP-β-CD/M₄N powder complex may be then dissolved or resuspended in water or other solubilizing agents. The final M₄N/HP-β-CD powder complex was stored at room temperature and was kept protected from light. This process may be scaled up or down to obtain the requisite volume or concentration of M₄N. Formulations containing M₄N with other cyclodextrins may be similarly made, such as, for example, by substituting HP-β-CD in the process described above with other cyclodextrins. Results shown in Table 1 demonstrate that a powder complex of HP-β-CD/M₄N was obtained after lyophilization of the HP-β-CD/M₄N suspension consisting of about 81.5% HP-β-CD and 18.5% M₄N.

A 400 mg lyophilized powder of M₄N at a concentration of about 150 mg/g (w/w) in HP-β-CD/HPMC was made as follows. About 1 mL of a 50% HP-β-CD/0.5% HPMC solution, made as described above, was added to a 1.5 mL-sized polypropylene tube. About 60 mg M₄N were added to the polypropylene tube containing the HP-β-CD/HPMC suspension and vortexed for 1 minute to produce a HP-β-CD/HPMC/M₄N suspension. The HP-β-CD/HPMC/M₄N suspension was frozen at -20°C for 24 hours. The HP-β-CD/HPMC/M₄N suspension was then centrifuged at 1,400 rpm under vacuum at 60°C for about 5 hours to remove all the water from the suspension. The dry powder of HP-β-CD/HPMC/M₄N complex weighed about 400 mg. This HP-β-CD/HPMC/M₄N powder complex may be then dissolved or resuspended in water or other solubilizing agents. The final M₄N/HP-β-CD/HPMC powder complex was stored at room temperature and was kept protected from light. This process may be scaled up or down to obtain the requisite volume or concentration of M₄N. Formulations containing M₄N with other cyclodextrins/cellulose solutions may be similarly made, such as, for example, by substituting HP-β-CD in the process described above with other cyclodextrins, or by substituting HPMC with other modified celluloses. Results shown in Table 15 demonstrate that a powder complex of HP-β-CD/HPMC/M₄N was obtained after lyophilization of the HP-β-CD/HPMC/M₄N suspension consisting of about 84% HP-β-CD, 1% HPMC and 15% M₄N.

### Example 9. Absorption of M₄N in Sprague Dawley Rats upon oral administration of a Liquid Formulation (Reference Example)

Ten groups of male rats (n = 5 per group) (age = 8-10 weeks) were exposed by oral gavage to a single 500 mg/kg dose of M₄N in excipient to determine the optimal oral liquid excipient. The animals were fasted overnight prior to dosing. The excipients/formulation tested were: (a) HP-β-CD + HPMC; (b) HP-β-CD + CMC; (c) TPGS; (d) TPGS + PEG 400; (e) Tween® 20; (f) PEG 400 + Tween® 20; (g) PEG 400 + Tween® 20 + peppermint oil; (h) peppermint oil + PEG 400; (i) peppermint oil + Tween® 20; (j) peppermint oil + sesame oil, as shown in Table 19, using the formulations made as previously described. Blood was collected from each animal via the jugular vein at the following time points: predose, 0.5, 1,2 and 3 hr post dosing. Concentrations of M₄N in serum were determined by LC/MS/MS, LC meaning liquid chromatography, MS meaning mass spectrometry. This method analyzes M₄N that has been separated by LC, then detected and quantified by two consecutive rounds of MS.

As shown in Fig. 3 and Table 20, M₄N absorption varied between excipient formulations. Absorption was highest for PEG 400 + Tween® 20, which was higher than PEG 400 + Tween® 20 + peppermint oil, which was higher than peppermint oil + Tween® 20, which was higher than peppermint oil + PEG 400, which was higher than peppermint oil + sesame oil, which was higher than or equal to Tween® 20, which was higher than or equal to HP-β-CD + CMC, which was higher than HP-β-CD + HPMC, which was higher than TPGS, which was higher than TPGS + PEG 400.

In Table 20, four formulations were indicated as stable suspensions: HP-β-CD + HPMC, HP-β-CD + CMC, TPGS, and TPGS + PEG 400. The criterion used for designating those suspensions as "stable" was that the suspensions did not show precipitation of their contents (the ingredients did not settle down at the bottom of the beaker from the suspension). The other formulations in the rat study were clear liquid solutions, not suspensions, and did not have any floating ingredients.

**Table 19. Oral Formulations for Absorption Studies**

| GROUP | ITEM DESCRIPTION | M₄N CONCENTRATION |
|---|---|---|
| | | |
| Rat Excipient Study | | |
| | | |
| | 500 mg/mL HP-β-CD, | |
| 1 | 5 mg/mL HPMC | 60 mg/mL* |
| | 500 mg/mL HP-β-CD, | |
| 2 | 5 mg/mL CMC | 60 mg/mL* |
| 3 | 200 mg/mL TPGS | 60 mg/mL* |
| | 100 mg/mL TPGS | |
| 4 | 50% PEG400 (v/v) | 60 mg/mL* |
| 5 | 100% Tween® 20 (v/v) | 60 mg/mL |
| 6 | 50% PEG400 (v/v), 50% Tween® 20 (v/v) | 60 mg/mL |
| 7 | 33% PEG400 (v/v), 33% Tween® 20 (v/v), 33% Peppermint Oil (v/v) | 60 mg/mL |
| 8 | 50% Peppermint Oil (v/v), 50% PEG400 (v/v) | 60 mg/mL |
| 9 | 50% Peppermint Oil (v/v), 50% Tween® 20 (v/v) | 60 mg/mL |
| 10 | 50% Peppermint Oil (v/v), 50% Sesame Oil (v/v) | 60 mg/mL |
| | | *Stable suspensions |
| | | |
| GROUP | ITEM DESCRIPTION | M₄N CONCENTRATION |
| | | |
| Dog Excipient Study | | |
| | | |
| 1 | M₄N | powder |
| 2 | Lyophilized HP-β-CD | 185 mg/g (w/w) |
| 3 | 20% TPGS (w/v) | 133 mg/g (w/w) |
| 4 | 95% Soybean Oil (v/v), 5% Beeswax (w/v) | 200 mg/g (w/w) |
| 5 | 95% Olive Oil (v/v), 5% Beeswax (w/v) | 200 mg/g (w/w) |

**Table 20. Absorption of M₄N in Rats^{a} upon Oral Delivery**

| | Concentration (ng/mL) | | | |
|---|---|---|---|---|
| Excipient/Formutation | 0.5 hour | 1 hour | 2 hours | 3 hours |
| HP-β-CD + HPMC | 238±39 ^{b} | 398±89 | 305±48 | 482±128 |
| UP-β-CD + CMC | 403±116 | 601±125 | 470±96 | 448±94 |
| TPGS | 127+45 | 222±57 | 216+57 | 156±36 |
| TPGS + PEG400 | 87±14 | 120±17 | 77±13 | 130±45 |
| Tween® 20 | 424±138 | 620±211 | 581±149 | 371±61 |
| PEG400 + Tween® 20 | 1033±287 | 1300±425 | 1371±429 | 1598±379 |
| PEG400 + Tween®20 + Peppermint Oil | 557±169 | 1023±335 | 1404±719 | 977±546 |
| Peppermint Oil + PEG400 | 896±228 | 876±156 | 832±239 | 487±133 |
| Peppermint Oil + Tween® 20 | 851±346 | 502±96 | 1017±683 | 1233±722 |
| Peppermint Oil + Sesame Oil | 445±193 | 496±137 | 657±143 | 655±264 |

| | | | | |
|---|---|---|---|---|
| ^{a} n = 5 males/group ^{b} Values are expressed as means ± standard errors | | | | |

### Example 10. Absorption of M4N in Beagle Dogs Upon Oral Administration (Reference Example)

Five groups of dogs (n = 2 per group, one male and one female) (age = approximately 6 - 9 months old) were exposed to a single 100 mg/kg dose of M₄N in excipient to determine the optimal oral solid excipient. The composition of M₄N in excipient was encapsulated in size 12 hard gelatin capsules prior to oral administration. The animals were fasted overnight prior to dosing. The excipients/formulations tested are set forth in Table 19 and were: (a) no excipient, where M₄N was encapsulated; (b) HP-β-CD; (c) TPGS; (d) soybean oil + beeswax; and (e) olive oil + beeswax. Blood was collected from each animal via the jugular vein at the following time points: predose, 0.5 hr, 1 hr, 1.5 hr, 2 hr, 4 hr, 6 hr and 8 hr post dosing. Concentrations of M₄N in serum were determined by Liquid Chromatography combined with tandem Mass Spectroscopy (LC/MS/MS).

As shown in Fig. 4, absorption of M₄N varied depending on the excipient/formulation. Absorption was highest for the formulation containing olive oil + beeswax, which was higher than that containing HP-β-CD, which was higher than that containing TPGS, which was higher than that containing soybean oil + beeswax, which was higher than M₄N without an excipient.

Although the foregoing examples are illustrated with M₄N.

### Example 11. Collection of Samples for Determination of the Pharmacokinetics of Micronized and Non-Micronized M₄N After Oral Administration to Dogs (Reference Example)

The objective of this study was to evaluate the Pharmacokinetic profile of MaN when administered in a micronized or non-micronized form via oral gavage dosing in beagle dogs. The test articles were prepared in glycerol monooleate at 60 mg/mL. The study design is summarized in Table 21.

**Table 21. Study Design for Non-Micronized and Micronized M₄N Testing in Dogs**

| Group /Phase | Number of Animals | Test Article | Dose Route | Target Dose Level (mg/kg) | Target Dose Concentration (mg/mL) | Target Dose Volume (mL/Kg) |
|---|---|---|---|---|---|---|
| 1/1 | 3M,3F | Non-micronized M₄N | Oral | 100 | 60 | 1.67 |
| 1/2 | 3M,3F | Micronized M₄N | Oral | 100 | 60 | 1.67 |

| | | | | | | |
|---|---|---|---|---|---|---|
| M Male F Female Note: There was a washout period of approximately 7 days between phases. | | | | | | |

Blood (approximately 2 mL) was collected from a jugular vein into Monoject 2 mL red top tubes containing no anticoagulant predose and at 0.25 hr, 0.5 hr, 1 hr, 2 hr, 4 hr, 8 hr, 12 hr, 24 hr, and 36 hr postdose.

Absorption of M₄N occurred after administration of both formulations. Mean pharmacokinetic parameters are presented in Table 22, where Cₘₐₓ is the maximum concentration of M₄N absorbed and AUC represents the areas under curve, relating to the total M₄N absorbed. Absorption was higher after administration of micronized M₄N compared to non-micronized M₄N. However concentration curves tended to be more consistent among animals after administration of non-micronized M₄N (Figs. 5A and 5B, non-logarithmic and logarithmic scales, respectively, showing absorption of non-micronized M₄N; and Figs. 6A and 6B, non-logarithmic and logarithmic scales, respectively, showing absorption of micronized M₄N).

**Table 22. Mean Pharmacokinetic Parameters following administration of M₄N**

| | Non-Micronized EM-1421 | Micronized EM-1421 |
|---|---|---|
| Cₘₐₓ(ng/mL) | 3051 | 4574 |
| Standard Deviation | 380 | 2088 |
| %CV | 12.5 | 45.8 |
| Tₘₐₓ(h) | 3.3 | 3.7 |
| Standard Deviation | 1.0 | 0.8 |
| %CV | 31.0 | 22.3 |
| AUC₀₋₃₆ₕ (ng*h/mL) | 31470 | 37773 |
| Standard Deviation | 5607 | 9920 |
| %CV | 17.8 | 26.3 |
| AUC_{infinity} | 32572 | 40113 |
| Standard Deviation | 5683 | 8809 |
| %CV | 17.4 | 22.0 |

### Example 11. Oral Fed/Fasted IV/Oral Pharmacokinetic Comparison of Two Formulations of M₄N in Beagle Dogs

The purpose of this study was to evaluate serum levels achieved after a single 75 mg/kg dose of preformulated M₄N administered orally (by gavage or capsule) under fed or fasted conditions or administered IV. Three dogs/sex were administered EM-1421 in glycerol monooleate ("Glymo"), M₄N in Tween® 20/PEG400/Naringin ("TPN"), or M₄N in 20% hydroxypropyl-β-cyclodextrin (HPβCD) in 50% PEG 300 ("CPE"). Glymo and TPN were also administered as non-concentrated (60 mg/mL) or concentrated (300 mg/g, w/w), as indicated in the legends to Figs. 7B and 8B.

All animals survived throughout the study. Clinical observations included diarrhea, emesis, and ataxia (ataxia occurred in 1 female after IV dosing and lasted for approximately 1 hour).

Serum levels of M₄N varied greatly with formulation and condition. Table 23 summarizes these findings. Serum levels were lowest after administration with concentrated Glymo or TPN. Cₘₐₓ was highest after administration of TPN (non-concentrated, fasted condition) (Table 23 and Figs. 7A and 8A, non-logarithmic scales showing serum levels of M₄N orally administered to male and female dogs, respectively). AUCs were highest after administration of Glymo (non-concentrated, fasted condition) (Table 23 and Figs. 7B and 8B, logarithmic scales showing serum levels of M₄N orally administered to male and female dogs, respectively). AUCs achieved after oral administration of Glymo (non-concentrated, fasted condition) were 35% (males) and 47% (females) of the AUCs achieved after IV dosing, where IV dosing is assumed to be 100% (Table 23).

**Table 23. Pharmacokinetics of Formulated M₄N in Dogs after Single 75 mg/kg Dose**

| Formulation | Administration | Condition | Sex | Cₘₐₓ (ng/mL) | AUC (ng*h/mL) |
|---|---|---|---|---|---|
| CPE | IV | Fed | M | 37671 | 134103 |
| CPE | IV | Fed | F | 40580 | 137547 |
| TPN | Oral (Gavage) | Fasted | M | 6256 | 20792 |
| TPN | Oral (Gavage) | Fasted | F | 8221 | 41975 |
| TPN | Oral (Gavage) | Fed | M | 5712 | 37722 |
| TPN | Oral (Gavage) | Fed | F | 3144 | 22758 |
| Glymo | Oral (Gavage) | Fasted | M | 5157 | 46938 |
| Glymo | Oral (Gavage) | Fasted | F | 5439 | 64362 |
| Glymo | Oral (Gavage) | Fed | M | 2807 | 24367 |
| Glymo | Oral (Gavage) | Fed | F | 5300 | 57168 |
| TPN - Conc | Oral (Capsule) | Fasted | M | 890 | 7070 |
| TPN - Conc | Oral (Capsule) | Fasted | F | 695 | 9635 |
| TPN - Conc | Oral (Capsule) | Fed | M | 1232 | 9559 |
| TPN - Conc | Oral (Capsule) | Fed | F | 638 | 5924 |
| Glymo-Conc | Oral (Capsule) | Fasted | M | 1020 | 5092 |
| Glymo-Conc | Oral (Capsule) | Fasted | F | 1001 | 13185 |
| Glymo-Conc | Oral (Capsule) | Fed | M | 1851 | 12890 |
| Glymo-Conc | Oral (Capsule) | Fed | F | 817 | 10074 |

### Example 12. Oral (Gavage) Repeated Dose Pharmacokinetic Study of M4N in Rats (Reference Example)

The purpose of this study was to provide information on pharmacokinetics of a 500 mg/kg dose of M₄N in ten different excipient formulations. Fifty Crl:(CD)SD male and female rats were assigned to ten dosage groups, five rats per sex per group. Samples were prepared having carriers as follows that were administered to the following groups of subjects:

| | |
|---|---|
| Group I | PEG400/Tween® 20 |
| Group II | PEG400/Tween20® /Naringin |
| Group III | PEG400/Tween20® /Naringenin |
| Group IV | PEG400/Tween20® /Glyceryl Monostearate |
| Group V | Glycerol Monooleate |
| Group VI | PEG400/Tween20® /Piperine |
| Group VII | PEG400/Tween20® /PEG3350/Beeswax/Naringin |
| Group VIII | PEG400/Tween20® /Cinnamaldehyde |
| Group IX | PEG400/Tween20® /Peppermint Oil |
| Group X | PEG400/Tween20® /PEG3350/Beeswax |

Each formulation of M₄N was administered orally via gavage on three occasions. The concentration of the M₄N was 60 mg/mL administered at a dosage volume 8.33 mL/kg, based on the most recent body weight. The rats were given the first dose (day 1 of study) after being fasted overnight followed by a 72 hour washout (recovery) period. On day 5 of study (DS 5), the second dose of test article was administered to non-fasted rats followed by a one week washout (recovery) period. On DS 6, the rats were placed on a high fat diet (approximately 10% fat content versus approximately 5% in a standard diet). On DS 12, the non- fasted rats were administered the third dose. The rats were observed for viability at least twice daily and for clinical observations and general appearance weekly during the acclimation period. The rats were also examined for clinical observations, deaths before dosage and at approximately hourly intervals for the first four hours postdosage and at the end of the normal working day on the first day of dosage administration, and at approximately 2 hours on subsequent days of dosage administration. These observations were also recorded once daily on non dosing days and during the post dosage period. Body weights were recorded at least weekly during the acclimation period, daily during the dosage and at sacrifice terminal weight. Feed consumption values were recorded weekly during the dosage period and at sacrifice (feed left value). On days 1, 5 and 12 of the study, blood samples (approximately 0.5 mL each) were collected via the lateral tail vein from each rat assigned to the study. The time of each blood collection was recorded in the raw data. Blood samples were collected from each rat on days 1, 5, and 12 of study at the following time points: prior to dosage, 15 minutes post dosage, and 1 and 4 hours post dosage. The samples were transferred into serum separator tubes and spun in a centrifuge. The resulting serum was transferred into polypropylene tubes labeled with the protocol number, Sponsor study number, rat number, group number, dosage level, day of study, collection interval, date of collection, species, generation and storage conditions. All samples were immediately frozen on dry ice and maintained frozen (-68°C to -78°C) until shipment for analysis.

All rats survived to their scheduled sacrifice. Urine stained abdominal fur occurred in four of the five female rats administered PEG 400/Tween® 20/peppermint oil (Group IX). This sign occurred on DSs 2 to 3 and did not persist. Soft or liquid feces was the only clinical observation that occurred in at least a few rats in each group with the exception of PEG 400/Tween® 20/peppermint oil (Group IX) rats. All groups had average weight gains during the study. Weight gains for the male and female rats for DSs 6 to 13 when the high fat diets were fed were always less than for DSs 1 to 6 when the standard diet was fed. Weight gains were generally comparable among the groups with the exception of the female rats on DSs 6 to 13 in the PEG 400/ Tween® 20 and PEG 400/ Tween® 20/PEG 3350/beeswax dosage groups (Groups I and X, respectively) that had reduced body weight gains. Absolute and relative feed consumption values were comparable among the groups throughout the study. No gross lesions related to the test article formulations were observed at necropsy. Absorption of M₄N occurred from all vehicles. Blood levels of rats on a high fat diet were always higher than those achieved on a standard diet or after fasting. The highest absorption occurred in the PEG 400/ Tween® 20/naringin group after administration of a high fat diet. The highest absorption occurred in the PEG 400/ Tween® 20/naringenin group after administration of a standard diet. The highest absorption occurred in the PEG 400/ Tween® 20/peppermint oil group after fasting conditions. The highest exposure levels were generally achieved within one hour post dose, except for the glycerol monooleate formulation, which appeared to be continuing to rise at four hours post dose.

In conclusion, all test article formulations were tolerated with no mortality and all groups gained weight. Soft and liquid feces was the most common clinical observation but no effect on feed consumption occurred for any vehicle. Blood levels of M₄N were always higher when rats were fed the high fat diet. Peak exposure occurred within one hour post dosage with the exception of the glycerol monooleate formulation.

### Example 13. A human phase 0, three-way crossover microdose pharmacokinetic study of ¹⁴C-labelled M₄N in eight healthy male subjects

This study was designed to assess the absorption of M₄N when administered to humans as a sub-therapeutic dose either as a single oral dose under fed and fasted conditions or a single intravenous dose (Regimens A - C, respectively, in Table 24). The study was a three-way crossover study design in a target population of healthy male subjects and consisted of three study periods of approximately 35 hours duration, each separated by a minimum period of at least 7 days between dosing. During the course of each study period, pharmacokinetic blood samples were taken at specified time points after dosing and urine was collected over pre-defined time intervals. The subjects were able to leave the clinical unit after the completion of study specific procedures at 24 hours post-dose.

In this study, M₄N was administered to humans in a 100 µg quantity. M₄N was lightly-labelled with ¹⁴C (3.3 kBq per 100 µg) and administered to healthy volunteers. Each oral administration of M₄N consisted of 0.1 mg of ¹⁴C labeled M₄N and 376.8 mg of glycerol monooleate in a size 0 gelatin capsule. The single intravenous infusion of M₄N consisted of 0.1 mg/mL ¹⁴C labelled M₄N, 30% (w/v) HP-β-CD, and 25% (v/v) PEG diluted with water to 1mL for bolus injection. Following collection of blood and urine from each subject, samples were analyzed for ¹⁴C content using Accelerator Mass Spectrometry (AMS) to determine the maximum concentration of M₄N (Cₘₐₓ) that occurred at time Tₘₐₓ, the overall area under the curve (AUC) that corresponds to the overall absorption of M₄N at the times of testing (AUC₀₋ₜ) and overall (AUC_{0-∞}), the terminal half-life (T_{½}) of each sample and the relative and overall bioavailability (Fᵣₑₗ and F), of the oral dose of M₄N compared to the IV dose of M₄N. The mean ± SD values of pharmacokinetic parameters for ¹⁴C are presented in Table 24. The baseline levels of M₄N were corrected for any residual M₄N remaining in the subjects following the periods between dosing so as not to inflate the levels of M₄N for any subsequent dosing.

**Table 24. Mean ± SD values of pharmacokinetic parameters for ¹⁴C (Baseline Corrected)**

| Parameter | Regimen A (oral, fed) | Regimen B (oral, fasted) | Regimen C (Intravenous) |
|---|---|---|---|
| Cₘₐₓ (pmol/L) | 5.94 ± 1.33 | 9.29 ± 1.40 | 10.31 ± 1.77 |
| Tₘₐₓ (hours) | 2.50^{a} | 1.00^{a} | 0.08^{a} |
| AUC₀₋ₜ (pmol.h/L) | 88.0 ± 19.4 | 117.2 ± 9.2 | 96.6 ± 9.23 |
| AUC_{0-∞} (pmol.h/L) | 625.6 ± 183.7 | 416.6 ± 142.6 | 707.7 ± 380.4 |
| T½ (hours) | 96.5 ± 20.7 | 50.9 ± 22.4 | 116.2 ± 64.0 |
| Fᵣₑₗ (%) | 75.1 ± 16.2 | - | - |
| F(%) | 91.2 ± 19.1 | 122.1 ± 14.5 | - |

| | | | |
|---|---|---|---|
| ^{a}Media Regimen A: 100µg ¹⁴C-labelled M₄N (3.3 kBq) administered as a single oral dose following a high fat breakfast. Regimen B: 100µg ¹⁴C-labelled M₄N (3.3 kBq) administered as a single oral dose following an overnight fast. Regimen C: 100µg ¹⁴C-labelled M₄N (3.3 kBq) administered as 1mL bolus intravenous solution following an overnight fast. | | | |

For the oral doses, the Cₘₐₓ values for total 14C were lower following oral administration of 100µg ¹⁴C-labelled M₄N after a high fat breakfast (Cₘₐₓ=5.94 ± 1.33pmol/L) than following oral administration after an overnight fast (Cₘₐₓ=9.29 ± 1.40pmol/L). Tₘₐₓ tended to occur later in fed subjects than in fasted subjects. In fed subjects, Tₘₐₓ, the time of occurrence of Cₘₐₓ, was highly variable and ranged from 1.5 to 24 hours post-dose, and in fasted subjects Tₘₐₓ generally occurred at 1 hour post-dose (range 0.50 to 2.00 hours). The AUC₀₋ₜ values were generally lower in fed subjects than in fasted subjects.

Following the intravenous dose the maximum concentration (Cₘₐₓ=10.31 ± 1.77pmol/L) occurred, as expected, at the first sampling time (0.08 hours post-dose) in eight of the ten subjects. In two subjects, Tₘₐₓ was 0.17 hours post-dose. The AUC₀₋ₜ values for total ¹⁴C plasma concentrations were slightly lower following the single oral dose in fed subjects than following the intravenous dose. Conversely, the corresponding AUC₀₋ₜ values were slightly higher following the single oral dose in the fasted subjects than following the intravenous dose.

The study formulations were well tolerated in both oral and intravenous administrations. There were no serious or severe adverse events and no subjects discontinued because of a study treatment related adverse event. No clinically significant changes in vital signs or ECGs were seen.

In conclusion regarding this study, the apparent absorption of M₄N was very high following oral administration in the fed and fasted state. In the presence of food, the rate and extent of absorption were lower compared with the fasted state and the time of occurrence of Cₘₐₓ was prolonged in the fed state. These conclusions are made on the assumption that the orally administered doses of ¹⁴C-labelled M₄N were not degraded prior to absorption.

### Example 14. Additional Studies of Solubility of M4N in Water-soluble Organic Solvents (Reference Example)

The solubility of N₄N in combinations of water-soluble organic solvents as noted in Table 25 was evaluated up to 48 hours. Following 2, 24 and 48 hours incubation at room temperature samples were analyzed via Reverse Phase-HPLC ("RP-HPLC") to quantify the solubility of M₄N. To prepare M₄N samples, 200 µL of 100 mg/mL M₄N dissolved in acetone were placed in 1.5 mL polypropylene microtubes. The solvent was allowed to evaporate at room temperature for 48 hours until the samples were completely dry.

The water miscible organic solvent formulations were prepared in 15 mL polypropylene centrifuge tubes. 10 mL of each formulation was prepared. Each solvent was added on the basis of weight using its respective density at 25°C. Following brief mixing, each formulation was filtered through a 0.45 µm surfactant free cellulose acetate ("SFCA") filter into a fresh 15 mL tube. Formulations were kept at room temperature until ready for use.

400 µL of each formulation combination (Table 25, where Benz = benzyl alcohol; Crem = Cremophor® EL; DMA = dimethylacetamide; T80 = Tween® 80) were added to the microtube, enabling a maximum solubility of 50 mg/Ml M₄N. The M₄N solubility was evaluated by RP-HPLC at 2, 24 and 48 hours incubation at room temperature. At each time point, the samples were centrifuged for 2 minutes at 13,000 rpm to pellet any solid M₄N. As noted in Table 25, over half of the formulation conditions examined were able to solubilize M₄N to a concentration of greater than 10 mg/Ml. The solubility of M₄N in glycerol at 2 and 48 hours was not detectable.

**Table 25. M₄N solubility in water miscible organic solvents up to 48 hours**

| | M₄N (mg/Ml) | | |
|---|---|---|---|
| | Time (hrs) | | |
| Composition | 2 | 24 | 48 |
| 100% EtOH | 7.20 | 7.22 | 7.91 |
| 100% PG | 1.10 | 1.33 | 1.76 |
| 100% PEG300 | 5.81 | 10.37 | 11.52 |
| 100% Glycerol | XXX | 0.08 | XXX |
| 100% Crem | 1.19 | 7.51 | 12.48 |
| 50% EtOH, 50% PG | 3.64 | 4.15 | 4.43 |
| 50% EtOH, 50% PEG300 | 10.94 | 15.07 | 16.61 |
| 50% EtOH, 50% Glycerol | 1.14 | 1.53 | 1.60 |
| 50% EtOH, 50% Crem | 13.95 | 17.58 | 18.49 |
| 50% EtOH, 50% T80 | 15.91 | 19.28 | 19.68 |
| 50% PG, 50% PEG300 | 2.65 | 4.88 | 5.30 |
| 50% PG 50% Glycerol | 0.13 | 0.48 | 0.51 |
| 50% PG, 50% Crem | 2.94 | 6.33 | 7.20 |
| 50% PG, 50% T80 | 5.07 | 8.16 | 8.41 |
| 48%EtOH, 50%PG, 2%Benz | 4.42 | 4.79 | 4.92 |
| 48%EtOH, 50%PEG300, 2%Benz | 14.51 | 15.78 | 16.49 |
| 48%EtOH, 50%Glycerol, 2%Benz | 1.25 | 1.66 | 1.73 |
| 48%EtOH, 50%Crem, 2%Benz | 14.02 | 18.17 | 18.51 |
| 48%EtOH, 50%T80, 2%Benz | 16.17 | 19.55 | 19.96 |
| 44%EtOH, 50%PG, 6%DMA | 4.80 | 5.48 | 2.93 |
| 44%EtOH, 50%PEG300, 6%DMA | 15.12 | 18.61 | 18.27 |
| 44%EtOH, 50%Glycerol, 6%DMA | 1.43 | 1.90 | 2.01 |
| 44%EtOH, 50%Crem, 6%DMA | 14.85 | 20.42 | 21.08 |
| 44%EtOH, 50%T80, 6%DMA | 17.72 | 22.41 | 23.00 |
| 18%EtOH,30%PG,40%PEG300,10%T80,2%Benz | 7.84 | 9.79 | 10.16 |
| 18%EtOH,30%PG,40%Glyc,10%T80,2%Benz | 0.58 | 1.01 | 1.15 |
| 18%EtOH,30%PG,40%Crem,10%T80,2%Benz | 8.64 | 11.78 | 11.86 |
| 14%EtOH,30%PG,40%PEG300,10%T80,6%DMA | 9.40 | 10,55 | 11.30 |
| 14%EtOH,30%PG,40%Glyc,10%T80,6%DMA | 0.85 | 1.24 | 1.48 |
| 14%EtOH,30%PG,40%Crem,10%T80,6%DMA | 9.03 | 12.08 | 12.28 |
| 28%EtOH,30%PG,30%PEG300,10%T80,2%Benz | 8.87 | 10.19 | 10.27 |
| 28%EtOH,30%PG,30%Glyc,10%T80,2%Benz | 1.86 | 2.22 | 2.53 |
| 28%EtOH,30%PG,30%Crem,10%T80,2%Benz | 8.98 | 11.35 | 11.28 |
| 24%EtOH,30%PG,30%PEG300,10%T80,6%DMA | 9.91 | 10.80 | 10.67 |
| 24%EtOH,30%PG,30%Glyc,10%T80,6%DMA | 1.63 | 2.26 | 2.52 |
| 24%EtOH,30%PG,30%Crem,10%T80,6%DMA | 10.42 | 11.25 | 12.48 |

### Example 15. M₄N Solubility in aqueous solutions (Reference Example)

The solubility of M₄N in aqueous solutions containing either hydroxypropyl HP-β-CD or sulfobutyl ether β-cyclodextrin (SE-β-CD) (Captisol®, CyDex, Inc., Lenexa, KS, U.S.A.) was evaluated up to 48 hours at room temperature described above in Example 14. Ten 50 % solutions of HP-β-CD and SE-β-CD were prepared on a weight to volume basis. The M₄N for use in the samples was prepared as set forth in Example 14. Between 1.0 g and 5.0 g of either compound was weighed into a 10 mL volumetric flask on an OHAUS Analytical Plus Balance. Each sample was q.s. to 10 mL with water for injection (WFI). Following a 1 hour incubation at 40°C, the preparations were filtered through a 0.45 µm SFCA filter into a fresh 15 mL tube. Preparations were kept at room temperature until ready for use.

As shown in Table 26, the solubility of M₄N in WFI, 0.9% saline, 5% dextrose (D5W) was below the quantitation limit of the RP-HPLC method throughout the course of this study. Note the increased M₄N solubility as a function of HP-β-CD and Captisol® concentration and time.

**Table 26. M₄N solubility in aqueous solutions up to 48 hours**

| | M₄N(mg/mL) | | |
|---|---|---|---|
| | Time (hrs) | | |
| Composition | 2 | 24 | 48 |
| WFI | XXX | XXX | XXX |
| 0.9% Saline | XXX | XXX | XXX |
| D5W | XXX | XXX | XXX |
| 10% HP-β-CD | 0.35 | 0.45 | 0.46 |
| 20% HP-β-CD | 0.66 | 0.91 | 0.88 |
| 30% HP-β-CD | 1.38 | 1.97 | 2.02 |
| 40% HP-β-CD | 1.89 | 3.01 | 3.23 |
| 50% HP-β-CD | 2.52 | 4.95 | 4.98 |
| 10% Captisol® | 0.48 | 0.74 | 0.94 |
| 20% Captisol® | 0.76 | 1.45 | 1.39 |
| 30% Captisol® | 1.57 | 2.87 | 2.69 |
| 40% Captisol® | 1.42 | 3.90 | 4.15 |
| 50% Captisol® | 1.17 | 4.49 | 6.41 |

More than 20 formulation conditions using water miscible organic solvents were able to solubilize M₄N to a concentration of greater than 10 mg/mL. The solubility of M₄N in WFI, 0.9% saline, D5W was below the detection limit of the RP-HPLC method. The solubility of M₄N increases as a function of HP-β-CD and Captisol® concentration and time.

### Example 16. M₄N Solubility in Hydroxypropyl β-cyclodextrin (Reference Example)

The aqueous solubility of M₄N at various concentrations of HP-β-CD was evaluated by the method reported by Higuchi and Connors (1965). Briefly, M₄N was accurately weighed and added in quantities exceeding its aqueous solubility were gently rotated (∼12 rpm) at room temperature with aqueous solutions of HP-β-CD in increasing concentrations (0-350 mmol/L), for a period of 48 hours. The M₄N/HP-β-CD solutions were then filtered through a 0.45 µm SFCA filter and analyzed via RP-HPLC.

Although most drug/cyclodextrin complexes are thought to be inclusion complexes, cyclodextrins are also known to form non-inclusion complexes and complex aggregates capable of dissolving drugs through micelle-like structures. The phase-solubility profiles did not verify formation of inclusion complexes, but only detail how the increasing concentration of cyclodextrin influences drug solubility. Formation of the M₄N/HP-β-CD complex is non-linear, but accurate determination of stoichiometry (as well as stability constants) was not studied in the experiments of this Example, but could be determined by other means such as NMR or potentiometry.

### Example 17. M₄N Stability in HP-β-CD/PEG 300 buffer solutions

The stability of 10 mg/mL M₄N (prepared in a ratio of 75:25 40% HP-β-CD: 40 mg/mL M₄N PEG 300) in 15 mM buffer solutions was evaluated following incubation at 60°C.

Buffered 40 % solutions of HP-β-CD were prepared on a weight to volume basis. The M₄N for use in the samples was prepared as set forth in Example 14. 2.0 g HP-β-CD was weighed into 5 mL volumetric flasks on an OHAUS Analytical Plus Balance. 1 mL of a 100 mM buffer solution was added to each flask. Each sample was q.s. to 5 mL with WFI. Following a 1 hour incubation at 40°C, the preparations were filtered through a 0.45 µm SFCA filter into a fresh 15 mL tube. Preparations were kept at room temperature until ready for use.

750 µL 40% HP-β-CD buffered solution was placed in 1.5 mL polypropylene microtubes. 250 µL of a 40 mg/mL M₄N PEG 300 was added to each microtube enabling a solubility of 10 mg/mL M₄N. After gentle inversion of the sample tubes, the pH of each solution was measured using a Orion, Model 420A pH meter. An initial aliquot was removed for RP-HPLC analysis. Test samples were then placed in a Precision 60°C incubator. The M₄N stability was evaluated by RP-HPLC. At each time point, the samples were centrifuged for 2 minutes at 13,000 rpm to pellet any solid M₄N.

As shown in Table 27, a slight decrease in the concentration of the various M₄N solutions is observed following the 14 day incubation at 60°C. RP-HPLC data did not reveal an increase in sample impurities which could account for the magnitude of decrease in the concentration of M₄N. However, a pH-dependent change was observed in the M₄N impurities, yet these impurities made up less than 0.1 % of the total peak area. Little if any changes are observed in the apparent sample pH during the incubation period (Table 27).

**Table 27. M₄N stability in HP-β-CD/PEG solutions up to 14 days at 60°C**

| Composition | M₄N (mg/mL) | | | | |
|---|---|---|---|---|---|
| | Time (days) | | | | |
| | **0** | **2** | **6** | **10** | **14** |
| WFI, 30% HP-β-CD, 25% PEG300 | 10.4 | 10.1 | 9.9 | 9.7 | 9.6 |
| 15 mM Phosphate, pH 3 30% HP-β-CD, 25% PEG300 | 10.3 | 10.1 | 9.6 | 9.7 | 9.6 |
| 15 mM Phosphate, pH 4 30% HP-β-CD, 25% PEG300 | 10.5 | 10.1 | 9.6 | 9.7 | 9.6 |
| 15 mM Acetate, pH 5 30% HP-β-CD, 25% PEG300 | 10.6 | 10.2 | 9.6 | 9.7 | 9.6 |
| 15 mM Acetate, pH 6 30% HP-β-CD, 25% PEG300 | 10.5 | 10.1 | 9.7 | 9.7 | 9.6 |
| 15 mM Phosphate, pH 7 30% HP-β-CD, 25% PEG300 | 10.5 | 10.3 | 9.7 | 9.7 | 9.7 |
| 15 mM Phosphate, pH 8 30% HP-β-CD, 25% PEG300 | 10.6 | 10.4 | 9.7 | 9.8 | 9.6 |
| 15 mM Phosphate, pH 9 30% HP-β-CD, 25% PEG300 | 10.4 | 10.2 | 9.7 | 9.8 | 9.7 |
| 15 mM Borate, pH 10 30% HP-β-CD, 25% PEG300 | 10.5 | 10.1 | 9.7 | 9.6 | 9.6 |
| 15 mM Borate, pH 11 30% HP-β-CD, 25% PEG300 | 10.5 | 10.3 | 9.8 | 9.5 | 9.4 |

A uniform decrease in stability regardless of apparent sample pH or buffer, as indicated by a loss in the recovery of M₄N, was observed after 14 days of incubation at 60°C.

### Example 18 - M₄N Stability in 11-14 mg/mL PEG300/HP-β-CD solutions

To support manufacturing specifications, this study examined the 24-hour room temperature stability of combinations of PEG300/HP-β-CD at varying M₄N target concentrations. Stock samples of M₄N stocks were prepared in 100% PEG 300 at drug concentrations of 33-56 mg/mL at 60°C as follows.

M₄N bulk drug was solubilized to concentrations of 33, 44, 48, 52, 55 and 56 mg/mL (w/w) in PEG 300 using the procedure set forth in Examples 14 and 17, following incubation of at least 2 hours at 60°C. Vigorous vortexing and mixing were necessary for complete solubilization of M₄N above 44 mg/mL. The M₄N stock solutions were filtered through a 0.45 µm SFCA filter and used within 30 minutes of preparation. Separately, a solution of 40% (w/v) HP-β-CD was prepared in sterile WFI and filtered. Combinations of the 40% HP-β-CD stock and the M₄N/PEG 300 stocks were combined in 1.5 mL polypropylene microtubes. The M₄N solubility was evaluated by RP-HPLC at 2 and 24 hours incubation at room temperature.

The requisite amount of M₄N stock was added to 40% HP-β-CD to yield the final concentrations of drug and excipients listed in Table 28. Samples were gently rotated (∼12 rpm) at room temperature. At 2 and 24 hours of incubation, samples were centrifuged 2 minutes at 13,000 rpm and 50 µL aliquots were removed for RP-HPLC analysis. Regardless of target M₄N or formulation, little if any changes in solubility were observed after the 24 hour incubation (Table 28).

**Table 28 M₄N Stability in 11-14 mg/mL PEG300/HP-β-CD solutions**

| | Observed M₄N mg/mL | |
|---|---|---|
| Composition | t=2 hours | t=24 hours |
| 11 mg/mL M₄N, 25% PEG300,30% HP-β-CD | 11.3 | 11.3 |
| 12 mg/mL M₄N, 25% PEG300,30% HP-β-CD | 11.8 | 11.6 |
| 13 mg/mL M₄N, 25% PEG300,30% HP-β-CD | 12.7 | 12.7 |
| 14 mg/mL M₄N, 25% PEG300,30% HP-β-CD | 13.5 | 13.5 |
| 11 mg/mL M₄N, 33% PEG300,27% HP-β-CD | 11.4 | 11.3 |
| 11 mg/mL M₄N, 20% PEG300,32% HP-β-CD | 11.5 | 11.4 |

Test samples containing between 11-14 mg/mL M₄N formulated to a final concentration of 25% PEG 300 and 30% HP-β-CD were stable after 24 hours incubation at room temperature.

### Example 19. 40 mg/mL M₄N/PEG300 Stability (Reference Example)

The stability of 40 mg/mL M₄N dissolved in 100% PEG 300 was evaluated up to 24 hours incubation at 30°C, 45°C and 60°C. A stock 40 mg/mL M₄N in PEG 300 was prepared at 60°C, following the procedure set forth in Example 18. Subsequently, aliquots were removed and incubated at the appropriate temperature. Samples were rotated at 450 rpm during the entire course of incubation. Visual observations and RP-HPLC data were collected throughout. As shown in Table 29, after 6 hours incubation at 30°C tiny crystals were observed in the 40 mg/mL M₄N/PEG 300 formulation, with more appearing after 24 hours. The formation of crystals coincided with a loss of soluble M₄N (Table 30). The 40 mg/mL M₄N/PEG 300 samples incubated at 45°C and 60°C were stable after 24 hours incubation as assessed by visual observations and RP-HPLC analysis (Tables 29 and 30). No changes in the amount or types of impurities peaks were observed at any of the incubation temperatures.

**TABLE 29. Visual appearance of 40 mg/mL M₄N/PEG 300 stability samples**

| Incubation Condition | Visual Appearance | | | |
|---|---|---|---|---|
| | Time (hours) | | | |
| | 2 | 4 | 6 | 24 |
| 30°C | Clear | Clear | Few tiny crystals | Many tiny crystals |
| 45°C | Clear | Clear | Clear | Clear |
| 60°C | Clear | Clear | Clear | Clear |

**TABLE 30. 40 mg/mL M₄N/PEG 300 stability samples: RP-HPLC analysis**

| Incubation Condition | M₄N (mg/mL) | | | |
|---|---|---|---|---|
| | Time (hours) | | | |
| | 0 | 2 | 6 | 24 |
| 30°C | | 40.8 | 40.6 | 37.4 |
| 45°C | | 40.4 | 39.8 | 40.6 |
| 60°C | 39.5 | 39.1 | 39.2 | 39.2 |

After 6 hours incubation at 30°C tiny crystals were observed in the 40 mg/mL M₄N/PEG 300 formulation. After 24 hours, even more crystals were observed as well as a >5% loss in soluble M₄N as determined by RP-HPLC analysis.

40 mg/mL M₄N/PEG 300 samples incubated at 45°C and 60°C were stable after 24 hours incubation as assessed by visual observations and RP-HPLC analysis.

### BIBLIOGRAPHY

Ansel, H.C. et at. (2004). Pharmaceutical Dosage Forms and Drug Delivery Systems eds., 8th ed., Lippincott Williams & Wilkins.
Garg, S. et al. (2001). Compendium of Pharmaceutical Excipients for Vaginal Formulations. Pharmaceutical Technol. Drug Delivery. Sept. 1,2001, pp. 14 - 24.
Gennaro, A.R. (2003). Remington: The Science and Practice of Pharmacy, 20th ed., with Facts and Comparisons: DrugfactsPlus. Lippincott Williams & Williams.
Higuchi T and Connors KA (1965) "Phase solubility techniques", Adv Anal Chem Instr. 4, 117-212.
Hwu, J.R. et al. (1998). Antiviral activities of methylated nordihydroguaiaretic acids. 1. Synthesis, structure identification, and inhibition of Tat-regulated HIV transactivation. J. Med. Chem. 41: 2994 - 3000.
McDonald, R.W. et al. (2001). Synthesis and anticancer activity of nordihydroguaiaretic acid (NDGA) and analogues. Anti-Cancer Drug Design 16: 261 - 270.
Rowe, R.C. et al. eds. (2003). Handbook of Pharmaceutical Excipients. 4th edition. Pharmaceutical Press and American Pharmaceutical Association.

## Claims

1. A composition for oral administration to an animal comprising an active pharmaceutical ingredient and a pharmaceutically acceptable carrier, wherein the active pharmaceutical ingredient comprises tetra-O-methyl NDGA, and the carrier comprises polyethylene glycol, a cyclodextrin and optionally at least one of a solubilizing agent and an excipient selected from the group consisting of: (a) a water-soluble organic solvent other than DMSO; provided that when the water-soluble organic solvent is propylene glycol, the propylene glycol is in the absence of white petrolatum, in the absence of xanthan gum and in the absence of at least one of glycerine or glycine, when the water-soluble organic solvent is polyethylene glycol, the polyethylene glycol is present in the absence of ascorbic acid or butylated hydroxytoluene, and when the polyethylene glycol is polyethylene glycol 400, the polyethylene glycol 400 is present in the absence of polyethylene glycol 8000; (b) an ionic, non-ionic or amphipathic surfactant, provided that when the surfactant is a non-ionic surfactant, the non-ionic surfactant is present in the absence of xanthan gum; (c) a modified cellulose; (d) a water-insoluble lipid, provided that when the water-insoluble lipid is castor oil, the castor oil is present in the absence of beeswax or carnuba wax; and a combination of any of the carriers (a) - (d).

2. The composition of claim 1, wherein the composition comprises 0.1 mg to 200 mg of the active pharmaceutical ingredient.

3. The composition of claim 2, wherein the composition comprises 10 mg, 20 mg, 25 mg, 30 mg,40 mg, 50 mg, 60 mg, 75 mg, 100 mg or 200 mg of the active pharmaceutical agent.

4. The composition of claim 1, wherein the active pharmaceutical ingredient is present at a concentration of 1 mg/mL to 200 mg/mL or 1 mg/g to 250mg/g.

5. The composition of claim 4, wherein the active pharmaceutical ingredient is present at a concentration of 1 mg/mL, 2 mg/mL, 2.5 mg/mL, 5 mg/mL, 10 mg/mL, 12.5 mg/mL, 15 mg/mL, 20 mg/mL, 25 mg/mL, 30 mg/mL, 40 mg/mL, 50 mg/mL, 55 mg/mL, 60 mg/mL, 75 mg/mL, 100 mg/mL, 125 mg/mL, 150 mg/mL or 175 mg/mL.

6. The composition of claim 4, wherein the active pharmaceutical ingredient is present at a concentration of 20 mg/g, 50 mg/g, 75 mg/g, 100 mg/g, 120 mg/g, 130 mg/g, 140 mg/g, 150 mg/g, 175 mg/g or 200 mg/g.

7. The composition of claim 1, wherein the water-soluble organic solvent is selected from the group consisting of polypropylene glycol, polyvinyl pyrrolidone, ethyl alcohol, benzyl alcohol and dimethylacetamide.

8. The composition of claim 1, wherein the polyethylene glycol is present at a concentration of 5% (v/v) to 100% (v/v).

9. The composition of claim 8, wherein the polyethylene glycol is present at a concentration of 20% (v/v) to 80% (v/v).

10. The composition of claim 9, wherein the polyethylene glycol is present at a concentration of 50% (v/v).

11. The composition of claim 9, wherein the polyethylene glycol is present at a concentration of (v/v).

12. The composition of claim 9, wherein the polyethylene glycol is present at a concentration of 33% (v/v).

13. The composition of claim 1, wherein the polyethylene glycol is PEG 300.

14. The composition of claim 13, wherein the PEG 300 is present at a concentration of 10% (v/v), 20% (v/v), 30% (v/v), 40% (v/v) or 50% (v/v).

15. The composition of claim 1, wherein the polyethylene glycol is PEG 400.

16. The composition of claim 15, wherein the PEG 400 is present at a concentration of 10% (v/v), 20% (v/v), 30% (v/v), 40% (v/v) or 50% (v/v).

17. The composition of claim 1 wherein the polyethylene glycol is PEG 400 monolaurate.

18. The composition of claim 17, wherein the PEG 400 monolaurate is present at a concentration of 20% (v/v) to 50% (v/v).

19. The composition of claim 1, wherein the carrier comprises an unmodified cyclodextrin or a modified cyclodextrin.

20. The composition of claim 19, wherein the modified cyclodextrin is selected from the group consisting of hydroxypropyl-β-cyclodextrin and sulfobutyl ether β-cyclodextrin.

21. The composition of claim 19, wherein the modified cyclodextrin is present at a concentration of 5% (w/v) to 80% (w/v).

22. The composition of claim 21, wherein the modified cyclodextrin is present at a concentration of 15% (w/v), 20% (w/v), 25% (w/v),30% (w/v), 35% (w/v), 40% (w/v) or 50% (w/v).

23. The composition of claim 1, wherein the carrier comprises a surfactant.

24. The composition of claim 23, wherein the surfactant is present at a concentration of 5% (v/v) to 100% (v/v).

25. The composition of claim 24, wherein the surfactant is present at a concentration of 30% (v/v), 40% (v/v) or 50% (v/v).

26. The composition of claim 1, wherein the carrier comprises a surfactant selected from the group consisting of polysorbate, d-alpha-tocopheryl polyethylene glycol 1000 succinate, an esterified fatty acid, and the reaction product of ethylene oxide and castor oil in a 35:1 molar ratio.

27. The composition of claim 25, wherein the surfactant is selected from the group consisting of polysorbate 20 and polysorbate 80.

28. The composition of claim 27, wherein the surfactant is polysorbate 20.

29. The composition of claim 23, wherein the polyethylene glycol is selected from the group consisting of PEG 300 and PEG 400.

30. The composition of claim 29, wherein the surfactant is polysorbate 20.

31. The composition of claim 23, wherein the surfactant is an esterified fatty acid.

32. The composition of claim 1, wherein the carrier comprises a modified cellulose.

33. The composition of claim 32, wherein the modified cellulose is selected from the group consisting of ethyl cellulose, hydroxypropyl methylcellulose, methylcellulose and carboxy methylcellulose.

34. The composition of claim 32, wherein the modified cellulose is present at a concentration of 0.1 % (w/v) to 10% (w/v).

35. The composition of claim 1, wherein the carrier comprises a water-insoluble lipid.

36. The composition of claim 35, wherein the water-insoluble lipid is selected from the group consisting of at least one of an oil, a wax and a fat emulsion, provided that when the composition contains castor oil, it does not contain beeswax or carnuba wax.

37. The composition of claim 35, wherein the water-insoluble lipid is a fat emulsion.

38. The composition of claim 37, wherein the fat emulsion is present at a concentration of 10% to 30%.

39. The composition of claim 37, wherein the fat emulsion is present at a concentration of 20%.

40. The composition of claim 31, wherein the water-insoluble lipid is oil.

41. The composition of claim 40, wherein the oil is selected from at least one of the group consisting of corn oil, olive oil, peppermint oil, soy bean oil, sesame seed oil, mineral oil and glycerol.

42. The composition of claim 40, wherein the oil is present at a concentration of 10% to 100%.

43. The composition of claim 40, wherein the oil is peppermint oil.

44. The composition of claim 43, further comprising sesame oil.

45. The composition of claim 40, further comprising polysorbate 20.

46. The composition of claim 40, wherein the polyethylene glycol is selected from the group consisting of PEG 300 and PEG 400.

47. A pharmaceutical composition comprising the composition of claim 1.

48. The composition of claim 1 for use in the treatment of a proliferative disease, hypertension, obesity, diabetes, a central nervous system disease, a neurodegenerative disease, pain, Alzheimer's disease, amyotrophic lateral sclerosis, dementia, Parkinson's disease, stroke, an inflammatory disease, premalignant neoplasia, dysplasia or an infection.

49. The composition for use according to claim 48, wherein the proliferative disease is cancer or psoriasis.

50. The composition for use according to claim 48, wherein the inflammatory disease is selected from the group consisting of rheumatoid arthritis, osteoarthritis, ulcerative colitis, Crohn's disease, atherosclerosis, chronic obstructive pulmonary disease (COPD) and multiple sclerosis.

51. The composition for use according to claim 48, wherein the disease is an intraepithelial neoplasia.

52. The composition for use according to claim 48, wherein the infection is a viral infection.

53. The composition for use according to claim 52, wherein the virus is selected from the group consisting of HIV, HTLV, HPV, HSV, HBV, EBV, Varicella-zoster virus, adenovirus, parvovirus or JC virus.

54. The composition for use according to claim 48, wherein the composition is administered at a dose in a range of 10 mg of active pharmaceutical ingredient per kg weight of the subject to 600 mg of active pharmaceutical ingredient per kg weight of the subject.

55. The composition for use according to claim 48, wherein the composition is administered one or more times per week.

56. The composition for use according to claim 48, wherein the composition is administered one or more times per month.

57. A kit comprising the composition of claim 1 and instructions for use thereof.

## Patentansprüche

1. Zusammensetzung zur oralen Verabreichung an ein Lebewesen, umfassend einen pharmazeutischen Wirkstoff und einen pharmazeutisch verträglichen Träger, wobei der pharmazeutische Wirkstoff Tetra-O-Methyl-NDGA umfasst und der Träger Polyethylenglykol, ein Cyclodextrin und optional mindestens eines aus einem Solubilisierungsmittel und einem Hilfsstoff umfasst, ausgewählt aus der Gruppe, bestehend aus: (a) einem wasserlöslichen organischen Lösungsmittel außer DMSO; vorausgesetzt, dass, wenn das wasserlösliche organische Lösungsmittel Propylenglykol ist, das Propylenglykol in Abwesenheit von weißem Petrolatum, in Abwesenheit von Xanthangummi und in Abwesenheit von mindestens einem aus Glycerin oder Glycin ist, wenn das wasserlösliche organische Lösungsmittel Polyethylenglykol ist, das Polyethylenglykol in Abwesenheit von Ascorbinsäure oder butyliertem Hydroxytoluol vorhanden ist, und wenn das Polyethylenglykol Polyethylenglykol 400 ist, das Polyethylenglykol 400 in Abwesenheit von Polyethylenglykol 8000 vorhanden ist; (b) einem ionischen, nichtionischen oder amphipathischen Tensid, vorausgesetzt, dass, wenn das Tensid ein nichtionisches Tensid ist, das nichtionische Tensid in Abwesenheit von Xanthangummi vorliegt; (c) einer modifizierten Cellulose; (d) einem wasserunlöslichen Lipid, vorausgesetzt, dass, wenn das wasserunlösliche Lipid Castoröl ist, das Castoröl in Abwesenheit von Bienenwachs oder Carnubawachs vorhanden ist; und einer Kombination von irgendwelchen der Träger (a) - (d).

2. Zusammensetzung nach Anspruch 1, wobei die Zusammensetzung 0,1 mg bis 200 mg des pharmazeutischen Wirkstoffs umfasst.

3. Zusammensetzung nach Anspruch 2, wobei die Zusammensetzung 10 mg, 20 mg, 25 mg, 30 mg, 40 mg, 50 mg, 60 mg, 75 mg, 100 mg oder 200 mg des pharmazeutischen Wirkstoffs umfasst.

4. Zusammensetzung nach Anspruch 1, wobei der pharmazeutische Wirkstoff in einer Konzentration von 1 mg/ml bis 200 mg/ml oder 1 mg/g bis 250 mg/g vorliegt.

5. Zusammensetzung nach Anspruch 4, wobei der pharmazeutische Wirkstoff in einer Konzentration von 1 mg/ml, 2 mg/ml, 2,5 mg/ml, 5 mg/ml, 10 mg/ml, 12,5 mg/ml, 15 mg/ml, 20 mg/ml, 25 mg/ml, 30 mg/ml, 40 mg/ml, 50 mg/ml, 55 mg/ml, 60 mg/ml, 75 mg/ml, 100 mg/ml, 125 mg/ml, 150 mg/ml oder 175 mg/ml vorliegt.

6. Zusammensetzung nach Anspruch 4, wobei der pharmazeutische Wirkstoff in einer Konzentration von 20 mg/g, 50 mg/g, 75 mg/g, 100 mg/g, 120 mg/g, 130 mg/g, 140 mg/g, 150 mg/g, 175 mg/g oder 200 mg/g vorhanden ist.

7. Zusammensetzung nach Anspruch 1, wobei das wasserlösliche organische Lösungsmittel ausgewählt ist aus der Gruppe, bestehend aus Polypropylenglykol, Polyvinylpyrrolidon, Ethylalkohol, Benzylalkohol und Dimethylacetamid.

8. Zusammensetzung nach Anspruch 1, wobei das Polyethylenglykol in einer Konzentration von 5% (v/v) bis 100% (v/v) vorhanden ist.

9. Zusammensetzung nach Anspruch 8, wobei das Polyethylenglykol in einer Konzentration von 20% (v/v) bis 80% (v/v) vorhanden ist.

10. Zusammensetzung nach Anspruch 9, wobei das Polyethylenglykol in einer Konzentration von 50% (v/v) vorhanden ist.

11. Zusammensetzung nach Anspruch 9, wobei das Polyethylenglykol in einer Konzentration von 40% (v/v) vorhanden ist.

12. Zusammensetzung nach Anspruch 9, wobei das Polyethylenglykol in einer Konzentration von 33% (v/v) vorhanden ist.

13. Zusammensetzung nach Anspruch 1, wobei das Polyethylenglykol PEG 300 ist.

14. Zusammensetzung nach Anspruch 13, wobei das PEG 300 in einer Konzentration von 10% (v/v), 20% (v/v), 30% (v/v), 40% (v/v) oder 50% (v/v) vorhanden ist.

15. Zusammensetzung nach Anspruch 1, wobei das Polyethylenglykol PEG 400 ist.

16. Zusammensetzung nach Anspruch 15, wobei das PEG 400 in einer Konzentration von 10% (v/v), 20% (v/v), 30% (v/v), 40% (v/v) oder 50% (v/v) vorhanden ist.

17. Zusammensetzung nach Anspruch 1, wobei das Polyethylenglykol PEG 400-Monolaurat ist.

18. Zusammensetzung nach Anspruch 17, wobei das PEG 400-Monolaurat in einer Konzentration von 20% (v/v) bis 50% (v/v) vorhanden ist.

19. Zusammensetzung nach Anspruch 1, wobei der Träger ein nicht modifiziertes Cyclodextrin oder ein modifiziertes Cyclodextrin umfasst.

20. Zusammensetzung nach Anspruch 19, wobei das modifizierte Cyclodextrin ausgewählt ist aus der Gruppe, bestehend aus Hydroxypropyl-β-cyclodextrin und Sulfobutylether-β-cyclodextrin.

21. Zusammensetzung nach Anspruch 19, wobei das modifizierte Cyclodextrin in einer Konzentration von 5% (w/v) bis 80% (w/v) vorhanden ist.

22. Zusammensetzung nach Anspruch 21, wobei das modifizierte Cyclodextrin in einer Konzentration von 15% (w/v), 20% (w/v), 25% (w/v), 30% (w/v), 35% (w/v), 40% (w/v) oder 50% (w/v) vorhanden ist.

23. Die Zusammensetzung nach Anspruch 1, wobei der Träger ein Tensid umfasst.

24. Zusammensetzung nach Anspruch 23, wobei das Tensid in einer Konzentration von 5% (v/v) bis 100% (v/v) vorhanden ist.

25. Zusammensetzung nach Anspruch 24, wobei das Tensid in einer Konzentration von 30% (v/v), 40% (v/v) oder 50% (v/v) vorhanden ist.

26. Zusammensetzung nach Anspruch 1, wobei der Träger ein Tensid umfasst, ausgewählt aus der Gruppe, bestehend aus Polysorbat, D-Alpha-Tocopheryl-Polyethylenglycol-1000-Succinat, einer veresterten Fettsäure, und dem Reaktionsprodukt aus Ethylenoxid und Castoröl in einem Molverhältnis von 35:1.

27. Zusammensetzung nach Anspruch 25, wobei das Tensid ausgewählt ist aus der Gruppe, bestehend aus Polysorbat 20 und Polysorbat 80.

28. Zusammensetzung nach Anspruch 27, wobei das Tensid Polysorbat 20 ist.

29. Zusammensetzung nach Anspruch 23, wobei das Polyethylenglykol ausgewählt ist aus der Gruppe, bestehend aus PEG 300 und PEG 400.

30. Zusammensetzung nach Anspruch 29, wobei das Tensid Polysorbat 20 ist.

31. Zusammensetzung nach Anspruch 23, wobei das Tensid eine veresterte Fettsäure ist.

32. Zusammensetzung nach Anspruch 1, wobei der Träger eine modifizierte Cellulose umfasst.

33. Zusammensetzung nach Anspruch 32, wobei die modifizierte Cellulose ausgewählt ist aus der Gruppe, bestehend aus Ethylcellulose, Hydroxypropylmethylcellulose, Methylcellulose und Carboxymethylcellulose.

34. Zusammensetzung nach Anspruch 32, wobei die modifizierte Cellulose in einer Konzentration von 0,1% (w/v) bis 10% (w/v) vorhanden ist.

35. Zusammensetzung nach Anspruch 1, wobei der Träger ein wasserunlösliches Lipid umfasst.

36. Zusammensetzung nach Anspruch 35, wobei das wasserunlösliche Lipid ausgewählt ist aus der Gruppe, bestehend aus mindestens einem aus einem Öl, einem Wachs und einer Fettemulsion, vorausgesetzt, dass, wenn die Zusammensetzung Castoröl enthält, sie nicht Bienenwachs oder Carnaubawachs enthält.

37. Zusammensetzung nach Anspruch 35, wobei das wasserunlösliche Lipid eine Fettemulsion ist.

38. Zusammensetzung nach Anspruch 37, wobei die Fettemulsion in einer Konzentration von 10% bis 30% vorhanden ist.

39. Zusammensetzung nach Anspruch 37, wobei die Fettemulsion in einer Konzentration von 20% vorhanden ist.

40. Zusammensetzung nach Anspruch 31, wobei das wasserunlösliche Fett ein Öl ist.

41. Zusammensetzung nach Anspruch 40, wobei das Öl ausgewählt ist aus wenigstens einem aus der Gruppe, bestehend aus Maisöl, Olivenöl, Pfefferminzöl, Sojabohnenöl, Sesamöl, Mineralöl und Glycerin.

42. Zusammensetzung nach Anspruch 40, wobei das Öl in einer Konzentration von 10% bis 100% vorhanden ist.

43. Zusammensetzung nach Anspruch 40, wobei das Öl Pfefferminzöl ist.

44. Zusammensetzung nach Anspruch 43, ferner umfassend Sesamöl.

45. Zusammensetzung nach Anspruch 40, ferner umfassend Polysorbat 20.

46. Zusammensetzung nach Anspruch 40, wobei das Polyethylenglykol ausgewählt ist aus der Gruppe, bestehend aus PEG 300 und PEG 400.

47. Pharmazeutische Zusammensetzung, umfassend die Zusammensetzung nach Anspruch 1.

48. Zusammensetzung nach Anspruch 1 zur Verwendung bei der Behandlung einer proliferativen Erkrankung, von Bluthochdruck, Fettsucht, Diabetes, einer Krankheit des Zentralnervensystems, einer neurodegenerativen Krankheit, von Schmerz, der Alzheimer-Krankheit, von amyotropher Lateralsklerose, Demenz, der Parkinson-Krankheit, von Schlaganfall, einer entzündlichen Erkrankung, von prämaligner Neoplasie, Dysplasie oder einer Infektion.

49. Zusammensetzung zur Verwendung nach Anspruch 48, wobei die proliferative Erkrankung Krebs oder Psoriasis ist.

50. Zusammensetzung zur Verwendung nach Anspruch 48, wobei die entzündliche Erkrankung ausgewählt ist aus der Gruppe, bestehend aus rheumatoider Arthritis, Osteoarthritis, Colitis ulcerosa, Morbus Crohn, Atherosklerose, chronischer obstruktiver Lungenerkrankung (COPD) und Multipler Sklerose.

51. Zusammensetzung zur Verwendung nach Anspruch 48, wobei die Krankheit eine intraepitheliale Neoplasie ist.

52. Zusammensetzung zur Verwendung nach Anspruch 48, wobei die Infektion eine virale Infektion ist.

53. Zusammensetzung zur Verwendung gemäß Anspruch 52, wobei das Virus ausgewählt ist aus der Gruppe, bestehend aus HIV, HTLV, HPV, HSV, HBV, EBV, Varicella-Zoster-Virus, Adenovirus, Parvovirus oder JC-Virus.

54. Zusammensetzung zur Verwendung nach Anspruch 48, wobei die Zusammensetzung in einer Dosis von 10 mg an pharmazeutischem Wirkstoff pro kg Gewicht des Subjekts bis 600 mg an pharmazeutischem Wirkstoff pro kg Körpergewicht des Subjekts verabreicht wird.

55. Zusammensetzung zur Verwendung nach Anspruch 48, wobei die Zusammensetzung ein oder mehrere Male pro Woche verabreicht wird.

56. Zusammensetzung zur Verwendung nach Anspruch 48, wobei die Zusammensetzung ein oder mehrere Male pro Monat verabreicht wird.

57. Kit, umfassend die Zusammensetzung nach Anspruch 1 und Anweisungen zur Verwendung derselben.

## Revendications

1. Composition pour l'administration par voie orale à un animal comprenant un ingrédient pharmaceutique actif et un support pharmaceutiquement acceptable, où l'ingrédient pharmaceutique actif comprend du tétra-O-méthyl NDGA, et le support comprend du polyéthylène glycol, une cyclodextrine et éventuellement au moins l'un parmi un agent solubilisant et un excipient sélectionné dans le groupe constitué de : (a) un solvant organique soluble dans l'eau autre que le DMSO ; à condition que lorsque le solvant organique soluble dans l'eau est le propylène glycol, le propylène glycol soit présent en l'absence de pétrolatum blanc, en l'absence de gomme de xanthane et en l'absence d'au moins l'une parmi la glycérine ou la glycine, lorsque le solvant organique soluble dans l'eau est le polyéthylène glycol, le polyéthylène glycol est présent en l'absence d'acide ascorbique ou d'hydroxytoluène butylé, et lorsque le polyéthylène glycol est le polyéthylène glycol 400, le polyéthylène glycol 400 est présent en l'absence de polyéthylène glycol 8000 ; (b) un tensioactif ionique, non-ionique ou amphipathique, à condition que lorsque le tensioactif est un tensioactif non ionique, le tensioactif non ionique soit présent en l'absence de gomme de xanthane ; (c) une cellulose modifiée ; (d) un lipide insoluble dans l'eau, à condition que lorsque le lipide insoluble dans l'eau est l'huile de ricin, l'huile de ricin soit présente en l'absence de cire d'abeille ou de cire de carnuba ; et une combinaison de n'importe lequel des supports (a) à (d).

2. Composition selon la revendication 1, où la composition comprend de 0,1 mg à 200 mg de l'ingrédient pharmaceutique actif.

3. Composition selon la revendication 2, où la composition comprend 10 mg, 20 mg, 25 mg, 30 mg, 40 mg, 50 mg, 60 mg, 75 mg, 100 mg ou 200 mg de l'agent pharmaceutique actif.

4. Composition selon la revendication 1, où l'ingrédient pharmaceutique actif est présent à une concentration de 1 mg/ml à 200 mg/ml ou 1 mg/g à 250 mg/g.

5. Composition selon la revendication 4, où l'ingrédient pharmaceutique actif est présent à une concentration de 1 mg/ml, 2 mg/ml, 2,5 mg/ml, 5 mg/ml, 10 mg/ml, 12,5 mg/ml, 15 mg/ml, 20 mg/ml, 25 mg/ml, 30 mg/ml, 40 mg/ml, 50 mg/ml, 55 mg/ml, 60 mg/ml, 75 mg/ml, 100 mg/ml, 125 mg/ml, 150 mg/ml ou 175 mg/ml.

6. Composition selon la revendication 4, où l'ingrédient pharmaceutique actif est présent à une concentration de 20 mg/g, 50 mg/g, 75 mg/g, 100 mg/g, 120 mg/g, 130 mg/g, 140 mg/g, 150 mg/g, 175 mg/g ou 200 mg/g.

7. Composition selon la revendication 1, où le solvant organique soluble dans l'eau est sélectionné dans le groupe constitué du polypropylène glycol, de la polyvinyl pyrrolidone, de l'alcool d'éthyle, de l'alcool de benzyle et du diméthylacétamide.

8. Composition selon la revendication 1, où le polyéthylène glycol est présent à une concentration de 5 % (en vol/vol) à 100 % (en vol/vol).

9. Composition selon la revendication 8, où le polyéthylène glycol est présent à une concentration de 20 % (en vol/vol) à 80 % (en vol/vol).

10. Composition selon la revendication 9, où le polyéthylène glycol est présent à une concentration de 50 % (en vol/vol).

11. Composition selon la revendication 9, où le polyéthylène glycol est présent à une concentration de 40 % (en vol/vol).

12. Composition selon la revendication 9, où le polyéthylène glycol est présent à une concentration de 33 % (en vol/vol).

13. Composition selon la revendication 1, où le polyéthylène glycol est le PEG 300.

14. Composition selon la revendication 13, où le PEG 300 est présent à une concentration de 10 % (en vol/vol), 20 % (en vol/vol), 30 % (en vol/vol), 40 % (en vol/vol) ou 50 % (en vol/vol).

15. Composition selon la revendication 1, où le polyéthylène glycol est le PEG 400.

16. Composition selon la revendication 15, où le PEG 400 est présent à une concentration de 10 % (en vol/vol), 20 % (en vol/vol), 30 % (en vol/vol), 40 % (en vol/vol) ou 50 % (en vol/vol).

17. Composition selon la revendication 1 où le polyéthylène glycol est le monolaurate de PEG 400.

18. Composition selon la revendication 17, où le monolaurate de PEG 400 est présent à une concentration de 20 % (en vol/vol) à 50 % (en vol/vol).

19. Composition selon la revendication 1, où le support comprend une cyclodextrine non modifiée ou une cyclodextrine modifiée.

20. Composition selon la revendication 19, où la cyclodextrine modifiée est sélectionnée dans le groupe constitué de l'hydroxypropyl-β-cyclodextrine et de la sulfobutyl éther β-cyclodextrine.

21. Composition selon la revendication 19, où la cyclodextrine modifiée est présente à une concentration de 5 % (en pds/vol) à 80 % (en pds/vol).

22. Composition selon la revendication 21, où la cyclodextrine modifiée est présente à une concentration de 15 % (en pds/vol), 20 % (en pds/vol), 25 % (en pds/vol), 30 % (en pds/vol), 35 % (en pds/vol), 40 % (en pds/vol) ou 50 % (en pds/vol).

23. Composition selon la revendication 1, où le support comprend un tensioactif.

24. Composition selon la revendication 23, où le tensioactif est présent à une concentration de 5 % (en vol/vol) à 100 % (en vol/vol).

25. Composition selon la revendication 24, où le tensioactif est présent à une concentration de 30 % (en vol/vol), 40 % (en vol/vol) ou 50 % (en vol/vol).

26. Composition selon la revendication 1, où le support comprend un tensioactif sélectionné dans le groupe constitué du polysorbate, du succinate de d-alpha-tocophéryl polyéthylène glycol 1000, d'un acide gras estérifié, et du produit réactionnel de l'oxyde d'éthylène et de l'huile de ricin en un rapport molaire de 35:1.

27. Composition selon la revendication 25, où le tensioactif est sélectionné dans le groupe constitué du polysorbate 20 et du polysorbate 80.

28. Composition selon la revendication 27, où le tensioactif est le polysorbate 20.

29. Composition selon la revendication 23, où le polyéthylène glycol est sélectionné dans le groupe constitué du PEG 300 et du PEG 400.

30. Composition selon la revendication 29, où le tensioactif est le polysorbate 20.

31. Composition selon la revendication 23, où le tensioactif est un acide gras estérifié.

32. Composition selon la revendication 1, où le support comprend une cellulose modifiée.

33. Composition selon la revendication 32, où la cellulose modifiée est sélectionnée dans le groupe constitué de l'éthyl cellulose, de l'hydroxypropyl méthylcellulose, de la méthylcellulose et de la carboxy méthylcellulose.

34. Composition selon la revendication 32, où la cellulose modifiée est présente à une concentration de 0,1 % (en pds/vol) à 10 % (en pds/vol).

35. Composition selon la revendication 1, où le support comprend un lipide insoluble dans l'eau.

36. Composition selon la revendication 35, où le lipide insoluble dans l'eau est sélectionné dans le groupe constitué d'au moins l'une parmi une huile, une cire et une émulsion grasse, à condition que lorsque la composition contient de l'huile de ricin, elle ne contienne pas de cire d'abeille ou de cire de carnauba.

37. Composition selon la revendication 35, où le lipide insoluble dans l'eau est une émulsion grasse.

38. Composition selon la revendication 37, où l'émulsion grasse est présente à une concentration de 10 % à 30 %.

39. Composition selon la revendication 37, où l'émulsion grasse est présente à une concentration de 20 %.

40. Composition selon la revendication 31, où le lipide insoluble dans l'eau est une huile.

41. Composition selon la revendication 40, où l'huile est sélectionnée parmi au moins l'un du groupe constitué de l'huile de maïs, de l'huile d'olive, de l'huile de menthe poivrée, de l'huile de soja, de l'huile de graines de sésame, de l'huile minérale et du glycérol.

42. Composition selon la revendication 40, où l'huile est présente à une concentration de 10 % à 100 %.

43. Composition selon la revendication 40, où l'huile est l'huile de menthe poivrée.

44. Composition selon la revendication 43, comprenant en outre de l'huile de sésame.

45. Composition selon la revendication 40, comprenant en outre du polysorbate 20.

46. Composition selon la revendication 40, où le polyéthylène glycol est sélectionné dans le groupe constitué du PEG 300 et du PEG 400.

47. Composition pharmaceutique comprenant la composition selon la revendication 1.

48. Composition selon la revendication 1 pour l'utilisation dans le traitement d'une maladie proliférative, de l'hypertension, de l'obésité, du diabète, d'une maladie du système nerveux central, d'une maladie de neurodégénérescence, de la douleur, de la maladie d'Alzheimer, de la sclérose latérale amyotrophique, de la démence, de la maladie de Parkinson, de l'accident vasculaire cérébral, d'une maladie inflammatoire, de la néoplasie prémaligne, de la dysplasie ou d'une infection.

49. Composition pour l'utilisation selon la revendication 48, où la maladie proliférative est le cancer ou le psoriasis.

50. Composition pour l'utilisation selon la revendication 48, où la maladie inflammatoire est sélectionnée dans le groupe constitué de l'arthrite rhumatoïde, de l'ostéoarthrite, de la rectocolite hémorragique, de la maladie de Crohn, de l'athérosclérose, de la maladie pulmonaire obstructive chronique (MPOC) et de la sclérose en plaques.

51. Composition pour l'utilisation selon la revendication 48, où la maladie est une néoplasie intraépithéliale.

52. Composition pour l'utilisation selon la revendication 48, où l'infection est une infection virale.

53. Composition pour l'utilisation selon la revendication 52, où le virus est sélectionné dans le groupe constitué du VIH, du HTLV, du VPH, du VHS, du VHB, du VEB, du virus de la varicelle et du zona, d'un adénovirus, d'un parvovirus ou du virus JC.

54. Composition pour l'utilisation selon la revendication 48, où la composition est administrée à une dose située dans une plage de 10 mg d'ingrédient pharmaceutique actif par poids en kg du sujet à 600 mg de l'ingrédient pharmaceutique actif par poids en kg du sujet.

55. Composition pour l'utilisation selon la revendication 48, où la composition est administrée une ou plusieurs fois par semaine.

56. Composition pour l'utilisation selon la revendication 48, où la composition est administrée une ou plusieurs fois par mois.

57. Trousse comprenant la composition selon la revendication 1 et ses instructions d'utilisation.
